# EUROPÄISCHE PATENTANMELDUNG

(11) **EP 3 329 920 A2**
(43) Veröffentlichungstag der Anmeldung: **06.06.2018**
(21) Anmeldenummer: 17208289.3
(22) Anmeldetag: 16.12.2014
(51) Int. Cl.: A61K 31/5377, A61K 31/506, C07D 401/14, C07D 413/14, C07D 417/14, C07D 491/107, A61K 31/541, A61P 7/00, A61P 9/00, A61P 17/02

(54) **ADRENOREZEPTOR ALPHA2C REZEPTOR ANTAGONISTEN**

(30) Priorität: 19.12.2013 EP 13198385; 12.11.2014 EP 14192877
(62) Teilanmeldung aus: 14812245.0
(71) Anmelder: Bayer Pharma Aktiengesellschaft, 13353 Berlin (DE)
(72) Erfinder: BECKER-PELSTER, Eva Maria, 42327 Wuppertal (DE); BUCHGRABER, Philipp, 10435 Berlin (DE); BUCHMÜLLER, Anja, 45259 Essen (DE); ENGEL, Karen, 64380 Roßdorf (DE); GEIß, Volker, 40883 Ratingen (DE); GÖLLER, Andreas, 42113 Wuppertal (DE); HIMMEL, Herbert, 45239 Essen (DE); KAST, Raimund, 42349 Wuppertal (DE); KNORR, Andreas, 40699 Erkrath (DE); LANG, Dieter, 42553 Velbert (DE); REDLICH, Gorden, 44799 Bochum (DE); SCHMECK, Carsten, 45472 Mülheim (DE); TINEL, Hanna, 42113 Wuppertal (DE); WUNDER, Frank, 42117 Wuppertal (DE)
(74) Vertreter: BIP Patents

(57) **Zusammenfassung**

Die Erfindung betrifft Adrenorezeptor α2C Rezeptor Antagonisten zur Verwendung in einem Verfahren zur Behandlung und/oder Prophylaxe von Begleit- und/oder Folgeerkrankungen von Diabetes mellitus, diabetischen Herzerkrankungen, diabetischen koronaren Herzerkrankungen, diabetischen koronaren mikrovaskulären Herzerkrankungen, diabetischer Herzinsuffizienz, diabetischer Kardiomyopathie und Herzinfarkt, diabetischer Mikroangiopathie, diabetischer Retinopathie, diabetischer Neuropathie, diabetischer Nephropathie, diabetischer erektiler Dysfunktion, diabetischen Geschwüren an den Extremitäten, diabetischen Fußulzera, zur Förderung der diabetischen Wundheilung und zur Förderung der Wundheilung von diabetischen Fußulzera.

## Beschreibung

Die Erfindung betrifft Adrenorezeptor α2C Rezeptor Antagonisten zur Verwendung in einem Verfahren zur Behandlung und/oder Prophylaxe von Begleit- und/oder Folgeerkrankungen von Diabetes mellitus, diabetischen Herzerkrankungen, diabetischen koronaren Herzerkrankungen, diabetischen koronaren mikrovaskulären Herzerkrankungen, diabetischer Herzinsuffizienz, diabetischer Kardiomyopathie und Herzinfarkt, diabetischer Mikroangiopathie, diabetischer Retinopathie, diabetischer Neuropathie, diabetischer Nephropathie, diabetischer erektiler Dysfunktion, diabetischen Geschwüren an den Extremitäten, diabetischen Fußulzera, zur Förderung der diabetischen Wundheilung und zur Förderung der Wundheilung von diabetischen Fußulzera.

Adrenoreceptor α₂ Rezeptoren (α₂-ARs) gehören zur Familie der G-Protein gekoppelten Rezeptoren. Sie binden an die Pertussis-Toxin sensitiven inhibitorischen G Protein Gᵢ und G₀ und verringern die Adenylatcyclase Aktivität. Sie sind an der Vermittlung von diversen physiologischen Effekten in unterschiedlichen Geweben nach Stimulation durch endogene Katecholamine (Adrenalin, Noradrenalin) beteiligt, die entweder von Synapsen freigesetzt werden oder über das Blut den Wirkort erreichen. α₂-AR spielen eine wichtige physiologische Rolle, hauptsächlich für das kardiovaskuläre System aber auch im zentralen Nervensystem. Biochemische, physiologische und pharmakologische Untersuchungen haben gezeigt, dass neben verschiedenen α₁-AR drei α₂-AR Subtypen (α_{2A}, α_{2B} und α_{2C}) in vielen kardiovaskulär relevanten Zielzellen und Geweben existieren, was sie zu attraktiven Zielproteinen für therapeutische Interventionen macht. Allerdings bleibt bis heute die Aufklärung der präzisen physiologischen Aufgabe der Rezeptoruntertypen schwierig, da es an hoch selektiven Liganden und/oder Antagonisten der jeweiligen α₂-AR mangelt (Gyires et al., α2-Adrenoceptor subtypes-mediated physiological, pharmacological actions, Neurochemistry International 55, 447-453, 2009; Tan und Limbird, The α2-Adrenergic Receptors: Adrenergic Receptors in the 21st Century/Receptors, 2005, 241-265).

Kardiovaskuläre Veränderungen wie zum Beispiel die Regulation der Kontraktionskraft des Herzens werden zum einen durch die zentrale Modulation der sympathischen Efferenzen reguliert. Desweiteren reguliert das sympathische Efferenzsystem auch direkte Effekte auf die Glattmuskelzellen und Endothelzellen der Gefäße. So ist das sympathische System an der Regulation der Auswurfleistung des Herzens aber auch an der Steuerung der lokalen Durchblutung unterschiedlicher Gefäßbetten beteiligt. Dies wird auch über die α₂-AR gesteuert, die an der Regulation des peripheren Widerstands beteiligt sind. So sind Blutgefäße mit sympathischen Nervenfasern innerviert, die in der Adventitia verlaufen und an deren Endigungen sich Varikositäten zur Freisetzung von Noradrenalin befinden. Freigesetztes Noradrenalin moduliert über die α₂-AR in Endothelzellen und Glattmuskelzellen den jeweiligen lokalen Gefäßtonus.

Zusätzlich zu den Effekten auf die sympathischen Efferenzen wird die kardiovaskuläre Funktion in der Peripherie auch durch prä- und postsynaptische α₂-AR reguliert. Glattmuskelzellen und Endothelzellen exprimieren verschiedene α₂-AR Subtypen. Die Aktivierung von α_{2A}, α_{2B} und α_{2C} Rezeptoren auf Glattmuskelzellen führt zur Kontraktion mit resultierender Vasokonstriktion (Kanagy, Clinical Science 109:431-437, 2005). Jedoch variiert die Verteilung der jeweiligen Rezeptorsubtypen in den unterschiedlichen Gefäßbetten, zwischen den Spezies und zwischen unterschiedlichen Gefäßgrößen. So scheinen α_{2A}-AR nahezu exklusiv in großen Arterien exprimiert zu werden, während α_{2B}-AR mehr zum Gefäßtonus in kleinen Arterien und Venen beitragen. ARα_{2B} scheint eine Rolle zu spielen bei der Salzinduzierten Hypertonie (Gyires et al., α2-Adrenoceptor subtypes-mediated physiological, pharmacological actions, Neurochemistry International 55, 447-453, 2009). Obwohl die Rolle von ARα_{2C} auf die Hämodynamik noch nicht voll verstanden ist, scheinen ARα_{2C} Rezeptoren eine venöse Vasokonstriktion zu vermitteln. Sie sind auch an der kälteinduzierten Verstärkung von Adrenoceptor induzierter Vasokonstriktion beteiligt (Chotani et al., Silent α2C adrenergic receptors enable cold-induced vasoconstriction in cutaneous arteries. Am J Physiol 278:H1075-H1083, 2000; Gyires et al., α2-Adrenoceptor subtypes-mediated physiological, pharmacological actions, Neurochemistry International 55, 447-453, 2009). Kälte und andere Faktoren (z.B. Gewebeproteine, Östrogen) regulieren die funktionale Kopplung von ARα_{2C} zu intrazellulären Signalwegen (Chotani et al., Distinct cAMP signaling pathways differentially regulate α2C adrenenoxceptor expression: role in serum induction in human arteriolar smooth muscle cells. Am J Physiol Heart Circ Physiol 288: H69-H76, 2005). Deswegen ist es sinnvoll, selektive Inhibitoren AR-α₂ Subtypen auf ihre perfusionsmodulierende Wirkung auf unterschiedliche Gefäßbetten unter unterschiedlichen pathophysiologischen Bedingungen zu untersuchen.

Unter pathophysiologischen Bedingungen kann das adrenerge System aktiviert sein, was zum Beispiel zu Bluthochdruck, Herzinsuffizienz, erhöhter Plättchenaktivierung, endothelialer Dysfunktion, Atherosklerose, Angina pectoris, Myokardinfarkt, Thrombosen, peripheren Durchblutungsstörungen, Schlaganfall und sexueller Dysfunktion fuhren kann. So ist zum Beispiel die Pathophysiologie von Raynaud Syndrom und Sklerodermie weitgehend unklar, wird aber mit einer veränderten adrenergen Aktivität in Zusammenhang gebracht. So zeigen z.B. Patienten mit spastischem Raynaud Syndrom eine signifikant erhöhte Expression von ARα₂ Rezeptoren auf ihren Thrombozyten. Dies könnte in Zusammenhang stehen mit den vasospastischen Attacken, die bei diesen Patienten beobachtet werden (Keenan und Porter, α₂-Adrenergic receptors in platelets from patients with Raynaud's syndrome, Surgery, V94(2),1983).

Eine auf die Beeinflussung des aktivierten adrenergen Systems in Organismen abzielende Behandlungsmöglichkeit für derartige Erkrankungen ist aufgrund der zu erwartenden hohen Effizienz und geringer Nebenwirkungen ein vielversprechender Ansatz. Insbesondere bei Diabetikern, die oft zu hohe Catecholaminspiegel aufweisen, spielen periphere Durchblutungsstörungen (Mikroangiopathien) wie z.B. die diabetische Retinopathie, Nephropathie aber auch ausgeprägte Wundheilungsstörungen (diabetische Fußulzera) eine große Rolle. Bei der peripheren Verschlusskrankheit ist Diabetes eine der wichtigsten Komorbididäten und spielt auch im Krankheitsverlauf eine entscheidende Rolle (Mikro- und Makroangiopathie). Eine höhere Expression der Adrenorezeptor α_{2C} Rezeptoren verbunden mit erhöhten Catecholaminspiegeln könnte in diese pathophysiologischen Prozesse bei Diabetikern involviert sein.

2011 gab es weltweit 350 Mio Diabetiker (≈ 6.6% der Bevölkerung) und es wird erwartet, dass sich diese Zahl bis 2028 verdoppelt. Diabetische Fußulzera sind die häufigste Ursache für Krankenhausaufenthalte von Diabetikern. Das Risiko eines Diabetikers, im Laufe seines Lebens einen diabetischen Fußulcus zu entwickeln liegt bei 15-25%, 15% aller diabetischen Fußulzera führen zur Amputation. 40-70% aller nicht-traumatischen Amputationen weltweit werden an Diabetikern vorgenommen. Risikofaktoren für diabetische Fußulzera sind Traumata, schlechte Stoffwechselkontrolle, sensorische, motorische und autonome Polyneuropathie, nicht angemessenes Schuhwerk, Infektionen und periphere Arterienerkrankungen. Die Behandlung diabetischer Fußulzera erfordert interdisziplinäre Teams und verwendet einen multifaktoriellen Ansatz: Gewichtsverlust, Revaskularisierung (im Falle von peripherer arterieller Verschlusskrankheit, pAVK), Verbesserung der Stoffwechselkontrolle, Wundausschneidung, Verbände, Dalteparin, Regranex (PDGF) und Amputation. Die Behandlungskosten belaufen sich pro diabetischem Fußulcus (ohne Amputation) auf 7.000-10.000 USD. 33% aller diabetischen Fußulzera heilen nicht innerhalb von 2 Jahren und es gibt eine hohe Rückfallrate (34% im ersten Jahr, 61% in 3 Jahren).

Eine Aufgabe der vorliegenden Erfindung ist es daher, neue selektive Adrenorezeptor α_{2C} Rezeptor Antagonisten zur Behandlung und/oder Prophylaxe von Krankheiten, wie z. B. kardiovaskulären Erkrankungen, bei Menschen und Tieren zur Verfügung zu stellen.

Eine Aufgabe der vorliegenden Erfindung ist es auch, neue selektive Adrenorezeptor α_{2C} Rezeptor Antagonisten zur Behandlung und/oder Prophylaxe von peripheren Durchblutungsstörungen (Mikroangiopathien) wie z.B. diabetischer Retinopathie, diabetischer Nephropathie und Wundheilungsstörungen (diabetischen Fußulzera) zur Verfügung zu stellen.

WO 2005/042517, WO 2003/020716, WO 2002/081449 und WO 2000/066559 beschreiben strukturell ähnliche Bipiperidinyl-Derivate als Inhibitoren des CCR5 Rezeptors unter anderem zur Behandlung von HIV. WO 2005/077369 beschreibt strukturell ähnliche Bipiperidinyl-Derivate als Inhibitoren des CCR3 Rezeptors unter anderem zur Behandlung von Asthma. WO 94/22826 beschreibt strukturell ähnliche Piperidine als peripher gefäßerweiternde Wirkstoffe.

Gegenstand der Erfindung sind Verbindungen der Formel (I) 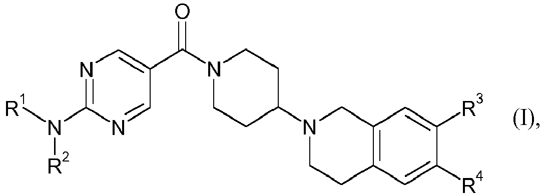 in welcher
- R¹: für C₁-C₆-Alkyl oder C₃-C₅-Cycloalkyl steht,
wobei Alkyl substituiert ist mit 1 bis 2 Substituenten, unabhängig voneinander ausgewählt aus der Gruppe bestehend aus Hydroxy, C₁-C₄-Alkoxy und Haloalkoxy,
und
- R²: für Wasserstoff oder C₁-C₄-Alkyl steht,
oder
- R¹ und R²: zusammen mit dem Stickstoffatom an das sie gebunden sind einen 4- bis 7-gliedrigen N-Heterocyclus bilden,
wobei der N-Heterocyclus substituiert sein kann mit 1 bis 3 Substituenten, unabhängig voneinander ausgewählt aus der Gruppe bestehend aus Oxo, Hydroxy, Monofluormethyl, Difluormethyl, Trifluormethyl, Hydroxycarbonyl, tert-Butoxycarbonyl, Aminocarbonyl, C₁-C₄-Alkyl, C₁-C₄-Alkoxy, C₁-C₄-Alkoxy-C₁-C₄-Alkyl, Halogen und Hydroxyalkyl
oder
wobei der N-Heterocyclus zwei Substituenten aufweisen kann, die zusammen mit dem Kohlenstoffatom des N-Heterocyclus, an das sie gemeinsam gebunden sind, einen 4- bis 6-gliedrigen Heterocyclus bilden,
worin dieser Heterocyclus seinerseits substituiert sein kann mit 1 bis 3 Substituenten, unabhängig voneinander ausgewählt aus der Gruppe bestehend aus Oxo, Methyl und Ethyl,
- R³: für Wasserstoff, Fluor, Methoxy oder Ethoxy steht,
und
- R⁴: für Wasserstoff, Fluor, Methoxy oder Ethoxy steht,
und ihre Salze, ihre Solvate und die Solvate ihrer Salze.

Erfindungsgemäße Verbindungen sind die Verbindungen der Formel (I) und deren Salze, Solvate und Solvate der Salze, sowie die von Formel (I) umfassten, nachfolgend als Ausführungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Im Rahmen der vorliegenden Erfindung bedeutet der Begriff "x Säure" in einer beliebigen Formel kein stöchiometrisch definiertes Verhältnis von Säure und der jeweiligen Substanz. In Abhängigkeit von z.B. der Basizität der jeweiligen Substanz bezeichnet der Begriff "x Säure" unterschiedliche Verhältnisse zwischen Substanz und Säure, wie 10:1 bis 1:10; 8:1 bis 1:8; 7:1 bis 1:7; 5:1 bis 1:5; 4,5:1 bis 1:4,5; 4:1 bis 1:4; 3,5:1 bis 1: 3,5; 3:1 bis 1:3; 2,5:1 bis 1: 2,5; 2:1 bis 1:2; 1,5:1 bis 1:1,5; und 1:1.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler beziehungsweise chiraler Phase.

Sofern die erfindungsgemäßen Verbindungen in tautomeren Formen vorkommen können, umfasst die vorliegenden Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N-*Methylmorpholin, Arginin, Lysin, Ethylendiamin, *N-*Methylpiperidin und Cholin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Außerdem umfasst die vorliegende Erfindung auch Prodrugs der erfindungsgemäßen Verbindungen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Im Rahmen der vorliegenden Erfindung haben die Substituenten, soweit nicht anders spezifiziert, die folgende Bedeutung:
Alkyl per se und "Alk" und "Alkyl" in Alkoxy, Alkoxyalkyl. Alkylamino und Alkoxycarbonyl stehen für einen linearen oder verzweigten Alkylrest mit 1 bis 6 Kohlenstoffatomen, bevorzugt 1 bis 4 Kohlenstoffatomen, beispielhaft und vorzugsweise für Methyl, Ethyl, n-Propyl, iso-Propyl, n-Butyl, sec-Butyl, tert-Butyl, n-Pentyl und n-Hexyl.

Alkoxy steht beispielhaft und vorzugsweise für Methoxy, Ethoxy, n-Propoxy, iso-Propoxy, n-Butoxy und tert-Butoxy.

Alkoxyalkyl steht beispielhaft und vorzugsweise für Methoxymethyl, Ethoxymethyl, n-Propoxymethyl, iso-Propoxymethyl, n-Butoxymethyl, tert-Butoxymethyl, Methoxyethyl, Ethoxyethyl, n-Propoxyethyl, isoPropoxyethyl, n-Butoxyethyl und tert-Butoxyethyl.

N-Heterocyclus in der Definition der Reste R¹ und R² steht für einen gesättigten oder teilweise ungesättigten monocyclischen Rest mit 4 bis 7 Ringatomen mit einem N-Heteroatom und bis zu 3 weiteren Heteroatomen und/oder Heterogruppen aus der Reihe S, O, N, SO und SO₂, wobei ein Stickstoffatom auch ein N-Oxid bilden kann, beispielhaft und vorzugsweise für Azetidin, Pyrrolidin, Piperidin, Azepan, Piperazin, Morpholin, Thiomorpholin, 1-Oxidothiomorpholin und 1,1-Dioxidothiomorpholin, besonders bevorzugt Azetidin, Pyrrolidin, Morpholin, und 1,1-Dioxidothiomorpholin.

Heterocyclus in der Definition der Reste R¹ und R², der ein gemeinsames Kohlenstoffatom mit dem N-Heterocyclus hat, an den er gebunden ist, steht für einen gesättigten oder teilweise ungesättigten monocyclischen Rest mit 4 bis 6 Ringatomen und bis zu 4 Heteroatomen und/oder Heterogruppen aus der Reihe S, O, N, SO und SO₂, wobei ein Stickstoffatom auch ein N-Oxid bilden kann, beispielhaft und vorzugsweise für Azetidin, Oxetan, Thietan, Pyrrolidin, Tetrahydrofuran, Piperidin, Morpholin, Thiomorpholin, Piperazin, Tetrahydropyran und 1,1-Dioxidothietan, besonders bevorzugt Azetidin und Oxetan und noch mehr bevorzugt für Oxetan.

Halogen steht für Fluor, Chlor, Brom und Jod, vorzugsweise für Fluor und Chlor.

Bevorzugt sind Verbindungen der Formel (I), in welcher
- R¹: für C₁-C₆-Alkyl oder C₃-C₅-Cycloalkyl steht,
wobei Alkyl substituiert ist mit 1 bis 2 Substituenten, unabhängig voneinander ausgewählt aus der Gruppe bestehend aus Hydroxy und C₁-C₄-Alkoxy,
und
- R²: für Wasserstoff oder C₁-C₄-Alkyl steht,
oder
R¹ und R² zusammen mit dem Stickstoffatom an das sie gebunden sind einen 4- bis 7-gliedrigen N-Heterocyclus bilden,
wobei der N-Heterocyclus substituiert sein kann mit 1 bis 3 Substituenten, unabhängig voneinander ausgewählt aus der Gruppe bestehend aus Oxo, Hydroxy, Monofluormethyl, Difluormethyl, Trifluormethyl, Hydroxycarbonyl, tert-Butoxycarbonyl, Aminocarbonyl, C₁-C₄-Alkyl, C₁-C₄-Alkoxy und Halogen,
   oder
wobei der N-Heterocyclus zwei Substituenten aufweisen kann, die zusammen mit dem Kohlenstoffatom des N-Heterocyclus, an das sie gemeinsam gebunden sind, einen 4- bis 6-gliedrigen Heterocyclus bilden,
   worin dieser Heterocyclus seinerseits substituiert sein kann mit 1 bis 3 Substituenten, unabhängig voneinander ausgewählt aus der Gruppe bestehend aus Oxo, Methyl und Ethyl,

   - R³: für Wasserstoff, Fluor, Methoxy oder Ethoxy steht,
   und
   - R⁴: für Wasserstoff, Fluor, Methoxy oder Ethoxy steht,
   und ihre Salze, ihre Solvate und die Solvate ihrer Salze.

Bevorzugt sind Verbindungen der Formel (I), in welcher
- R¹: für C₂-C₆-Alkyl steht,
wobei Alkyl substituiert ist mit einem Substituenten ausgewählt aus der Gruppe bestehend aus Hydroxy, Methoxy und Ethoxy
und
- R²: für Wasserstoff steht oder

R¹ und R² zusammen mit dem Stickstoffatom an das sie gebunden sind ein Azetidin, Pyrrolidin, Piperidin, Azepan, Piperazin, Morpholin, Thiomorpholin, 1-Oxidothiomorpholin oder 1,1-Dioxidothiomorpholin bilden,
   wobei Azetidin, Pyrrolidin, Piperidin, Azepan, Piperazin, Morpholin, Thiomorpholin, 1-Oxidothiomorpholin und 1,1-Dioxidothiomorpholin substituiert sein können mit 1 bis 2 Substituenten, unabhängig voneinander ausgewählt aus der Gruppe bestehend aus Hydroxy, Trifluormethyl, Hydroxycarbonyl, C₁-C₃-Alkyl, Methoxy und Methoxymethyl,
      oder
   wobei Azetidin, Pyrrolidin, Piperidin, Azepan, Piperazin und Morpholin zwei Substituenten aufweisen können, die zusammen mit dem Kohlenstoffatom des Azetidins, Pyrrolidins, Piperidins, Azepans, Piperazins und Morpholins, an das sie gemeinsam gebunden sind, ein Azetidin, Oxetan oder 1,1-Dioxidothietan bilden,
      worin dieses Azetidin, Oxetan und 1,1-Dioxidothietan seinerseits substituiert sein kann mit 1 bis 2 Substituenten, unabhängig voneinander ausgewählt aus der Gruppe bestehend aus Methyl und Ethyl,

- R³: für Wasserstoff steht,
und
- R⁴: für Wasserstoff, Fluor oder Methoxy steht,
oder
- R³: für Wasserstoff, Fluor oder Methoxy steht,
und
- R⁴: für Wasserstoff steht,
und ihre Salze, ihre Solvate und die Solvate ihrer Salze.

Bevorzugt sind Verbindungen der Formel (I), in welcher
- R¹: für C₂-C₄-Alkyl steht,
wobei Alkyl substituiert ist mit einem Substituenten ausgewählt aus der Gruppe bestehend aus Hydroxy und Methoxy,
und
- R²: für Wasserstoff steht,
oder
R¹ und R² zusammen mit dem Stickstoffatom an das sie gebunden sind ein Azetidin, Pyrrolidin, Morpholin oder 1,1-Dioxidothiomorpholin bilden,
wobei Azetidin, Pyrrolidin, Morpholin und 1,1-Dioxidothiomorpholin substituiert sein können mit 1 bis 2 Substituenten, unabhängig voneinander ausgewählt aus der Gruppe bestehend aus, Hydroxycarbonyl, Methyl, Trifluormethyl, Methoxy und Methoxymethyl,
   oder
R¹ und R² zusammen mit dem Stickstoffatom an das sie gebunden sind ein Azetidin bilden,
wobei das Azetidin zwei Substituenten aufweisen kann, die zusammen mit dem Kohlenstoffatom des Azetidins, an das sie gemeinsam gebunden sind, ein Oxetan oder 1,1-Dioxidothietan bilden,
R³ für Wasserstoff, Fluor oder Methoxy steht, und
- R⁴: für Wasserstoff steht,
oder
- R³: für Wasserstoff steht,
und
- R⁴: für Wasserstoff, Fluor oder Methoxy steht,
und ihre Salze, ihre Solvate und die Solvate ihrer Salze.

Bevorzugt sind Verbindungen der Formel (I), in welcher
- R¹: für C₂-C₄-Alkyl steht,
wobei Alkyl substituiert ist mit einem Substituenten ausgewählt aus der Gruppe bestehend aus Hydroxy und Methoxy,
und
- R²: für Wasserstoff steht,
oder
R¹ und R² zusammen mit dem Stickstoffatom an das sie gebunden sind ein Azetidin, Pyrrolidin, Morpholin oder 1,1-Dioxidothiomorpholin bilden,
wobei Azetidin, Pyrrolidin, Morpholin und 1,1-Dioxidothiomorpholin substituiert sein können mit 1 bis 2 Substituenten, unabhängig voneinander ausgewählt aus der Gruppe bestehend aus Hydroxycarbonyl und Methyl,
oder
R¹ und R² zusammen mit dem Stickstoffatom an das sie gebunden sind ein Azetidin bilden,
wobei das Azetidin zwei Substituenten aufweisen kann, die zusammen mit dem Kohlenstoffatom des Azetidins, an das sie gemeinsam gebunden sind, ein Oxetan bilden,
R³ für Wasserstoff steht,
und
- R⁴: für Wasserstoff, Fluor oder Methoxy steht,
oder
- R³: für Wasserstoff, Fluor oder Methoxy steht,
und
- R⁴: für Wasserstoff steht,
und ihre Salze, ihre Solvate und die Solvate ihrer Salze.

Bevorzugt sind Verbindungen der Formel (I), in welcher
R¹ und R² zusammen mit dem Stickstoffatom an das sie gebunden sind ein Azetidin bilden,
   wobei das Azetidin zwei Substituenten aufweist, die zusammen mit dem Kohlenstoffatom des Azetidins, an das sie gemeinsam gebunden sind, ein Oxetan bilden,
R³ für Wasserstoff steht,
und
- R⁴: für Wasserstoff steht,
und ihre Salze, ihre Solvate und die Solvate ihrer Salze.

Bevorzugt sind Verbindungen der Formel (I), in welcher
- R¹: für C₁-C₆-Alkyl steht,
wobei Alkyl substituiert ist mit 1 bis 2 Substituenten, unabhängig voneinander ausgewählt aus der Gruppe bestehend aus Hydroxy, C₁-C₄-Alkoxy und Cycloalkyloxy,
und
- R²: für Wasserstoff oder C₁-C₄-Alkyl steht,
oder
R¹ und R² zusammen mit dem Stickstoffatom an das sie gebunden sind einen 4- bis 7-gliedrigen N-Heterocyclus bilden,
wobei der N-Heterocyclus substituiert sein kann mit 1 bis 3 Substituenten, unabhängig voneinander ausgewählt aus der Gruppe bestehend aus Oxo, Hydroxy, Monofluormethyl, Difluormethyl, Trifluormethyl, Hydroxycarbonyl, tert-Butoxycarbonyl, Aminocarbonyl, C₁-C₄-Alkyl, C₁-C₄-Alkoxy und Halogen,
   oder
wobei der N-Heterocyclus zwei Substituenten aufweisen kann, die zusammen mit dem Kohlenstoffatom des N-Heterocyclus, an das sie gemeinsam gebunden sind, einen 4- bis 6-gliedrigen Heterocyclus bilden,
   worin dieser Heterocyclus seinerseits substituiert sein kann mit 1 bis 3 Substituenten, unabhängig voneinander ausgewählt aus der Gruppe bestehend aus Oxo, Methyl und Ethyl,
R³ für Wasserstoff, Fluor, Methoxy oder Ethoxy steht,
und
- R⁴: für Wasserstoff, Fluor, Methoxy oder Ethoxy steht,
und ihre Salze, ihre Solvate und die Solvate ihrer Salze.

Bevorzugt sind Verbindungen der Formel (I), in welcher
- R¹: für C₂-C₆-Alkyl steht,
wobei Alkyl substituiert ist mit einem Substituenten ausgewählt aus der Gruppe bestehend aus Hydroxy, Methoxy und Ethoxy,
und
- R²: für Wasserstoff steht,
oder
R¹ und R² zusammen mit dem Stickstoffatom an das sie gebunden sind ein Azetidin, Pyrrolidin, Piperidin, Azepan, Piperazin, Morpholin, Thiomorpholin, 1-Oxidothiomorpholin oder 1,1-Dioxidothiomorpholin bilden,
wobei Azetidin, Pyrrolidin, Piperidin, Azepan, Piperazin, Morpholin, Thiomorpholin, 1-Oxidothiomorpholin und 1,1-Dioxidothiomorpholin substituiert sein können mit 1 bis 2 Substituenten, unabhängig voneinander ausgewählt aus der Gruppe bestehend aus, Hydroxy, Hydroxycarbonyl, C₁-C₃-Alkyl und Methoxy,
   oder
wobei Azetidin, Pyrrolidin, Piperidin, Azepan, Piperazin und Morpholin zwei Substituenten aufweisen können, die zusammen mit dem Kohlenstoffatom des Azetidins, Pyrrolidins, Piperidins, Azepans, Piperazins und Morpholins, an das sie gemeinsam gebunden sind, ein Azetidin oder Oxetan bilden,
   worin dieses Azetidin und Oxetan seinerseits substituiert sein kann mit 1 bis 2 Substituenten, unabhängig voneinander ausgewählt aus der Gruppe bestehend aus Methyl und Ethyl,
R³ für Wasserstoff steht,
und
- R⁴: für Wasserstoff, Fluor oder Methoxy steht,
oder
- R³: für Wasserstoff, Fluor oder Methoxy steht,
und
- R⁴: für Wasserstoff steht,
und ihre Salze, ihre Solvate und die Solvate ihrer Salze.

Bevorzugt sind auch Verbindungen der Formel (I), in welcher
R¹ für C₂-C₆-Alkyl steht,
wobei Alkyl substituiert ist mit einem Substituenten ausgewählt aus der Gruppe bestehend aus Hydroxy, Methoxy und Ethoxy,
und
R² für Wasserstoff steht,
oder
R¹ und R² zusammen mit dem Stickstoffatom an das sie gebunden sind ein Azetidin, Pyrrolidin, Piperidin, Azepan, Piperazin, Morpholin, Thiomorpholin, 1-Oxidothiomorpholin oder 1,1-Dioxidothiomorpholin bilden,
wobei Azetidin, Pyrrolidin, Piperidin, Azepan, Piperazin, Morpholin, Thiomorpholin, 1-Oxidothiomorpholin und 1,1-Dioxidothiomorpholin substituiert sein können mit 1 bis 2 Substituenten, unabhängig voneinander ausgewählt aus der Gruppe bestehend aus, Hydroxy, Hydroxycarbonyl, C₁-C₃-Alkyl und Methoxy,
   oder
wobei Azetidin, Pyrrolidin, Piperidin und Azepan, zwei Substituenten aufweisen können, die zusammen mit dem Kohlenstoffatom des Azetidins, Pyrrolidins, Piperidins und Azepans, an das sie gemeinsam gebunden sind, ein Azetidin oder Oxetan bilden,
   worin dieses Azetidin und Oxetan seinerseits substituiert sein kann mit 1 bis 2 Substituenten, unabhängig voneinander ausgewählt aus der Gruppe bestehend aus, Methyl und Ethyl,
R³ für Wasserstoff steht,
und
- R⁴: für Wasserstoff, Fluor oder Methoxy steht,
oder
- R³: für Wasserstoff, Fluor oder Methoxy steht,
und
- R⁴: für Wasserstoff steht,
und ihre Salze, ihre Solvate und die Solvate ihrer Salze.

Bevorzugt sind auch Verbindungen der Formel (I), in welcher
- R¹: für C₂-C₄-Alkyl steht,
wobei Alkyl substituiert ist mit einem Substituenten ausgewählt aus der Gruppe bestehend aus Hydroxy und Methoxy,
und
- R²: für Wasserstoff steht,
oder
R¹ und R² zusammen mit dem Stickstoffatom an das sie gebunden sind ein Azetidin, Pyrrolidin, Morpholin oder 1,1-Dioxidothiomorpholin bilden,
wobei Azetidin, Pyrrolidin, Morpholin und 1,1-Dioxidothiomorpholin substituiert sein können mit 1 bis 2 Substituenten, unabhängig voneinander ausgewählt aus der Gruppe bestehend aus Oxo, Hydroxy, Hydroxycarbonyl und Methyl,
oder
R¹ und R² zusammen mit dem Stickstoffatom an das sie gebunden sind ein Azetidin bilden,
wobei das Azetidin zwei Substituenten aufweisen kann, die zusammen mit dem Kohlenstoffatom des Azetidins, an das sie gemeinsam gebunden sind, ein Oxetan bilden,
R³ für Wasserstoff steht,
und
- R⁴: für Wasserstoff steht,
und ihre Salze, ihre Solvate und die Solvate ihrer Salze.

Bevorzugt sind auch Verbindungen der Formel (I), in welcher
- R¹: für C₂-C₆-Alkyl steht,
wobei Alkyl substituiert ist mit einem Substituenten ausgewählt aus der Gruppe bestehend aus Hydroxy, Methoxy und Ethoxy,
und
- R²: für Wasserstoff steht,
und ihre Salze, ihre Solvate und die Solvate ihrer Salze.

Bevorzugt sind auch Verbindungen der Formel (I), in welcher
R¹ und R² zusammen mit dem Stickstoffatom an das sie gebunden sind ein Azetidin, Pyrrolidin, Morpholin oder 1,1-Dioxidothiomorpholin bilden,
wobei Azetidin, Pyrrolidin, Morpholin und 1,1-Dioxidothiomorpholin substituiert sein können mit 1 bis 2 Substituenten, unabhängig voneinander ausgewählt aus der Gruppe bestehend aus Oxo, Hydroxy, Hydroxycarbonyl und Methyl,
oder
R¹ und R² zusammen mit dem Stickstoffatom an das sie gebunden sind ein Azetidin bilden,
wobei das Azetidin zwei Substituenten aufweisen kann, die zusammen mit dem Kohlenstoffatom des Azetidins, an das sie gemeinsam gebunden sind, ein Oxetan bilden,
und ihre Salze, ihre Solvate und die Solvate ihrer Salze.

Bevorzugt sind auch Verbindungen der Formel (I), in welcher R² für Wasserstoff steht.

Bevorzugt sind auch Verbindungen der Formel (I), in welcher R¹ und R² zusammen mit dem Stickstoffatom an das sie gebunden sind für 2-Oxa-6-azaspiro[3.3]hept-6-yl stehen.

Bevorzugt sind auch Verbindungen der Formel (I), in welcher R¹ und R² zusammen mit dem Stickstoffatom an das sie gebunden sind für 1,1-Dioxidothiomorpholin-4-yl stehen.

Bevorzugt sind auch Verbindungen der Formel (I), in welcher R³ für Wasserstoff steht.

Bevorzugt sind auch Verbindungen der Formel (I), in welcher R⁴ für Wasserstoff steht.

Bevorzugt sind auch Verbindungen der Formel (I), in welcher R³ und R⁴ für Wasserstoff stehen.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Gegenstand der Erfindung ist weiterhin ein Verfahren zur Herstellung der Verbindungen der Formel (I), oder ihrer Salze, ihrer Solvate oder der Solvate ihrer Salze, wobei
[A] Verbindungen der Formel (II) 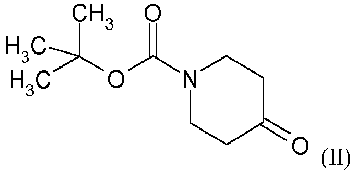 mit Verbindungen der Formel (III) 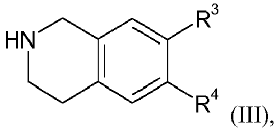 in welcher R³ und R⁴ die oben angegebenen Bedeutungen aufweisen, in Gegenwart eines Reduktionsmittels zu Verbindungen der Formel (IV) 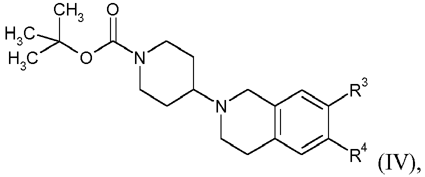 in welcher R³ und R⁴ die oben angegebenen Bedeutungen aufweisen,
   umgesetzt werden,
   oder
[B] Verbindungen der Formel (IV) 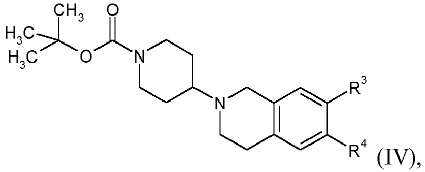 in welcher R³ und R⁴ die oben angegebenen Bedeutungen aufweisen
   in Gegenwart einer Säure zu Verbindungen der Formel (V) 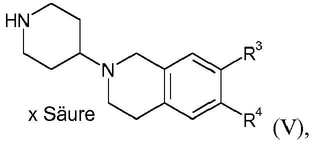 in welcher R³ und R⁴ die oben angegebenen Bedeutungen aufweisen,
   umgesetzt werden
   oder
[C] Verbindungen der Formel (VI) 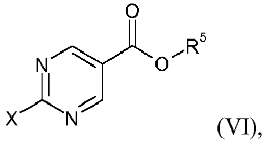 in welcher
   - X: für Halogen, bevorzugt Fluor, Chlor oder Brom, oder Sulfonylmethan und
   - R⁵: für C₁-C₄-Alkyl, bevorzugt Methyl oder Ethyl, steht,
   in Gegenwart einer Base mit Verbindungen der Formel (VII) 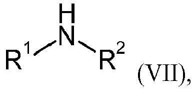 in welcher R¹ und R² die oben angegebene Bedeutung aufweisen,
   zu Verbindungen der Formel (VIII) 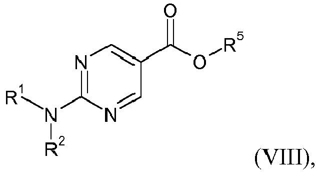 in welcher R¹, R² und R⁵ die oben angegebene Bedeutung aufweisen,
   umgesetzt werden
   oder
[D] Verbindungen der Formel (IX) 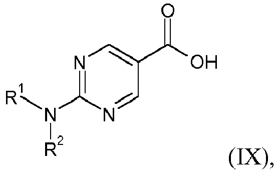 in welcher R¹ und R² die oben angegebene Bedeutung aufweisen,
   mit Verbindungen der Formel (V) 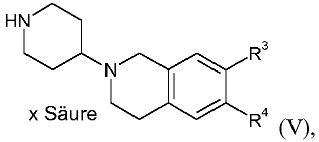 in welcher R³ und R⁴ die oben angegebenen Bedeutungen aufweisen,
   in Gegenwart eines Dehydratisierungsreagenzes,
   zu Verbindungen der Formel (I) umgesetzt werden.

Die Umsetzung nach Verfahren [A] erfolgt im Allgemeinen in inerten Lösungsmitteln, bevorzugt in einem Temperaturbereich von -20°C bis 60°C bei Normaldruck und gegebenenfalls in Gegenwart einer Base.

Inerte Lösungsmittel sind beispielsweise Alkohole wie Methanol, Ethanol, n-Propanol oder Isopropanol, oder Ether wie Dietylether, Dioxan oder Tetrahydrofuran, oder Dimethylformamid, oder Essigsäure bzw. Eisessig, oder Dichlormethan, Trichlormethan oder 1,2-Dichlorethan. Ebenso ist es möglich, Gemische der genannten Lösungsmittel einzusetzen. Bevorzugt sind Dichlormethan oder Tetrahydrofuran.

Basen sind beispielsweise organische Basen wie Trialkylamine z.B. Triethylamin, *N-*Methylmorpholin, *N-*Methylpiperidin, 4-Dimethylaminopyridin oder Diisopropylethylamin, bevorzugt ist Diisopropylethylamin.

Reduktionsmittel sind beispielsweise Natriumborhydrid, Lithiumborhydrid, Natriumcyanoborhydrid, Lithiumaluminiumhydrid, Natrium-bis-(2-methoxyethoxy)aluminium-hydrid, Natriumtriacetoxyborhydrid oder Boran-Tetrahydrofuran, bevorzugt ist Natriumtriacetoxyborhydrid.

Die Verbindungen der Formeln (II) und (III) sind bekannt oder lassen sich nach bekannten Verfahren aus den entsprechenden Ausgangsverbindungen synthetisieren.

Alternativ zu dem oben beschriebenen Verfahren [A] kann die Herstellung der Verbindungen der Formel (IV) auch ein Verfahren aufweisen, wobei
[E] Verbindungen der Formel (II) 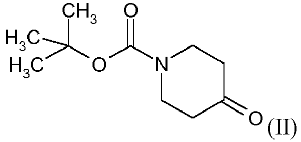 mit Verbindungen der Formel (III) 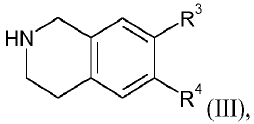 in welcher R³ und R⁴ die oben angegebenen Bedeutungen aufweisen,
   zu Verbindungen der Formel (IVa) 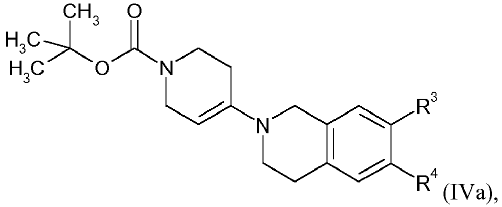 in welcher R³ und R⁴ die oben angegebenen Bedeutungen aufweisen,
   umgesetzt werden,
   oder
[F] Verbindungen der Formel (IVa) 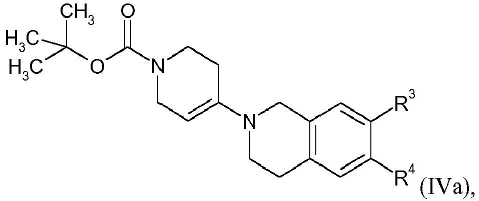 in welcher R³ und R⁴ die oben angegebenen Bedeutungen aufweisen,
   in Gegenwart eines Reduktionsmittels zu Verbindungen der Formel (IV) umgesetzt werden.

Reduktionsmittel in einer Umsetzung nach Verfahren [E] können beispielsweise Natriumborhydrid, Lithiumborhydrid, Natriumcyanoborhydrid, Lithiumaluminiumhydrid, Natrium-bis-(2-methoxyethoxy)aluminium-hydrid, Natriumtriacetoxyborhydrid, Boran-Tetrahydrofuran, oder Wasserstoff in Gegenwart von Palladiumkatalysatoren sein.

Die Umsetzung nach Verfahren [B] erfolgt im Allgemeinen in inerten Lösungsmitteln, bevorzugt in einem Temperaturbereich von -20°C bis 60°C bei Normaldruck.

Inerte Lösungsmittel sind beispielsweise Alkohole wie Methanol, Ethanol, n-Propanol oder Isopropanol, oder Ether wie Dietylether, Dioxan oder Tetrahydrofuran, oder Dimethylformamid, oder Dichlormethan, Trichlormethan oder 1,2-Dichlorethan. Ebenso ist es möglich, Gemische der genannten Lösungsmittel einzusetzen. Bevorzugt ist Dichlormethan.

Säuren sind beispielsweise Chlorwasserstoff und Trifluoressigsäure, bevorzugt ist Chlorwasserstoff. Bevorzugt werden diese Säuren in einem inerten Lösungsmittel gelöst zugegeben. Ein bevorzugtes Lösungsmittel hierfür ist Dioxan.

Die Umsetzung nach Verfahren [C] erfolgt im Allgemeinen in inerten Lösungsmitteln, bevorzugt in einem Temperaturbereich von 0°C bis 80°C bei Normaldruck.

Inerte Lösungsmittel sind beispielsweise Alkohole wie Isopropanol oder Ether wie Dietylether, Dioxan, Tetrahydrofuran oder N-Methylmorpholinon, oder Dimethylformamid, oder Dichlormethan, Trichlormethan, 1,2-Dichlorethan, oder Acetonitril. Bevorzugt sind Acetonitril und N-Methylmorpholinon. Ebenso ist es möglich, Gemische der genannten Lösungsmittel einzusetzen.

Basen sind beispielsweise Alkalicarbonate, wie z.B. Natrium-, Kalium- oder Caesiumcarbonat, oder Natrium-, Kalium- oder Caesium-hydrogencarbonat, oder organische Basen wie Trialkylamine z.B. Triethylamin, *N-*Methylmorpholin, *N-*Methylpiperidin, 4-Dimethylaminopyridin oder Diisopropylethylamin, bevorzugt sind Kaliumcarbonat und Natriumcarbonat.

Die Verbindungen der Formeln (VI) und (VII) sind bekannt oder lassen sich nach bekannten Verfahren aus den entsprechenden Ausgangsverbindungen synthetisieren.

Die Umsetzung nach Verfahren [D] erfolgt im Allgemeinen in inerten Lösungsmitteln, gegebenenfalls in Gegenwart einer Base, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der genannten Lösungsmittel einzusetzen. Besonders bevorzugt ist Acetonitril.

Als Dehydratisierungsreagenzien eignen sich hierbei beispielsweise Carbodiimide wie z.B. *N,N'-*Diethyl-, *N*,*N*,'-Dipropyl-, *N*,*N*'-Diisopropyl-, *N*,*N*'-Dicyclohexylcarbodiimid, *N*-(3-Dimethylaminoiso-propyl)-*N*'-ethylcarbodiimid-hydrochlorid (EDC), *N-*Cyclohexylcarbodiimid-*N*'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-*tert*-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid (T3P), oder Isobutylchloroformat, oder Bis-(2-oxo-3-oxazolidinyl)-phosphorylchlorid oder Benzotriazolyloxy-tri(dimethylamino)phosphoniumhexafluorophosphat, oder *O-*(Benzotriazol-1-yl)-*N*,*N*,*N*,*N*'-tetra-methyluronium-hexafluorophosphat (HBTU), 2-(2-Oxo-1-(2H)-pyridyl)-1,1,3,3-tetramethyluroniumtetrafluoro-borat (TPTU) oder *O*-(7-Azabenzotriazol-1-yl)-*N*,*N*,*N'*,*N*'-tetramethyl-uroniumhexafluorophosphat (HATU), oder 1-Hydroxybenzotriazol (HOBt), oder Benzotriazol-1-yloxytris(dimethylamino)-phosphoniumhexafluorophosphat (BOP), oder *N-*Hydroxysuccinimid, oder Mischungen aus diesen, mit Basen.

Basen sind beispielsweise Alkalicarbonate, wie z.B. Natrium-, Kalium- oder -, Caesiumcarbonat oder -hydrogencarbonat, oder organische Basen wie Trialkylamine z.B. Triethylamin, *N-*Methylmorpholin, *N-*Methylpiperidin, 4-Dimethylaminopyridin oder Diisopropylethylamin, bevorzugt ist Diisopropylethylamin.

Vorzugsweise wird die Kondensation mit Propanphosphonsäureanhydrid durchgeführt.

Die Verbindungen der Formel (IX) können hergestellt werden, indem bei Verbindungen der Formel (VIII) der Carbonsäureester verseift wird.

Die Verseifung erfolgt im Allgemeinen in inerten Lösungsmitteln in Gegenwart zumindest einer Base, bevorzugt in einem Temperaturbereich von 0°C bis 90°C bei Normaldruck.

Basen sind beispielsweise Alkalihydroxide, wie z.B. Lithium- oder Natriumhydroxid, die jeweils in Form einer wässrigen Lösung verwendet werden können. Bevorzugt sind wässrige Lösungen von Lithiumhydroxid und Natriumhydroxid.

Inerte Lösungsmittel sind beispielsweise polare Lösungsmittel wie Alkohole, beispielsweise Methanol, Ethanol, n-Propanol oder Isopropanol, oder Ether wie Dietylether, Dioxan, Tetrahydrofuran oder N-Methylmorpholin. Ebenso ist es möglich, Gemische der genannten Lösungsmittel einzusetzen. Bevorzugt sind Dioxan, Ethanol und Gemische von Tetrahydrofuran und Methanol.

Ferner kann die erfindungsgemäße Herstellung der Verbindungen der Formel (I) auch ein Verfahren aufweisen, wobei
[G] Verbindungen der Formel (IX) 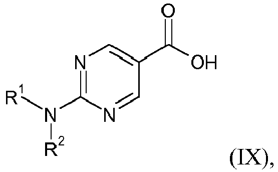 in welcher R¹ und R² die oben angegebene Bedeutung aufweisen,
   mit 4-Piperidinon
   zu Verbindungen der Formel (X) 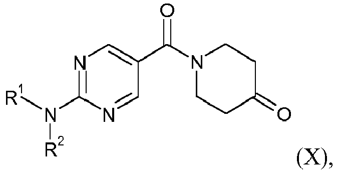 in welcher R¹ und R² die oben angegebenen Bedeutungen aufweisen,
   umgesetzt werden,
   oder
[H] Verbindungen der Formel (X) 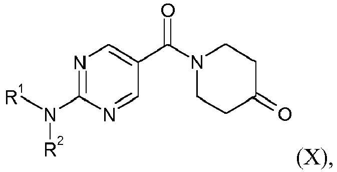 in welcher R¹ und R² die oben angegebenen Bedeutungen aufweisen,
   mit Verbindungen der Formel (III) 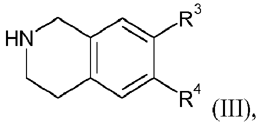 in welcher R³ und R⁴ die oben angegebenen Bedeutungen aufweisen,
   in Gegenwart eines Reduktionsmittels zu Verbindungen der Formel (I) umgesetzt werden.
   Die Umsetzung nach Verfahren [G] erfolgt dabei analog zu Umsetzungen nach Verfahren [D].
   Reduktionsmittel in einer Umsetzung nach Verfahren [H] können beispielsweise Natriumborhydrid, Lithiumborhydrid, Natriumcyanoborhydrid, Lithiumaluminiumhydrid, Natrium-bis-(2-methoxyethoxy)aluminium-hydrid, Natriumtriacetoxyborhydrid oder Boran-Tetrahydrofuran sein.

Ferner kann die erfindungsgemäße Herstellung der Verbindungen der Formel (I) auch ein Verfahren aufweisen, wobei
[I] Verbindungen der Formel (XI) 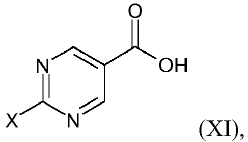 in welcher
   - X: für Halogen, bevorzugt Fluor, Chlor oder Brom, oder Sulfonylmethan steht,
   mit Verbindungen der Formel (V) 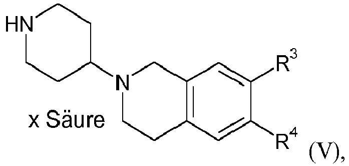 in welcher R³ und R⁴ die oben angegebenen Bedeutungen aufweisen,
   in Gegenwart eines Dehydratisierungsreagenzes zu Verbindungen der Formel (XII) 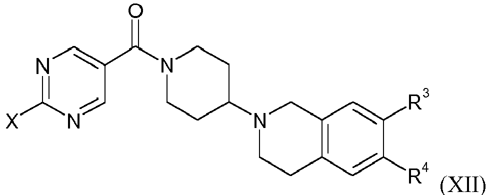 in welcher
   R³ und R⁴ die oben angegebenen Bedeutungen aufweisen und
   X für Halogen, bevorzugt Fluor, Chlor oder Brom, oder Sulfonylmethan steht, umgesetzt werden,
   oder
[J] Verbindungen der Formel (XII) 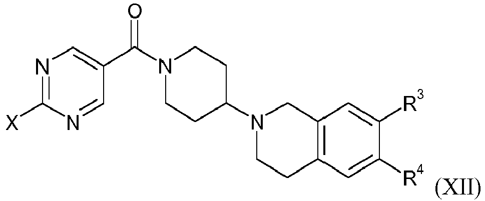 in welcher
   R³ und R⁴ die oben angegebenen Bedeutungen aufweisen und
   X für Halogen, bevorzugt Fluor, Chlor oder Brom, oder Sulfonylmethan steht,
   mit Verbindungen der Formel (VII) 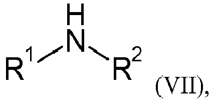 in welcher R¹ und R² die oben angegebene Bedeutung aufweisen,
   zu Verbindungen der Formel (I) umgesetzt werden.

Die in der Umsetzung nach Verfahren [I] angegebenen Dehydratisierungsreagenzien können beispielsweise solche sein, wie sie im Zusammenhang mit den Umsetzungen nach Verfahren [D] beschrieben wurden.

Die in der Umsetzung nach Verfahren [J] angegebenen Reduktionsmittel können beispielsweise solche sein, wie sie im Zusammenhang mit den Umsetzungen nach Verfahren [A] beschrieben wurden.

Gegenstand der Erfindung ist ferner ein Verfahren zur Herstellung der Verbindungen der Formel (I), oder ihrer Salze, ihrer Solvate oder der Solvate ihrer Salze, wobei dieses Verfahren Umsetzungen nach den zuvor beschriebenen Verfahren, ausgewählt aus einer Gruppe umfassend die Kombinationen
[A] und [B],
[E], [F] und [B],
[C] und [D],
[A], [B] und [D],
[E], [F], [B] und [D],
[A], [B], [C] und [D], und
[E], [F], [B], [C] und [D],
aufweist.

Die Herstellung der Verbindungen der Formel (I) kann durch folgende Syntheseschemata verdeutlicht werden.

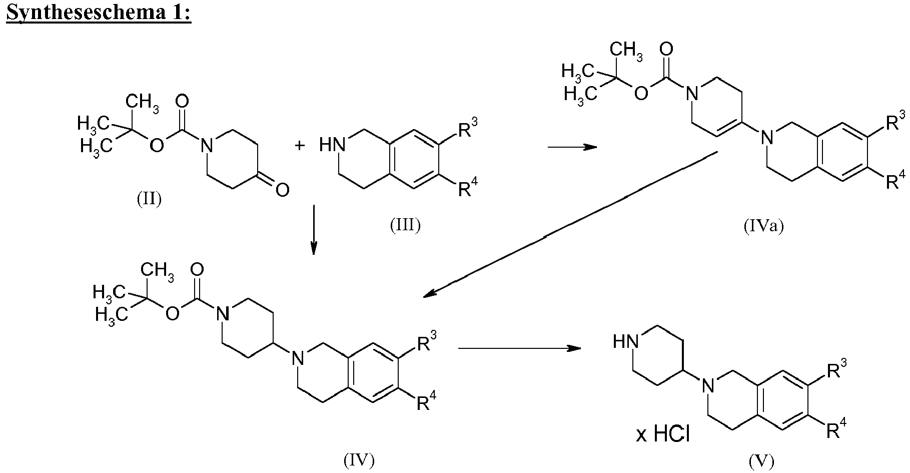

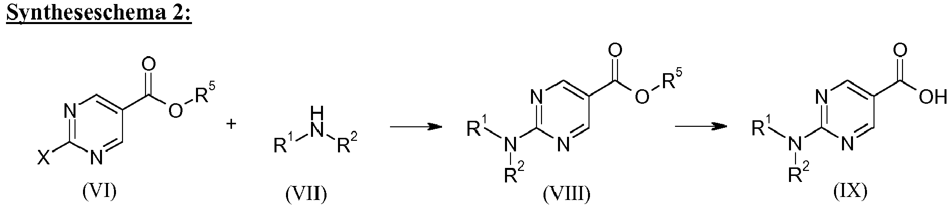

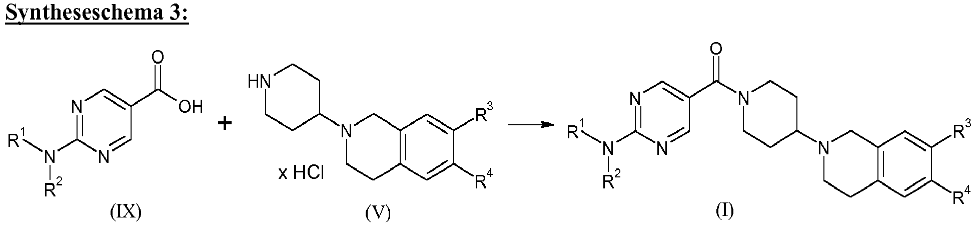

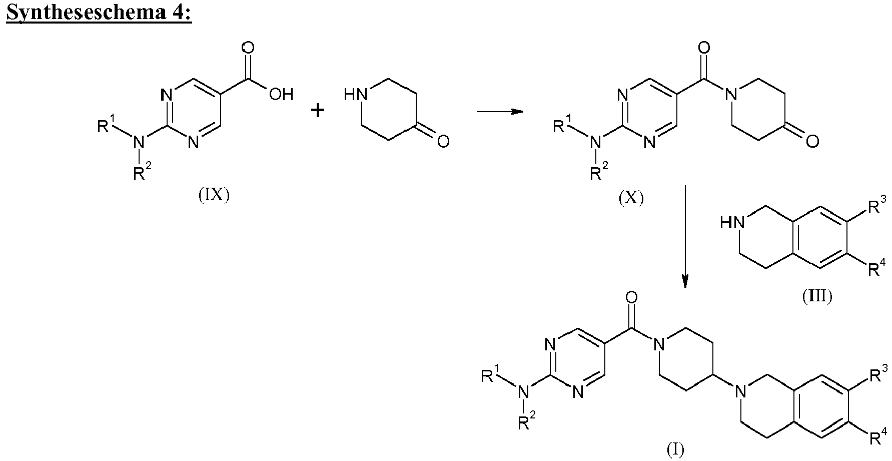

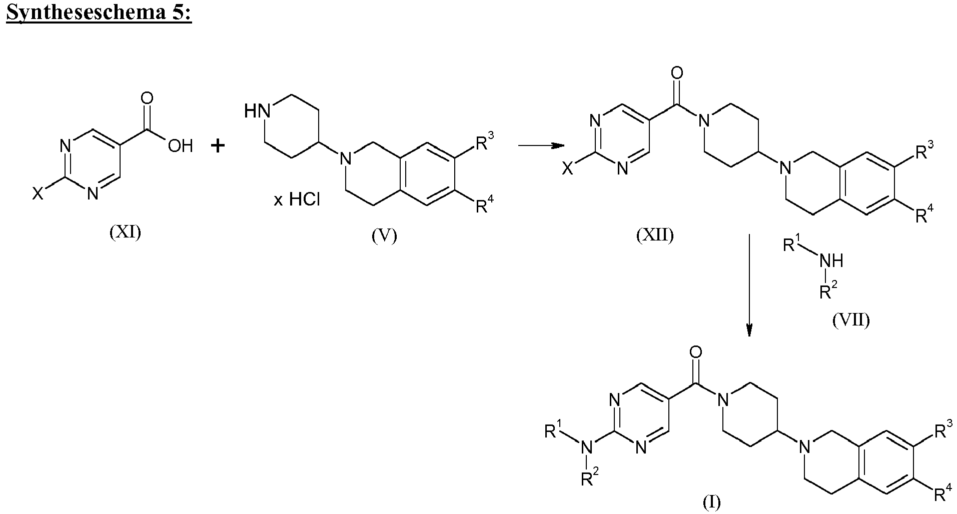

Gegenstand der Erfindung sind auch Verbindungen der Formel (VIII) oder (IX), 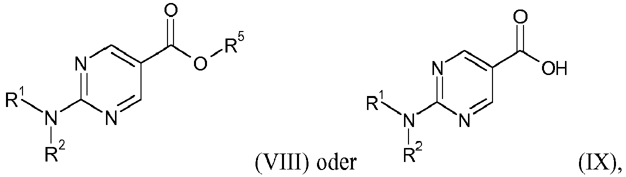 in welcher
R¹ und R² zusammen mit dem Stickstoffatom an das sie gebunden sind ein Azetidin bilden,
   wobei das Azetidin zwei Substituenten aufweist, die zusammen mit dem Kohlenstoffatom des Azetidins, an das sie gemeinsam gebunden sind, ein Oxetan bilden
und
- R⁵: für C₁-C₄-Alkyl, bevorzugt Methyl und Ethyl, steht,
und ihre Salze, ihre Solvate und die Solvate ihrer Salze.

Die erfindungsgemäßen Verbindungen zeigen ein nicht vorhersehbares, wertvolles pharmakologisches Wirkspektrum, einschließlich wertvoller pharmakokinetischer Eigenschaften. Es handelt sich dabei um selektive Adrenoreceptor α_{2C} Rezeptor Antagonisten, die zu einer Gefäßrelaxation und/oder zu einer Thrombozytenaggregationshemmung und/oder zu einer Blutdrucksenkung und/oder zu einer Steigerung des koronaren oder peripheren Blutflusses führen. Sie eigenen sich daher zur Behandlung und/oder Prophylaxe von Krankheiten, vorzugsweise von kardiovaskulären Erkrankungen, diabetischen Mikroangiopathien, diabetischen Geschwüre an den Extremitäten, insbesondere zur Förderung der Wundheilung von diabetischen Fußulzera, diabetischer Herzinsuffizienz, diabetischen koronaren mikrovaskulären Herzerkrankungen, peripheren und kardialen Gefäßerkrankungen, thromboembolischen Erkrankungen und Ischämien, peripheren Durchblutungsstörungen, Raynaud-Phänomen, CREST-Syndrom, Mikrozirkulationsstörungen, Claudicatio intermittens, und peripheren und autonomen Neuropathien bei Menschen und Tieren.

Insbesondere zeigen die erfindungsgemäßen Verbindungen eine krankheitsselektive Verbesserung des peripheren Blutflusses (Mikro und Makrozirkulation) unter pathophysiologisch veränderten Bedingungen wie z.B. in Folge von Diabetes oder Atherosklerose.

Die erfindungsgemäßen Verbindungen eigenen sich daher zur Verwendung als Arzneimittel zur Behandlung und/oder Prophylaxe von Krankheiten bei Menschen und Tieren.

Die erfindungsgemäßen Verbindungen eignen sich daher zur Behandlung von kardiovaskulären Erkrankungen wie beispielsweise zur Behandlung des Bluthochdrucks, der primären und/oder sekundären Prävention sowie Behandlung der Herzinsuffizienz, zur Behandlung stabiler und instabiler Angina pectoris, pulmonaler Hypertonie, peripheren und kardialen Gefäßerkrankungen (z.B periphere Verschlusskrankheit), Arrhythmien, zur Behandlung von thromboembolischen Erkrankungen und Ischämien wie Myokardinfarkt, Hirnschlag, transitorischen und ischämischen Attacken, peripheren Durchblutungsstörungen, zur Verhinderung von Restenosen wie nach Thrombolysetherapien, percutantransluminalen Angioplastien (PTA), percutan-transluminalen Koronarangioplastien (PTCA) und Bypass sowie zur Behandlung von Ischämiesyndrom, Atherosklerose, asthmatischen Erkrankungen, Krankheiten des Urogenitalsystems wie beispielsweise Prostatahypertrophie, erektile Dysfunktion, weibliche sexuelle Dysfunktion und Inkontinenz eingesetzt werden.

Außerdem können die erfindungsgemäßen Verbindungen zur Behandlung von primärem und sekundärem Raynaud-Phänomen, von Mikrozirkulationsstörungen, Claudicatio intermittens, peripheren und autonomen Neuropathien, diabetischen Mikroangiopathien, diabetischer Nephropathie, diabetischer Retinopathie, diabetischen Geschwüren an den Extremitäten, diabetischer erektiler Dysfunktion, CREST-Syndrom, Erythematose, Onychomykose, Tinnitus, Schwindelanfälle, Hörsturz, Morbus Meniere sowie von rheumatischen Erkrankungen verwendet werden.

Ferner eignen sich die erfindungsgemäßen Verbindungen zur Behandlung von Respiratory Distress-Syndromen und chronisch-obstruktiven Atemwegserkrankungen (COPD), von akuter und chronischer Niereninsuffizienz sowie zur Förderung der Wundheilung und hierbei insbesondere der diabetischen Wundheilung.

Außerdem eignen sich die erfindungsgemäßen Verbindungen zur Behandlung und/oder Prophylaxe von Begleit- und/oder Folgeerkrankungen von Diabetes mellitus. Beispiele für Begleit- und/oder Folgeerkrankungen von Diabetes mellitus sind diabetische Herzerkrankungen, wie beispielsweise diabetische koronare Herzerkrankungen, diabetische koronare mikrovaskuläre Herzerkrankungen (coronary microvascular disease, MVD), diabetische Herzinsuffizienz, diabetische Kardiomyopathie und Herzinfarkt, Bluthochdruck, diabetische Mikroangiopathie, diabetische Retinopathie, diabetische Neuropathie, Schlaganfall, diabetische Nephropathie, diabetische erektile Dysfunktion, diabetische Geschwüre an den Extremitäten und diabetisches Fußsyndrom. Die erfindungsgemäßen Verbindungen eignen sich außerdem zur Förderung der diabetischen Wundheilung, insbesondere zur Förderung der Wundheilung von diabetischen Fußulzera. Die Förderung der Wundheilung von diabetischen Fußulzera ist beispielsweise definiert als eine Verbesserung des Wundverschlusses.

Weiterhin eignen sich die erfindungsgemäßen Verbindungen auch zur Regulation der zerebralen Durchblutung und stellen wirkungsvolle Mittel zur Bekämpfung von Migräne dar. Auch eignen sie sich zur Prophylaxe und Bekämpfung der Folgen zerebraler Infarktgeschehen (Apoplexia cerebri) wie Schlaganfall, zerebraler Ischämien und des Schädel-Hirn-Traumas. Ebenso können die erfindungsgemäßen Verbindungen zur Bekämpfung von Schmerzzuständen eingesetzt werden.

Darüber hinaus können die erfindungsgemäßen Verbindungen auch zur Behandlung und/oder Prävention von mikro- und makrovaskulären Schädigungen (Vasculitis), Reperfusionsschäden, arteriellen sowie venösen Thrombosen, Ödemen, Krebserkrankungen (Hautkrebs, Liposarcome, Karzinome des Magen-Darm-Traktes, der Leber, Bauchspeicheldrüse, Lunge, Niere, Harnleiter, Prostata und des Genitaltraktes), von Erkrankungen des Zentralen Nervensystems und neurodegenerativen Störungen (Schlaganfall, Alzheimer'sche Krankheit, Parkinson'sche Krankheit, Demenz, Epilepsie, Depressionen, Multiple Sklerose, Schizophrenie), von Entzündungserkrankungen, Autoimmunerkrankungen (Morbus Crohn, Colitis ulcerosa, Lupus erythematodes, rheumatoide Arthritis, Asthma), Nierenerkrankungen (Glomerulonephritis), Schilddrüsenerkrankungen (Hyperthyreose), Hyperhidrose, Erkrankungen der Bauchspeicheldrüse (Pankreatitis), Leberfibrose, Hauterkrankungen (Psoriasis, Akne, Ekzeme, Neurodermitis, Dermatitis, Keratitis, Narbenbildung, Warzenbildung, Frostbeulen), Hauttransplantationen, viralen Erkrankungen (HPV, HCMV, HIV), Kachexie, Osteoporose, avaskulärer Knochennekrose, Gicht, Inkontinenz, zur Wundheilung, zur Wundheilung bei Patienten mit Sichelzellanämie, und zur Angiogenese eingesetzt werden.

Weiterer Gegenstand der vorliegenden Erfindung ist der Einsatz der erfindungsgemäßen Verbindungen zur Behandlung und/oder Prophylaxe von Erkrankungen, vorzugsweise von thromboembolischen Erkrankungen und/oder thromboembolischen Komplikationen.

Zu den "thromboembolischen Erkrankungen" im Sinne der vorliegenden Erfindung zählen insbesondere Erkrankungen wie Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie, Stentimplantation oder aortokoronarem Bypass, periphere arterielle Verschlusskrankheiten, Lungenembolien, tiefe venöse Thrombosen und Nierenvenenthrombosen, transitorische ischämische Attacken sowie thrombotischer und thromboembolischer Hirnschlag und pulmonale Hypertonie.

Die Substanzen eignen sich daher auch zur Prävention und Behandlung von kardiogenen Thromboembolien, wie beispielsweise Hirn-Ischämien, Schlaganfall und systemischen Thromboembolien und Ischämien, bei Patienten mit akuten, intermittierenden oder persistierenden Herzarrhythmien, wie beispielsweise Vorhofflimmern, und solchen, die sich einer Kardioversion unterziehen, ferner bei Patienten mit Herzklappen-Erkrankungen oder mit intravasalen Körpern, wie z. B. künstlichen Herzklappen, Kathetern, intraaortaler Ballongegenpulsation und Schrittmachersonden. Darüber hinaus sind die erfindungsgemäßen Verbindungen zur Behandlung der disseminierten intravasalen Gerinnung (DIC) geeignet.

Thromboembolische Komplikationen treten ferner auf bei mikroangiopathischen hämolytischen Anämien, extrakorporalen Blutkreisläufen, wie z. B. Hämodialyse, Hämofiltration, Herzunterstützungssystem und Kunstherz, sowie Herzklappenprothesen.

Besonders eignen sich die erfindungsgemäßen Verbindungen zur primären und/oder sekundären Prävention sowie Behandlung der Herzinsuffizienz.

Im Sinne der vorliegenden Erfindung umfasst der Begriff Herzinsuffizienz auch spezifischere oder verwandte Krankheitsformen wie Rechtsherzinsuffizienz, Linksherzinsuffizienz, Globalinsuffizienz, ischämische Kardiomyopathie, dilatative Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz.

Ganz besonders eignen sich die erfindungsgemäßen Verbindungen zur Behandlung und/oder Prophylaxe von kardiovaskulären Erkrankungen, insbesondere Herzinsuffizienz, und/oder Durchblutungsstörungen und Mikroangiopathien bei Diabetes.

Die erfindungsgemäßen Verbindungen eignen sich auch zur primären und/oder sekundären Prävention sowie Behandlung der oben genannten Erkrankungen bei Kindern.

Weiterer Gegenstand der vorliegenden Erfindung sind die erfindungsgemäßen Verbindungen zur Verwendung in einem Verfahren zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer therapeutisch wirksamen Menge einer erfindungsgemäßen Verbindung.

Weiterer Gegenstand der vorliegenden Erfindung sind Adrenorezeptor α2C Rezeptor Antagonisten zur Verwendung in einem Verfahren zur Behandlung und/oder Prophylaxe von Begleit- und/oder Folgeerkrankungen von Diabetes mellitus, diabetischen Herzerkrankungen, diabetischen koronaren Herzerkrankungen, diabetischen koronaren mikrovaskulären Herzerkrankungen, diabetischer Herzinsuffizienz, diabetischer Kardiomyopathie und Herzinfarkt, diabetischer Mikroangiopathie, diabetischer Retinopathie, diabetischer Neuropathie, diabetischer Nephropathie, diabetischer erektiler Dysfunktion, diabetischen Geschwüren an den Extremitäten, diabetischen Fußulzera, zur Förderung der diabetischen Wundheilung und zur Förderung der Wundheilung von diabetischen Fußulzera.

Weiterer Gegenstand der vorliegenden Erfindung sind Adrenorezeptor α2C Rezeptor Antagonisten zur Verwendung in einem Verfahren zur Behandlung und/oder Prophylaxe von diabetischen Mikroangiopathien, diabetischer Retinopathie, diabetischer Neuropathie, diabetischer Nephropathie, diabetischer erektiler Dysfunktion, diabetischer Herzinsuffizienz, diabetischen koronaren mikrovaskulären Herzerkrankungen, diabetischen Geschwüren an den Extremitäten, diabetischen Fußulzera, zur Förderung der diabetischen Wundheilung und zur Förderung der Wundheilung von diabetischen Fußulzera.

Weiterer Gegenstand der vorliegenden Erfindung sind kompetitive Adrenorezeptor α2C Rezeptor Antagonisten zur Verwendung in einem Verfahren zur Behandlung und/oder Prophylaxe von Begleit- und/oder Folgeerkrankungen von Diabetes mellitus, diabetischen Herzerkrankungen, diabetischen koronaren Herzerkrankungen, diabetischen koronaren mikrovaskulären Herzerkrankungen, diabetischer Herzinsuffizienz, diabetischer Kardiomyopathie und Herzinfarkt, diabetischer Mikroangiopathie, diabetischer Retinopathie, diabetischer Neuropathie, diabetischer Nephropathie, diabetischer erektiler Dysfunktion, diabetischen Geschwüren an den Extremitäten, diabetischen Fußulzera, zur Förderung der diabetischen Wundheilung und zur Förderung der Wundheilung von diabetischen Fußulzera.

Weiterer Gegenstand der vorliegenden Erfindung sind Arzneimittel enthaltend mindestens einen Adrenorezeptor α2C Rezeptor Antagonisten, in Kombination mit einem oder mehreren inerten, nichttoxischen, pharmazeutisch geeigneten Hilfsstoffen, zur Behandlung und/oder Prophylaxe von Begleit- und/oder Folgeerkrankungen von Diabetes mellitus, diabetischen Herzerkrankungen, diabetischen koronaren Herzerkrankungen, diabetischen koronaren mikrovaskulären Herzerkrankungen, diabetischer Herzinsuffizienz, diabetischer Kardiomyopathie und Herzinfarkt, diabetischer Mikroangiopathie, diabetischer Retinopathie, diabetischer Neuropathie, diabetischer Nephropathie, diabetischer erektiler Dysfunktion, diabetischen Geschwüren an den Extremitäten, diabetischen Fußulzera, zur Förderung der diabetischen Wundheilung und zur Förderung der Wundheilung von diabetischen Fußulzera.

Weiterer Gegenstand der vorliegenden Erfindung sind Arzneimittel enthaltend mindestens einen Adrenorezeptor α2C Rezeptor Antagonisten, in Kombination mit einem oder mehreren inerten, nichttoxischen, pharmazeutisch geeigneten Hilfsstoffen, zur Behandlung und/oder Prophylaxe von diabetischen Mikroangiopathien, diabetischer Retinopathie, diabetischer Neuropathie, diabetischer Nephropathie, diabetischer erektiler Dysfunktion, diabetischer Herzinsuffizienz, diabetischen koronaren mikrovaskulären Herzerkrankungen, diabetischen Geschwüren an den Extremitäten, diabetischen Fußulzera, zur Förderung der diabetischen Wundheilung und zur Förderung der Wundheilung von diabetischen Fußulzera.

Weiterer Gegenstand der vorliegenden Erfindung sind Arzneimittel enthaltend mindestens einen kompetitiven Adrenorezeptor α2C Rezeptor Antagonisten, in Kombination mit einem oder mehreren inerten, nicht-toxischen, pharmazeutisch geeigneten Hilfsstoffen, zur Behandlung und/oder Prophylaxe von Begleit- und/oder Folgeerkrankungen von Diabetes mellitus, diabetischen Herzerkrankungen, diabetischen koronaren Herzerkrankungen, diabetischen koronaren mikrovaskulären Herzerkrankungen, diabetischer Herzinsuffizienz, diabetischer Kardiomyopathie und Herzinfarkt, diabetischer Mikroangiopathie, diabetischer Retinopathie, diabetischer Neuropathie, diabetischer Nephropathie, diabetischer erektiler Dysfunktion, diabetischen Geschwüren an den Extremitäten, diabetischen Fußulzera, zur Förderung der diabetischen Wundheilung und zur Förderung der Wundheilung von diabetischen Fußulzera.

Weiterer Gegenstand der vorliegenden Erfindung sind Arzneimittel enthaltend mindestens einen Adrenorezeptor α2C Rezeptor Antagonisten in Kombination mit einem oder mehreren weiteren Wirkstoffen ausgewählt aus der Gruppe bestehend aus den Fettstoffwechsel verändernden Wirkstoffen, Antidiabetika, Blutdruck-Senkern, Sympathikustonus senkenden Mitteln, durchblutungsfördernd und/oder antithrombotisch wirkenden Mitteln sowie Antioxidantien, Aldosteron- und Mineralokortikoid-Rezeptor-Antagonisten, Vasopressin-Rezeptor-Antagonisten, organischen Nitraten und NO-Donatoren, IP- Rezeptor Agonisten, positiv inotrop wirksamen Verbindungen, Calcium-Sensitizern, ACE Inhibitoren, cGMP und cAMP modulierenden Verbindungen, natriuretischen Peptiden, NO-unabhängigen Stimulatoren der Guanylatcyclase, NO-unabhängigen Aktivatoren der Guanylatcyclase, Inhibitoren der humanen neutrophilen Elastase, die Signaltransduktionskaskade inhibierenden Verbindungen, den Energiestoffwechsel des Herzens modulierenden Verbindungen, Chemokin-Rezeptor-Antagonisten, p38-Kinase-Inhibitoren, NPY-Agonisten, Orexin-Agonisten, Anorektika, PAF-AH-Inhibitoren, Antiphlogistika, Analgetika, Antidepressiva und anderen Psychopharmaka.

Weiterer Gegenstand der vorliegenden Erfindung sind Arzneimittel enthaltend mindestens einen kompetitiven Adrenorezeptor α2C Rezeptor Antagonisten in Kombination mit einem oder mehreren weiteren Wirkstoffen ausgewählt aus der Gruppe bestehend aus den Fettstoffwechsel verändernden Wirkstoffen, Antidiabetika, Blutdruck-Senkern, Sympathikustonus senkenden Mitteln, durchblutungsfördernd und/oder antithrombotisch wirkenden Mitteln sowie Antioxidantien, Aldosteron- und Mineralokortikoid-Rezeptor-Antagonisten, Vasopressin-Rezeptor-Antagonisten, organischen Nitraten und NO-Donatoren, IP- Rezeptor Agonisten, positiv inotrop wirksamen Verbindungen, Calcium-Sensitizern, ACE Inhibitoren, cGMP und cAMP modulierenden Verbindungen, natriuretischen Peptiden, NO-unabhängigen Stimulatoren der Guanylatcyclase, NO-unabhängigen Aktivatoren der Guanylatcyclase, Inhibitoren der humanen neutrophilen Elastase, die Signaltransduktionskaskade inhibierenden Verbindungen, den Energiestoffwechsel des Herzens modulierenden Verbindungen, Chemokin-Rezeptor-Antagonisten, p38-Kinase-Inhibitoren, NPY-Agonisten, Orexin-Agonisten, Anorektika, PAF-AH-Inhibitoren, Antiphlogistika, Analgetika, Antidepressiva und anderen Psychopharmaka.

Weiterer Gegenstand der vorliegenden Erfindung ist ein Verfahren zur Behandlung und/oder Prophylaxe von Begleit- und/oder Folgeerkrankungen von Diabetes mellitus, diabetischen Herzerkrankungen, diabetischen koronaren Herzerkrankungen, diabetischen koronaren mikrovaskulären Herzerkrankungen, diabetischer Herzinsuffizienz, diabetischer Kardiomyopathie und Herzinfarkt, diabetischer Mikroangiopathie, diabetischer Retinopathie, diabetischer Neuropathie, diabetischer Nephropathie, diabetischer erektiler Dysfunktion, diabetischen Geschwüren an den Extremitäten, diabetischen Fußulzera, zur Förderung der diabetischen Wundheilung und zur Förderung der Wundheilung von diabetischen Fußulzera, in Menschen und Tieren durch Verabreichung einer wirksamen Menge mindestens eines Adrenorezeptor α2C Rezeptor Antagonisten oder eines Arzneimittels enthaltend mindestens einen Adrenorezeptor α2C Rezeptor Antagonisten.

Weiterer Gegenstand der vorliegenden Erfindung ist ein Verfahren zur Behandlung und/oder Prophylaxe von diabetischen Mikroangiopathien, diabetischer Retinopathie, diabetischer Neuropathie, diabetischer Nephropathie, diabetischer erektiler Dysfunktion, diabetischer Herzinsuffizienz, diabetischen koronaren mikrovaskulären Herzerkrankungen, diabetischen Geschwüren an den Extremitäten, diabetischen Fußulzera, zur Förderung der diabetischen Wundheilung und zur Förderung der Wundheilung von diabetischen Fußulzera, in Menschen und Tieren durch Verabreichung einer wirksamen Menge mindestens eines Adrenorezeptor α2C Rezeptor Antagonisten oder eines Arzneimittels enthaltend mindestens einen Adrenorezeptor α2C Rezeptor Antagonisten.

Weiterer Gegenstand der vorliegenden Erfindung ist ein Verfahren zur Behandlung und/oder Prophylaxe von Begleit- und/oder Folgeerkrankungen von Diabetes mellitus, diabetischen Herzerkrankungen, diabetischen koronaren Herzerkrankungen, diabetischen koronaren mikrovaskulären Herzerkrankungen, diabetischer Herzinsuffizienz, diabetischer Kardiomyopathie und Herzinfarkt, diabetischer Mikroangiopathie, diabetischer Retinopathie, diabetischer Neuropathie, diabetischer Nephropathie, diabetischer erektiler Dysfunktion, diabetischen Geschwüren an den Extremitäten, diabetischen Fußulzera, zur Förderung der diabetischen Wundheilung und zur Förderung der Wundheilung von diabetischen Fußulzera, in Menschen und Tieren durch Verabreichung einer wirksamen Menge mindestens eines kompetitiven Adrenorezeptor α2C Rezeptor Antagonisten oder eines Arzneimittels enthaltend mindestens einen kompetitiven Adrenorezeptor α2C Rezeptor Antagonisten.

Adrenorezeptor α2C Rezeptor Antagonisten im Sinne der vorliegenden Erfindung sind Rezeptorliganden oder Verbindungen, die Adrenorezeptor α2C Rezeptor-Agonist-vermittelte biologische Antworten blockieren oder hemmen. Adrenorezeptor α2C Rezeptor Antagonisten im Sinne der vorliegenden Erfindung können beispielsweise kompetitive Adrenorezeptor α2C Rezeptor Antagonisten, nicht kompetitive Adrenorezeptor α2C Rezeptor Antagonisten, inverse Adrenorezeptor α2C Rezeptor Agonisten oder allosterische Adrenorezeptor α2C Rezeptor Modulatoren sein.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt: den Fettstoffwechsel verändernde Wirkstoffe, Antidiabetika, Blutdruck-Senker, Sympathikustonus senkende Mittel, durchblutungsfördernd und/oder antithrombotisch wirkende Mittel sowie Antioxidantien, Aldosteron- und Mineralokortikoid-Rezeptor-Antagonisten, Vasopressin-Rezeptor-Antagonisten, organische Nitrate und NO-Donatoren, IP- Rezeptor Agonisten, positiv inotrop wirksame Verbindungen, Calcium-Sensitizer, ACE Inhibitoren, cGMP und cAMP modulierende Verbindungen, natriuretische Peptide, NO-unabhängige Stimulatoren der Guanylatcyclase, NO-unabhängige Aktivatoren der Guanylatcyclase, Inhibitoren der humanen neutrophilen Elastase, die Signaltransduktionskaskade inhibierende Verbindungen, den Energiestoffwechsel des Herzens modulierende Verbindungen, Chemokin-Rezeptor-Antagonisten, p38-Kinase-Inhibitoren, NPY-Agonisten, Orexin-Agonisten, Anorektika, PAF-AH-Inhibitoren, Antiphlogistika (COX-Inhibitoren, LTB₄-Rezeptor-Antagonisten, LTB₄-Synthese Hemmer), Analgetika (Aspirin), Antidepressiva und andere Psychopharmaka.

Gegenstand der vorliegenden Erfindung sind insbesondere Kombinationen mit mindestens einer der erfindungsgemäßen Verbindungen mit mindestens einem den Fettstoffwechsel verändernden Wirkstoff, einem Antidiabetikum, einem blutdrucksenkenden Wirkstoff und/oder einem antithrombotisch wirkenden Mittel.

Die erfindungsgemäßen Verbindungen können vorzugsweise mit einem oder mehreren der im folgenden genannten Wirkstoffe kombiniert werden:
- den Fettstoffwechsel verändernden Wirkstoffen, beispielhaft und vorzugsweise aus der Gruppe der HMG-CoA-Reduktase-Inhibitoren aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, Inhibitoren der HMG-CoA-Reduktase-Expression, Squalensynthese-Inhibitoren wie beispielhaft und vorzugsweise BMS-188494 oder TAK-475, ACAT-Inhibitoren wie beispielhaft und vorzugsweise Melinamide, Pactimibe, Eflucimibe oder SMP-797, LDL-Rezeptor-Induktoren, Cholesterin-Absorptionshemmer wie beispielhaft und vorzugsweise Ezetimibe, Tiqueside oder Pamaqueside, polymeren Gallensäureadsorber, wie beispielhaft und vorzugsweise Cholestyramin, Colestipol, Colesolvam, CholestaGel oder Colestimide, Gallensäure-Reabsorptionshemmer, wie beispielhaft und vorzugsweise ASBT (= IBAT)-Inhibitoren wie z.B. Elobixibat (AZD-7806), S-8921, AK-105, Canosimibe (BARI-1741, AVE-5530), SC-435 oder SC-635, MTP-Inhibitoren wie beispielhaft und vorzugsweise Implitapide oder JTT-130, Lipase-Inhibitoren wie beispielhaft und vorzugsweise Orlistat, LpL-Aktivatoren, Fibrate, Niacin, CETP-Inhibitoren, wie beispielhaft und vorzugsweise Torcetrapib, Dalcetrapib (JTT-705) oder CETP Vakzine (Avant), PPAR-γ- und/oder PPAR-δ-Agonisten, wie beispielhaft und vorzugsweise Pioglitazone oder Rosiglitazone und/oder Endurobol (GW-501516), RXR-Modulatoren, FXR-Modulatoren, LXR-Modulatoren, Thyroidhormone und/oder Thyroidmimetika wie beispielhaft und vorzugsweise D-Thyroxin oder 3,5,3'-Triiodothyronin (T3), ATP-Citrat-Lyase-Inhibitoren, Lp(a)-Antagonisten, Cannabinoid-Rezeptor 1-Antagonisten, wie beispielhaft und vorzugsweise Rimonabant oder Surinabant (SR-147778), Leptin-Rezeptor-Agonisten, Bombesin-Rezeptor-Agonisten, Histamin-Rezeptor-Agonisten, Agonisten des Niacin-Rezeptors, wie beispielhaft und vorzugsweise Niacin, Acipimox, Acifran oder Radecol, sowie der Antioxidantien / Radikalfänger wie beispielhaft und vorzugsweise Probucol, Succinobucol (AGI-1067), BO-653 oder AEOL-10150;

- Antidiabetika, die in der Roten Liste 2014, Kapitel 12 genannt sind. Unter Antidiabetika werden vorzugsweise Insulin und Insulinderivate sowie oral wirksame hypoglykämische Wirkstoffe verstanden. Insulin und Insulinderivate umfasst hierbei sowohl Insuline tierischen, menschlichen oder biotechnologischen Ursprungs als auch Gemische hieraus. Die oral wirksamen hypoglykämischen Wirkstoffe umfassen vorzugsweise Sulphonylharnstoffe, Biguanide, Meglitinid-Derivate, Glukosidase-Inhibitoren und PPAR-gamma-Agonisten. Als Sulphonylharnstoffe seien beispielhaft und vorzugsweise genannt Tolbutamid, Glibenclamid, Glimepirid, Glipizid oder Gliclazid, als Biguanid sei beispielhaft und vorzugsweise genannt Metformin, als Meglitinid-Derivate seien beispielhaft und vorzugsweise genannt Repaglinid oder Nateglinid, als Glukosidase-Inhibitoren seien beispielhaft und vorzugsweise genannt Miglitol oder Acarbose, Oxadiazolidinone, Thiazolidindione, GLP 1-Rezeptor-Agonisten, Glukagon-Antagonisten, Insulin-Sensitizer, CCK 1-Rezeptor-Agonisten, Leptin-Rezeptor-Agonisten, Inhibitoren von Leberenzymen, die an der Stimulation der Glukoneogenese und/oder Glykogenolyse beteiligt sind, Modulatoren der Glukoseaufnahme sowie der Kaliumkanalöffner, wie z.B. denjenigen, die in WO 97/26265 und WO 99/03861 offenbart sind;
- den Blutdruck senkenden Wirkstoffen, beispielhaft und vorzugsweise aus der Gruppe der Calcium-Antagonisten wie beispielhaft und vorzugsweise Nifedipin, Amlodipin, Verapamil oder Diltiazem, Angiotensin AII-Antagonisten, wie beispielhaft und vorzugsweise Losartan, Valsartan, Candesartan, Embusartan oder Telmisartan, ACE-Inhibitoren, wie beispielhaft und vorzugsweise Enalapril, Captopril, Ramipril, Delapril, Fosinopril, Quinopril, Perindopril oder Trandopril, beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, alpha-Rezeptoren-Blocker, wie beispielhaft und vorzugsweise Prazosin, ECE-Inhibitoren, Rhokinase Inhibitoren und der Vasopeptidase-Inhibitoren, sowie der Diuretika, wie beispielhaft und vorzugsweise einem Schleifendiuretikum wie Furosemid, Bumetanid oder Torsemid, oder einem Thiazid- oder Thiazidähnlichen Diuretikum wie Chlorthiazid oder Hydrochlorthiazid oder A1 Antagonisten wie Rolofylline, Tonopofylline und SLV-320;
- den Sympathikustonus senkende Mittel wie beispielhaft und vorzugsweise Reserpin, Clonidin oder alpha-Methyl-Dopa, oder in Kombination mit einem Kaliumkanal-Agonisten, wie beispielhaft und vorzugsweise Minoxidil, Diazoxid, Dihydralazin oder Hydralazin;
- antithrombotisch wirkenden Mitteln, beispielhaft und vorzugsweise aus der Gruppe der Thrombozytenaggregationshemmer, wie beispielhaft und vorzugsweise Aspirin, Clopidogrel, Ticlopidin, Cilostazol oder Dipyridamol, oder der Antikoagulantien wie Thrombin-Inhibitoren, wie beispielhaft und vorzugsweise Ximelagatran, Melagatran, Bivalirudin oder Clexane, einem GPIIb/IIIa-Antagonisten, wie beispielhaft und vorzugsweise Tirofiban oder Abciximab, einem Faktor Xa-Inhibitor, wie beispielhaft und vorzugsweise Rivaroxaban, Edoxaban (DU-176b), Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, mit Heparin oder einem low molecular weight (LMW)-Heparin-Derivat oder mit einem Vitamin K-Antagonisten, wie beispielhaft und vorzugsweise Coumarin;
- Aldosteron- und Mineralokorticoid-Rezeptor-Antagonisten, wie beispielhaft und vorzugsweise Spironolacton, Eplerenon oder Finerenon;
- Vasopressin-Rezeptor-Antagonisten, wie beispielhaft und vorzugsweise Conivaptan, Tolvaptan, Lixivaptan oder Satavaptan (SR-121463);
- organischen Nitraten und NO-Donatoren wie beispielhaft und vorzugweise Natriumnitroprussid, Nitroglycerin, Isosorbidmononitrat, Isosorbiddinitrat, Molsidomin oder SIN-1, oder in Kombination mit inhalativem NO;
- IP-Rezeptor Agonisten wie wie beispielhaft und vorzugweise Iloprost, Treprostinil, Beraprost und Selexipag (NS-304);
- positiv-inotrop wirksame Verbindungen, wie beispielhaft und vorzugsweise Herzglycosiden (Digoxin), beta-adrenergen und dopaminergen Agonisten wie Isoproterenol, Adrenalin, Noradrenalin, Dopamin oder Dobutamin;
- Calcium-Sensitizern, wie beispielhaft und vorzugsweise Levosimendan;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil sowie PDE 3-Inhibitoren wie Milrinone;
- natriuretischen Peptiden, wie z.B. "atrial natriuretic peptide" (ANP, Anaritide), "B-type natriuretic peptide" oder "brain natriuretic peptide" (BNP, Nesiritide), "C-type natriuretic peptide" (CNP) sowie Urodilatin;
- NO-unabhängigen, jedoch Häm-abhängigen Stimulatoren der Guanylatcyclase, wie insbesondere den in WO 00/06568, WO 00/06569, WO 02/42301 und WO 03/095451 beschriebenen Verbindungen;
- NO- und Häm-unabhängigen Aktivatoren der Guanylatcyclase, wie insbesondere den in WO 01/19355, WO 01/19776, WO 01/19778, WO 01/19780, WO 02/070462 und WO 02/070510 beschriebenen Verbindungen;
- Inhibitoren der humanen neutrophilen Elastase (HNE), wie beispielsweise Sivelestat und DX-890 (Reltran);
- die Signaltransduktionskaskade inhibierenden Verbindungen, wie beispielsweise Tyrosinkinase-Inhibitoren und Multikinase Inhibitoren, insbesondere Sorafenib, Imatinib, Gefitinib und Erlotinib; und/oder
- den Energiestoffwechsel des Herzens beeinflussenden Verbindungen, wie beispielweise Etomoxir, Dichloracetat, Ranolazine und Trimetazidine.

Besonders bevorzugt im Rahmen der vorliegenden Erfindung sind Kombinationen enthaltend mindestens eine der erfindungsgemäßen Verbindungen sowie einen oder mehrere weitere Wirkstoffe ausgewählt aus der Gruppe bestehend aus HMG-CoA-Reduktase-Inhibitoren (Statine), Diuretika, beta-Rezeptoren-Blocker, organische Nitrate und NO-Donatoren, ACE-Inhibitoren, Angiotensin AII-Antagonisten, Aldosteron- und Mineralokortikoid-Rezeptor-Antagonisten, Vasopressin-Rezeptor-Antagonisten, Thrombozytenaggregationshemmer und Antikoagulantien, sowie deren Verwendung zur Behandlung und/oder Prävention der zuvor genannten Erkrankungen.

Besonders bevorzugt im Rahmen der vorliegenden Erfindung sind Kombinationen enthaltend mindestens eine der erfindungsgemäßen Verbindungen sowie einen oder mehrere weitere Wirkstoffe ausgewählt aus der Gruppe bestehend aus Heparin, Antidiabetika, ACE-Hemmer, Diuretika und Antibiotika, sowie deren Verwendung in einem Verfahren zur Förderung diabetischer Wundheilung und zur Behandlung und/oder Prävention von diabetischen Geschwüren an den Extremitäten, insbesondere zur Förderung der Wundheilung von diabetischen Fußulzera.

Besonders bevorzugt im Rahmen der vorliegenden Erfindung ist die Verwendung mindestens einer der erfindungsgemäßen Verbindungen in einem Verfahren zur Förderung diabetischer Wundheilung und zur Behandlung und/oder Prävention von diabetischen Geschwüren an den Extremitäten, insbesondere zur Förderung der Wundheilung von diabetischen Fußulzera, wobei die Verbindung der Formel (I) zusätzlich zu einer oder mehreren der folgenden physikalischen und/oder topischen Therapien eingesetzt wird: Wundmanagement wie Verbände, Wundausschneidung, Gewichtsabnahme bei angepasstem Schuhwerk, PDGF (Regranex), hyperbare Sauerstofftherapie, Wundtherapie mit negativem Druck.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nicht überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Bevorzugt ist die orale Applikation.

Bei der beispielhaften Verwendung der Verbindungen der Formel (I) zur Förderung der diabetischen Wundheilung, insbesondere zur Förderung der Wundheilung von diabetischen Fußulzera, ist neben der oralen Applikation auch die Applikation in Form einer topischen Formulierung bevorzugt.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, vorzugsweise zusammen mit einem oder mehreren inerten nichttoxischen, pharmazeutisch geeigneten Hilfsstoff enthalten, sowie deren Verwendung zu den zuvor genannten Zwecken.

Im Allgemeinen hat es sich als vorteilhaft erwiesen, bei oraler Applikation Mengen von etwa 0,1 bis 250 mg je 24 Stunden, bevorzugt von 0,1 bis 50 mg je 24 Stunden zur Erzielung wirksamer Ergebnisse zu verabreichen. Die Dosis kann in mehrere Applikationen pro Tag aufgeteilt werden. Beispiele sind eine zwei- oder dreimal tägliche Applikation.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt.

Ein weiterer Gegenstand der vorliegenden Erfindung ist eine Verbindung der Formel (I), wie zuvor beschrieben, zur Verwendung in einem Verfahren zur Behandlung und/oder Prophylaxe von primären und sekundären Formen von diabetischen Mikroangiopathien, diabetischer Wundheilung, diabetischen Geschwüren an den Extremitäten, insbesondere zur Förderung der Wundheilung von diabetischen Fußulzera, diabetischer Retinopathie, diabetischer Nephropathie, diabetischer erektiler Dysfunktion, diabetischer Herzinsuffizienz, diabetischen koronaren mikrovaskulären Herzerkrankungen, peripheren und kardialen Gefäßerkrankungen, thromboembolischen Erkrankungen und Ischämien, peripheren Durchblutungsstörungen, Raynaud-Phänomen, CREST-Syndrom, Mikrozirkulationsstörungen, Claudicatio intermittens, und peripheren und autonomen Neuropathien.

Ein weiterer Gegenstand der vorliegenden Erfindung ist eine Verbindung der Formel (I), wie zuvor beschrieben, zur Verwendung in einem Verfahren zur Behandlung und/oder Prophylaxe von primären und sekundären Formen von Herzinsuffizienz, peripheren und kardialen Gefäßerkrankungen, thromboembolischen Erkrankungen und Ischämien, peripheren Durchblutungsstörungen, Raynaud-Phänomen, Mikrozirkulationsstörungen, Claudicatio intermittens, peripheren und autonomen Neuropathien, diabetischen Mikroangiopathien, diabetischer Nephropathie, diabetischer Retinopathie, diabetischen Geschwüren an den Extremitäten und CREST-Syndrom, sowie zur diabetischen Wundheilung, insbesondere zur Förderung der Wundheilung von diabetischen Fußulzera.

Ein weiterer Gegenstand der vorliegenden Erfindung ist eine Verbindung der Formel (I), wie zuvor beschrieben, zur Herstellung eines Arzneimittels zur Behandlung und/oder Prophylaxe von primären und sekundären Formen von diabetischen Mikroangiopathien, diabetischer Wundheilung, diabetischen Geschwüren an den Extremitäten, insbesondere zur Förderung der Wundheilung von diabetischen Fußulzera, diabetischer Retinopathie, diabetischer Nephropathie, diabetischer erektiler Dysfunktion, diabetischer Herzinsuffizienz, diabetischen koronaren mikrovaskulären Herzerkrankungen, peripheren und kardialen Gefäßerkrankungen, thromboembolischen Erkrankungen und Ischämien, peripheren Durchblutungsstörungen, Raynaud-Phänomen, CREST-Syndrom, Mikrozirkulationsstörungen, Claudicatio intermittens, und peripheren und autonomen Neuropathien.

Ein weiterer Gegenstand der vorliegenden Erfindung ist die Verwendung einer Verbindung der Formel (I), wie zuvor beschrieben, zur Herstellung eines Arzneimittels zur Behandlung und/oder Prophylaxe von primären und sekundären Formen von Herzinsuffizienz, peripheren und kardialen Gefäßerkrankungen, thromboembolischen Erkrankungen und Ischämien, peripheren Durchblutungsstörungen, Raynaud-Phänomen, Mikrozirkulationsstörungen, Claudicatio intermittens, peripheren und autonomen Neuropathien, diabetischen Mikroangiopathien, diabetischer Nephropathie, diabetischer Retinopathie, diabetischen Geschwüren an den Extremitäten und CREST-Syndrom, sowie zur diabetischen Wundheilung, insbesondere zur Förderung der Wundheilung von diabetischen Fußulzera.

Ein weiterer Gegenstand der vorliegenden Erfindung ist ein Arzneimittel enthaltend eine Verbindung der Formel (I), wie zuvor beschrieben, in Kombination mit einem oder mehreren inerten, nichttoxischen, pharmazeutisch geeigneten Hilfsstoffen.

Ein weiterer Gegenstand der vorliegenden Erfindung ist ein Arzneimittel enthaltend eine Verbindung der Formel (I), wie zuvor beschrieben, in Kombination mit einem oder mehreren weiteren Wirkstoffen ausgewählt aus der Gruppe bestehend aus den Fettstoffwechsel verändernden Wirkstoffen, Antidiabetika, Blutdruck-Senkern, Sympathikustonus senkenden Mitteln, durchblutungsfördernd und/oder antithrombotisch wirkenden Mitteln sowie Antioxidantien, Aldosteron- und Mineralokortikoid-Rezeptor-Antagonisten, Vasopressin-Rezeptor-Antagonisten, organischen Nitraten und NO-Donatoren, IP- Rezeptor Agonisten, positiv inotrop wirksamen Verbindungen, Calcium-Sensitizern, ACE Inhibitoren, cGMP und cAMP modulierenden Verbindungen, natriuretischen Peptiden, NO-unabhängigen Stimulatoren der Guanylatcyclase, NO-unabhängigen Aktivatoren der Guanylatcyclase, Inhibitoren der humanen neutrophilen Elastase, die Signaltransduktionskaskade inhibierenden Verbindungen, den Energiestoffwechsel des Herzens modulierenden Verbindungen, Chemokin-Rezeptor-Antagonisten, p38-Kinase-Inhibitoren, NPY-Agonisten, Orexin-Agonisten, Anorektika, PAF-AH-Inhibitoren, Antiphlogistika, Analgetika, Antidepressiva und anderen Psychopharmaka.

Ein weiterer Gegenstand der vorliegenden Erfindung ist ein Arzneimittel entsprechend der vorherigen Ausführungen zur Behandlung und/oder Prophylaxe von primären und sekundären Formen von diabetischen Mikroangiopathien, diabetischer Wundheilung, diabetischen Geschwüren an den Extremitäten, insbesondere zur Förderung der Wundheilung von diabetischen Fußulzera, diabetischer Retinopathie, diabetischer Nephropathie, diabetischer erektiler Dysfunktion, diabetischer Herzinsuffizienz, diabetischen koronaren mikrovaskulären Herzerkrankungen, peripheren und kardialen Gefäßerkrankungen, thromboembolischen Erkrankungen und Ischämien, peripheren Durchblutungsstörungen, Raynaud-Phänomen, CREST-Syndrom, Mikrozirkulationsstörungen, Claudicatio intermittens, und peripheren und autonomen Neuropathien.

Ein weiterer Gegenstand der vorliegenden Erfindung ist ein Arzneimittel entsprechend der vorherigen Ausführungen zur Behandlung und/oder Prophylaxe von primären und sekundären Formen von Herzinsuffizienz, peripheren und kardialen Gefäßerkrankungen, thromboembolischen Erkrankungen und Ischämien, peripheren Durchblutungsstörungen, Raynaud-Phänomen, Mikrozirkulationsstörungen, Claudicatio intermittens, peripheren und autonomen Neuropathien, diabetischen Mikroangiopathien, diabetischer Nephropathie, diabetischer Retinopathie, diabetischen Geschwüren an den Extremitäten und CREST-Syndrom, sowie zur diabetischen Wundheilung, insbesondere zur Förderung der Wundheilung von diabetischen Fußulzera.

Ein weiterer Gegenstand der vorliegenden Erfindung ist ein Verfahren zur Behandlung und/oder Prophylaxe von primären und sekundären Formen von diabetischen Mikroangiopathien, diabetischer Wundheilung, diabetischen Geschwüren an den Extremitäten, insbesondere zur Förderung der Wundheilung von diabetischen Fußulzera, diabetischer Retinopathie, diabetischer Nephropathie, diabetischer erektiler Dysfunktion, diabetischer Herzinsuffizienz, diabetischen koronaren mikrovaskulären Herzerkrankungen, peripheren und kardialen Gefäßerkrankungen, thromboembolischen Erkrankungen und Ischämien, peripheren Durchblutungsstörungen, Raynaud-Phänomen, CREST-Syndrom, Mikrozirkulationsstörungen, Claudicatio intermittens, und peripheren und autonomen Neuropathien in Menschen und Tieren durch Verabreichung einer wirksamen Menge mindestens einer Verbindung der Formel (I), wie zuvor beschrieben, oder eines Arzneimittels, wie zuvor beschrieben.

Ein weiterer Gegenstand der vorliegenden Erfindung ist ein Verfahren zur Behandlung und/oder Prävention von Prophylaxe von primären und sekundären Formen von Herzinsuffizienz, peripheren und kardialen Gefäßerkrankungen, thromboembolischen Erkrankungen und Ischämien, peripheren Durchblutungsstörungen, Raynaud-Phänomen, Mikrozirkulationsstörungen, Claudicatio intermittens, peripheren und autonomen Neuropathien, diabetischen Mikroangiopathien, diabetischer Nephropathie, diabetischer Retinopathie, diabetischen Geschwüren an den Extremitäten und CREST-Syndrom, sowie zur diabetischen Wundheilung, insbesondere zur Förderung der Wundheilung von diabetischen Fußulzera, in Menschen und Tieren durch Verabreichung einer wirksamen Menge mindestens einer Verbindung der Formel (I), wie zuvor beschrieben, oder eines Arzneimittels, wie zuvor beschrieben.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Die Angabe "w/v" bedeutet "weight/volume" (Gewicht/Volumen). So bedeutet beispielsweise "10% w/v": 100 ml Lösung oder Suspension enthalten 10 g Substanz.

Wenn bei den im Folgenden beschriebenen Synthese-Intermediaten und Ausführungsbeispielen der Erfindung eine Verbindung in der Form eines Salzes der korrespondierenden Base bzw. Säure aufgeführt ist, so ist die exakte stöchiometrische Zusammensetzung eines solchen Salzes, wie es nach dem jeweiligen Herstell- und/oder Reinigungsverfahren erhalten wurde, in der Regel nicht bekannt. Sofern nicht genauer spezifiziert, sind daher Namens- und Strukturformel-Zusätze wie beispielsweise "Hydrochlorid", "Trifluoracetat", "Oxalat-Salz", "Natrium-Salz" bzw. "x HCl", "x CF₃COOH", "xC₂O₄²⁻", "x Na⁺" bei solchen Salzen nicht stöchiometrisch zu verstehen, sondern haben allein deskriptiven Charakter bezüglich der enthaltenen salzbildenden Komponenten.

Sinngemäß gleiches gilt für den Fall, dass Synthese-Intermediate oder Ausführungsbeispiele oder Salze hiervon nach den beschriebenen Herstell- und/oder Reinigungsverfahren in Form von Solvaten, wie beispielsweise Hydraten, erhalten wurden, deren stöchiometrische Zusammensetzung (sofern definierter Art) nicht bekannt ist.

### A) Beispiele

### Abkürzungen:

- ca.: circa
- CDI: Carbonyldiimidazol
- d: Tag(e), Dublett (bei NMR)
- DC: Dünnschicht-Chromatographie
- DCI: direkte chemische Ionisation (bei MS)
- dd: Doppeltes Dublett (bei NMR)
- DMAP: 4-Dimethylaminopyridin
- DMF: *N,N-*Dimethylformamid
- DMSO: Dimethylsulfoxid
- DSC: Disuccinimidylcarbonat
- d. Th.: der Theorie (bei Ausbeute)
- eq.: Äquivalent(e)
- ESI: Elektrospray-Ionisation (bei MS)
- h: Stunde(n)
- HATU: *O*-(7-Azabenzotriazol-1-yl)-*N*,*N*,*N',N*'-tetramethyluronium-Hexafluorphosphat
- HPLC: Hochdruck-, Hochleistungsflüssigchromatographie
- HV: Hochvakuum
- LDA: Lithiumdiisopropylamid
- m: Multiplett (bei NMR)
- min: Minute(n)
- MS: Massenspektroskopie
- NMR: Kernresonanzspektroskopie
- PYBOP: Benzotriazol-1-yloxy-tris(pyrrolidino)phosphonium-Hexafluorophosphat
- q: Quartett (bei NMR)
- RP: reverse phase (bei HPLC)
- RT: Raumtemperatur
- Rₜ: Retentionszeit (bei HPLC)
- s: Singulett (bei NMR)
- t: Triplett (bei NMR)
- T3P: Propylphosphonsäureanhydrid 50%ig in Essigsäureethylester oder DMF
- THF: Tetrahydrofuran

### LC-MS- und HPLC-Methoden:

Methode 1 (LC-MS): Instrument: Waters ACQUITY SQD UPLC System; Säule: Waters Acquity UPLC HSS T3 1.8 µ 50 mm x 1 mm; Eluent A: 1 l Wasser + 0.25 ml 99%ige Ameisensäure, Eluent B: 1 l Acetonitril + 0.25 ml 99%ige Ameisensäure; Gradient: 0.0 min 90% A → 1.2 min 5% A → 2.0 min 5% A; Ofen: 50°C; Fluss: 0.40 ml/min; UV-Detektion: 210 - 400 nm.
Methode 2 (LC-MS): Instrument: Waters ACQUITY SQD UPLC System; Säule: Waters Acquity UPLC HSS T3 1.8 µ 50 mm x 1 mm; Eluent A: 1 l Wasser + 0.25 ml 99%ige Ameisensäure, Eluent B: 1 l Acetonitril + 0.25 ml 99%ige Ameisensäure; Gradient: 0.0 min 90% A → 1.2 min 5% A → 2.0 min 5% A; Ofen: 50 °C; Fluss: 0.40 ml/min; UV-Detektion: 210 - 400 nm.
Methode 3 (LC-MS): Instrument: Waters ACQUITY SQD UPLC System; Säule: Waters Acquity UPLC HSS T3 1.8 µ 30 x 2 mm; Eluent A: 1 l Wasser + 0.25 ml 99%ige Ameisensäure , Eluent B: 1 l Acetonitril + 0.25 ml 99%ige Ameisensäure; Gradient: 0.0 min 90% A → 1.2 min 5% A → 2.0 min 5% A Ofen: 50°C; Fluss: 0.60 ml/min; UV-Detektion: 208 - 400 nm.
Methode 4 (LC-MS): Instrument: Micromass Quattro Premier mit Waters UPLC Acquity; Säule: Thermo Hypersil GOLD 1.9 µ 50 mm x 1 mm; Eluent A: 1 l Wasser + 0.5 ml 50%ige Ameisensäure, Eluent B: 1 l Acetonitril + 0.5 ml 50%ige Ameisensäure; Gradient: 0.0 min 90% A → 0.1 min 90% A → 1.5 min 10% A → 2.2 min 10% A; Ofen: 50°C; Fluss: 0.33 ml/min; UV-Detektion: 210 nm.
Methode 5 (LC-MS): Gerätetyp MS: Waters (Micromass) Quattro Micro; Gerätetyp HPLC: Agilent 1100 Serie; Säule: Thermo Hypersil GOLD 3 µ 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 100% A → 3.0 min 10% A → 4.0 min 10% A → 4.01 min 100% A (Fluss: 2.5 ml) → 5.00 min 100% A; Ofen: 50°C; Fluss: 2 ml/min; UV-Detektion: 210 nm.
Methode 6 (LC-MS): Gerätetyp MS: Waters ZQ; Gerätetyp HPLC: Agilent 1100 Series; UV DAD; Säule: Thermo Hypersil GOLD 3 µ 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 100% A → 3.0 min 10% A → 4.0 min 10% A → 4.1 min 100%; Ofen: 55°C; Fluss: 2 ml/min; UV-Detektion: 210 nm.
Methode 7 (LC-MS): Instrument MS: Waters (Micromass) QM; Instrument HPLC: Agilent 1100 Serie; Säule: Agient ZORBAX Extend-C18 3.0x50mm 3.5-Micron; Eluent A: 1 l Wasser + 0.01 mol Ammoniumcarbonat, Eluent B: 1 l Acetonitril; Gradient: 0.0 min 98% A → 0.2min 98% A → 3.0 min 5% A → 4.5 min 5% A ; Ofen: 40°C; Fluss: 1.75 ml/min; UV-Detektion: 210 nm.
Methode 8 (LC-MS): Instrument MS: Waters (Micromass) Quattro Micro; Instrument HPLC: Agilent 1100 Serie; Säule: YMC-Triart C18 3 µ 50 x 3 mm; Eluent A: 1 l Wasser + 0.01 mol Ammoniumcarbonat, Eluent B: 1 l Acetonitril; Gradient: 0.0 min 100% A → 2.75 min 5% A → 4.5 min 5% A; Ofen: 40°C; Fluss: 1.25 ml/min; UV-Detektion: 210 nm
Methode 9 (präparative HPLC): Säule: Waters XBridge, 50 x 19 mm, 10 µm, Eluent A: Wasser + 0.5% Ammoniumhydroxid, Eluent B: Acetonitril, 5 min = 95% A, 25min = 50% A, 38min = 50% A, 38,1min = 5% A, 43min= 5% A, 43,01min= 95% A, 48,0min= 5% A; Fluss 20 ml/min, UV-Detektion: 210 nm.

Die Zuordnung der NMR Daten erfolgt soweit die Signale nicht durch Lösemittel verdeckt sind.

### Ausgangsverbindungen

### Beispiel 1A

### tert-Butyl-4-(3,4-dihydroisochinolin-2(1H)-yl)piperidin-1-carboxylat

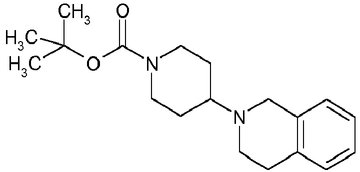

150 g (753 mmol) tert-Butyl-4-oxopiperidin-1-carboxylat sowie 120 g (903 mmol) 1,2,3,4-Tetrahydroisochinolin wurden in 1500 ml THF gelöst und mit 239 g (1129 mmol) Natriumtriacetoxyborhydrid versetzt wobei die Temperatur des Gemisches bei ca. 30°C gehalten wurde. Es wurde ca. 1 h bei RT nachgerührt und anschließend mit ca. 1000 ml gesättigter Natriumhydrogencarbonatlösung versetzt. Es wurde mit ca. 500 ml Essigsäureethylester extrahiert. Die organische Phase wurde mit weiteren 500 ml gesättigter Natriumhydrogencarbonatlösung sowie mit 200 ml gesättigter Natriumchloridlösung gewaschen. Anschließend wurde über Natriumsulfat getrocknet, filtriert und eingeengt. Es wurden 234 g (98% d. Th.) des Zielproduktes erhalten, das ohne weitere Reinigung weiterverarbeitet wurde.
LC-MS [Methode 1]: Rₜ = 0.72 min; MS (ESIpos): m/z = 317 (M + H)⁺
¹H-NMR (400MHz, CDCl₃): δ [ppm]= 1.47 (s, 9 H) 1.48 - 1.60 (m, 2 H) 1.75 - 1.94 (m, 3 H) 2.56 - 2.66 (m, 1 H) 2.67 - 2.81 (m, 2 H) 2.81 - 2.93 (m, 4 H) 3.78 (s, 2 H) 4.08 - 4.27 (m, 1 H) 6.98 - 7.05 (m, 1 H) 7.07 - 7.14 (m, 3 H).

### Beispiel 2A

### 2-(Piperidin-4-yl)-1,2,3,4-tetrahydroisochinolinhydrochlorid

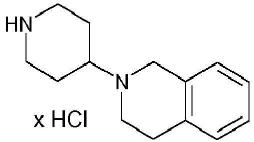

210 g (664 mmol) der Verbindung aus Beispiel 1A wurden in 1600 ml Dichlormethan gelöst und mit 830 ml (3318 mmol) 4M Chlorwasserstoff in Dioxan versetzt wobei die Temperatur des Gemisches bei 25-30°C gehalten wurde. Das Produkt begann nach ca. 1/3 der Zugabe zu kristallisieren. Es wurde ca. 20 h bei RT nachgerührt und anschließend mit ca. 2000 ml tert-Butyl-methylether versetzt. Der ausgefallene Niederschlag wurde abgesaugt, mit tert-Butyl-methylether gewaschen und im Vakuum getrocknet. Es wurden 185 g (97% d. Th.) des Zielproduktes als weißer Feststoff erhalten.
LC-MS [Methode 7]: Rₜ = 2.08 min; MS (ESIpos): m/z = 217 (M + H)⁺
¹H-NMR (400MHz, DMSO-d₆): δ [ppm]= 1.96 - 2.20 (m, 2H), 2.28 - 2.44 (m, 2H), 2.81 - 3.51 (m, 6H), 3.51 - 3.80 (m, 3H), 4.33 - 4.51 (m, 2H), 7.17 - 7.35 (m, 4H), 8.92 - 9.10 (m, 1H), 9.12 - 9.32 (m, 1H), 11.47 (br. s, 1H).

### Beispiel 3A

### tert-Butyl-4-(7-fluor-3,4-dihydroisochinolin-2(1H)-yl)piperidin-1-carboxylat

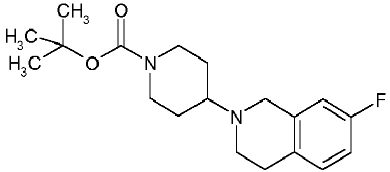

1.40 g (7.03 mmol) tert-Butyl-4-oxopiperidin-1-carboxylat, 1.58 g (8.43 mmol) 7-Fluor-1,2,3,4-tetrahydroisochinolinhydrochlorid sowie 2.45 ml (14.05 mmol) N,N-Diisopropylethylamin wurden in 50 ml Dichlormethan gelöst und mit ca. 1.5 g Molsieb (4Å) versetzt. Die Suspension wurde 1 h bei RT gerührt. Anschließend wurde mit 2.23 g (10.54 mmol) Natriumtriacetoxyborhydrid versetzt ca. 18 h bei RT gerührt. Zur Aufarbeitung wurde mit ca. 50 ml Dichlormethan verdünnt und anschließend zweimal mit ca. 100 ml gesättigter Natriumhydrogencarbonatlösung gewaschen. Die vereinigten wässrigen Phasen wurden einmal mit ca. 50 ml Dichlormethan extrahiert. Es wurde mit ca. 500 ml Essigsäureethylester extrahiert. Die organische Phase wurde mit weiteren 500 ml gesättigter Natriumhydrogencarbonatlösung sowie mit 200 ml gesättigter Natriumchloridlösung gewaschen. Anschließend wurden die vereinigten organischen Phasen über Natriumsulfat getrocknet, filtriert und eingeengt. Der erhaltene Rückstand wurde durch Chromatographie an Kieselgel (Elution mit Cyclohexan/Essigsäureethylester 5:1 - 2:1) gereinigt. Es wurden 1.58 g (67% d. Th.) des Zielproduktes erhalten.
LC-MS [Methode 3]: Rₜ = 0.62 min; MS (ESIpos): m/z = 335 (M + H)⁺

### Beispiel 4A

### 7-Fluor-2-(piperidin-4-yl)-1,2,3,4-tetrahydroisochinolinhydrochlorid

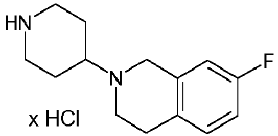

1.58 g (4.72 mmol) der Verbindung aus Beispiel 3A wurden in ca. 30 ml Dichlormethan gelöst und mit 7.1 ml (28.35 mmol) 4M Chlorwasserstoff in Dioxan versetzt. Es wurde ca. 20 h bei RT nachgerührt und anschließend mit ca. 100 ml Diethylether versetzt. Der ausgefallene Niederschlag wurde abgesaugt, mit Diethylether gewaschen und im HV getrocknet. Es wurden 1.17 g (81% d. Th.) des Zielproduktes als weißer Feststoff erhalten.
LC-MS [Methode 3]: Rₜ = 0.18 min; MS (ESIpos): m/z = 235 (M + H)⁺
¹H-NMR (400MHz, DMSO-d₆): δ [ppm]= 1.96 - 2.17 (m, 2 H), 2.27 - 2.43 (m, 2 H), 2.85 - 3.08 (m, 3 H), 3.50 - 3.62 (m, 1 H), 3.20 - 3.47 (m, 3H), 3.63 - 3.78 (m, 1 H), 4.30 - 4.58 (m, 2 H), 7.07 - 7.17 (m, 2 H), 7.21 - 7.41 (m, 1 H), 8.86 - 9.26 (m, 1 H), 11.49 - 11.79 (m, 2 H).

### Beispiel 5A

### tert-Butyl-4-(6-fluor-3,4-dihydroisochinolin-2(1H)-yl)piperidin-1-carboxylat

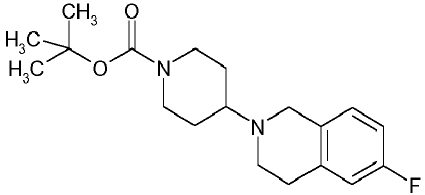

1.73 g (8.66 mmol) tert-Butyl-4-oxopiperidin-1-carboxylat, 1.95 g (10.39 mmol) 6-Fluor-1,2,3,4-tetrahydroisochinolinhydrochlorid sowie 3.02 ml (17.32 mmol) N,N-Diisopropylethylamin wurden in 50 ml Dichlormethan gelöst und mit ca. 10 g Molsieb (4Å) versetzt. Die Suspension wurde 1 h bei RT gerührt. Anschließend wurde mit 2.75 g (12.99 mmol) Natriumtriacetoxyborhydrid versetzt ca. 18 h bei RT gerührt. Zur Aufarbeitung wurde mit ca. 50 ml Dichlormethan verdünnt und anschließend zweimal mit ca. 100 ml gesättigter Natriumhydrogencarbonatlösung gewaschen. Die vereinigten wässrigen Phasen wurden einmal mit ca. 50 ml Dichlormethan extrahiert. Es wurde mit ca. 500 ml Essigsäureethylester extrahiert. Die organische Phase wurde mit weiteren 500 ml gesättigter Natriumhydrogencarbonatlösung sowie mit 200 ml gesättigter Natriumchloridlösung gewaschen. Anschließend wurden die vereinigten organischen Phasen über Natriumsulfat getrocknet, filtriert und eingeengt. Der erhaltene Rückstand wurde durch Chromatographie an Kieselgel (Elution mit Cyclohexan/Essigsäureethylester 2:1 - 1:1) gereinigt. Es wurden 2.73 g (94% d. Th.) des Zielproduktes erhalten.
LC-MS [Methode 1]: Rₜ = 0.70 min; MS (ESIpos): m/z = 335 (M + H)⁺

### Beispiel 6A

### 6-Fluor-2-(piperidin-4-yl)-1,2,3,4-tetrahydroisochinolinhydrochlorid

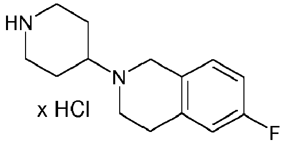

2.73 g (8.16 mmol) der Verbindung aus Beispiel 5A wurden in ca. 60 ml Dichlormethan gelöst und mit 10.2 ml (40.82 mmol) 4M Chlorwasserstoff in Dioxan versetzt. Es wurde ca. 20 h bei RT nachgerührt und anschließend mit ca. 100 ml Diethylether versetzt. Der ausgefallene Niederschlag wurde abgesaugt, mit Diethylether gewaschen und im HV getrocknet. Es wurden 2.24 g (89% d. Th.) des Zielproduktes als weißer Feststoff erhalten.
LC-MS MS [Methode 8]: Rₜ = 2.20 min; MS (ESIpos): m/z = 235 (M + H)⁺
¹H-NMR (400MHz, DMSO-d₆): δ [ppm]= 1.99 - 2.15 (m, 2 H), 2.28 - 2.42 (m, 2 H), 2.85 - 3.11 (m, 3 H), 3.50 - 3.62 (m, 1 H), 3.24 - 3.48 (m, 3H), 3.50 - 3.72 (m, 2 H), 4.30 - 4.50 (m, 2 H), 7.10 - 7.19 (m, 2 H), 7.25 - 7.34 (m, 1 H), 9.04 (s br, 1 H), 9.24 (s br, 1 H), 11.65 (s br, 1 H).

### Beispiel 7A

### tert-Butyl-4-(7-methoxy-3,4-dihydroisochinolin-2(1H)-yl)piperidin-1-carboxylat

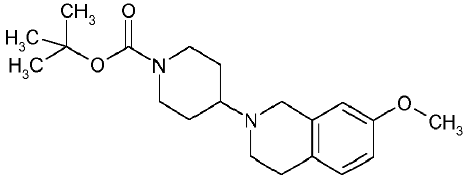

Analog zur Verbindung aus Beispiel 5A wurden 3.27 g (16.42 mmol) tert-Butyl-4-oxopiperidin-1-carboxylat, 3.93 g (10.39 mmol) 7-Methoxy-1,2,3,4-tetrahydroisochinolinhydrochlorid sowie 5.72 ml (32.84 mmol) N,N-Diisopropylethylamin mit 5.22 g (24.63 mmol) Natriumtriacetoxyborhydrid umgesetzt. Es wurden 5.33 g (92% d. Th.) des Zielproduktes erhalten.
LC-MS [Methode 1]: Rₜ = 0.60 min; MS (ESIpos): m/z = 347 (M + H)⁺

### Beispiel 8A

### 7-Methoxy-2-(piperidin-4-yl)-1,2,3,4-tetrahydroisochinolinhydrochlorid

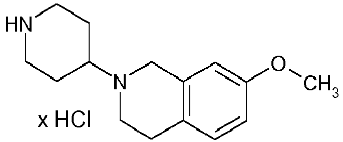

Analog zur Verbindung aus Beispiel 6A wurden 5.33 g (15.17 mmol) der Verbindung aus Beispiel 7A mit 22.75 ml (91.01 mmol) 4M Chlorwasserstoff in Dioxan umgesetzt. Es wurden 4.39 g (91% d. Th.) des Zielproduktes als weißer Feststoff erhalten.
LC-MS MS [Methode 8]: Rₜ = 3.04 min; MS (ESIpos): m/z = 247 (M + H)⁺
¹H-NMR (400MHz, DMSO-d₆): δ [ppm]= 1.97 - 2.05 (m, 2 H) 2.25 - 2.42 (m, 2 H) 2.84 - 3.03 (m, 3 H) 3.11 - 3.22 (m, 1 H) 3.30 - 3.61 (m, 4 H), 3.62 - 3.71 (m, 1 H), 3.74 (s, 3 H,) 4.31 - 4.47 (m, 2 H), 6.83 (s, 1 H), 6.89 (d, 1 H), 7.17 (d,1 H), 8.89 - 9.04 (m, 2 H), 11.21 (br. s, 1 H).

### Beispiel 9A

### tert-Butyl-4-(6-methoxy-3,4-dihydroisochinolin-2(1H)-yl)piperidin-1-carboxylat

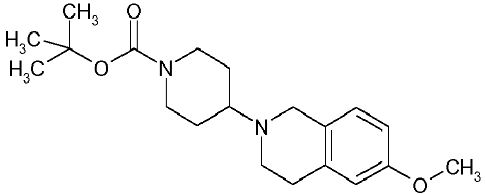

Analog zur Verbindung aus Beispiel 5A wurden 2.59 g (13.02 mmol) tert-Butyl-4-oxopiperidin-1-carboxylat und 2.55 g (15.62 mmol) 6-Methoxy-1,2,3,4-tetrahydroisochinolin mit 4.14 g (19.53 mmol) Natriumtriacetoxyborhydrid umgesetzt. Es wurden 4.28 g (91% d. Th.) des Zielproduktes erhalten. LC-MS [Methode 1]: Rₜ = 0.65 min; MS (ESIpos): m/z = 347 (M + H)⁺

### Beispiel 10A

### 6-Methoxy-2-(piperidin-4-yl)-1,2,3,4-tetrahydroisochinolinhydrochlorid

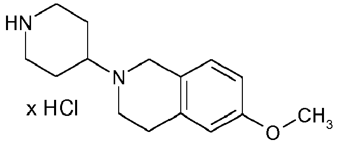

Analog zur Verbindung aus Beispiel 6A wurden 4.28 g (11.86 mmol) der Verbindung aus Beispiel 9A mit 17.79 ml (71.15 mmol) 4M Chlorwasserstoff in Dioxan umgesetzt. Es wurden 3.50 g (92% d. Th.) des Zielproduktes als weißer Feststoff erhalten.
LC-MS MS [Methode 8]: Rₜ = 2.62 min; MS (ESIpos): m/z = 247 (M + H)⁺
¹H-NMR (400MHz, DMSO-d₆): δ [ppm]= 1.97 - 2.13 (m, 2 H), 2.27 - 2.41 (m, 2 H), 2.85 - 3.06 (m, 3 H), 3.11 - 3.49 (m, 4 H), 3.49 - 3.71 (m, 2 H), 3.75 (s, 3 H), 4.26 - 4.43 (m, 2 H), 6.81 - 6.89 (m, 2 H), 7.15 (d, 1 H), 8.88 - 9.20 (m, 2 H), 11.31 (br. s, 1 H).

### Beispiel 11A

### Ethyl-2-[(2-methoxyethyl)amino]pyrimidin-5-carboxylat

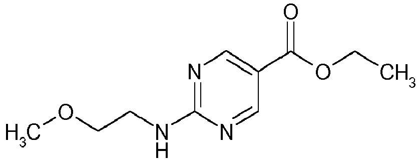

0.42 ml (4.8 mmol) 2-Methoxyethylamin wurden zu einer Suspension von 1.00 g (4.34 mmol) Ethyl-2-(methylsulfonyl)pyrimidin-5-carboxylat und 1.80 g (13.0 mmol) Kaliumcarbonat in 10 ml Acetonitril getropft. Nach 4 h Rühren bei RT wurde die Reaktionsmischung eingeengt, der Rückstand wurde mit Dichlormethan und Wasser aufgenommen. Die Phasen wurden getrennt, die wässrige Phase wurde mit Dichlormethan extrahiert und die vereinigten organischen Phasen wurden über Magnesiumsulfat getrocknet, filtriert und eingeengt. Das Rohprodukt wurde chromatographisch an Kieselgel (Elution mit Cyclohexan/Essigsäureethylester 95:5 - 70:30) gereingt wobei 485 mg (50 % d. Th.) der Titelverbindung isoliert wurden.
LC-MS [Methode 1]: Rₜ = 0.69 min; MS (ESIpos): m/z = 226 (M + H)⁺
¹H-NMR (500MHz, DMSO-d₆): δ [ppm]= 1.29 (t, 3H), 3.25 (s, 3H), 3.43 - 3.57 (m, 4H), 4.26 (q, 2H), 8.06 (br. s., 1H), 8.67 - 8.77 (m, 2H).

### Beispiel 12A

### 2-[(2-Methoxyethyl)amino]pyrimidin-5-carbonsäure

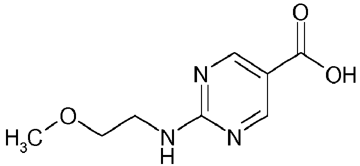

Eine Lösung von 485 mg (2.15 mmol) Ethyl-2-[(2-methoxyethyl)amino]pyrimidin-5-carboxylat in 10 ml Dioxan wurde mit 10.8 ml 1N Natriumhydroxidlösung versetzt und 4 h bei RT gerührt. Zur Aufarbeitung wurde die Reaktionsmischung eingeengt und mit 1N Salzsäure angesäuert. Der ausgefallene Niederschlag wurde abfiltriert, zweimal mit Wasser gewaschen und am HV getrocknet. Es wurden 280 mg (66% d. Th.) der Titelverbindung isoliert.
LC-MS [Methode 8]: Rₜ = 0.44 min; MS (ESIpos): m/z = 198 (M + H)⁺
¹H-NMR (400MHz, DMSO-d₆): δ [ppm]= 3.25 (s, 3H), 3.42 - 3.56 (m, 4H), 7.99 (t, 1H), 8.63 - 8.76 (m, 2H), 12.7 (br. s, 1H).

### Beispiel 13A

### (rac)-Ethyl-2-[(1-methoxybutan-2-yl)amino]pyrimidin-5-carboxylat

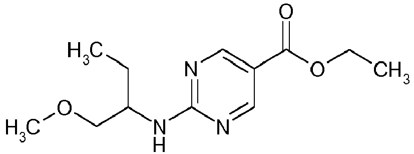

Analog der Verbindung aus Beispiel 11A wurden 493 mg (4.8 mmol) 1-Methoxy-2-aminobutan, 1.00 g (4.34 mmol) Ethyl-2-(methylsulfonyl)pyrimidin-5-carboxylat sowie 1.80 g (13.0 mmol) Kaliumcarbonat in 10 ml Acetonitril umgesetzt. Es wurden 412 mg (37 % d. Th.) der Titelverbindung isoliert.
LC-MS [Methode 8]: Rₜ = 2.56 min; MS (ESIpos): m/z = 254 (M + H)⁺
¹H-NMR (400MHz, DMSO-d₆): δ [ppm]= 0.86 (t, 3H), 1.28 (t, 3H), 1.39 - 1.53 (m, 1H), 1.59 (dd, 1H), 3.24 (s, 3H), 3.27 - 3.35 (m, 1H), 3.36 - 3.42 (m, 1H), 4.07 - 4.18 (m, 1H), 4.25 (q, 2H), 7.92 (d, 1H), 8.65 - 8.75 (m, 2H).

### Beispiel 14A

### (rac)-2-[(1-Methoxybutan-2-yl)amino]pyrimidin-5-carbonsäure

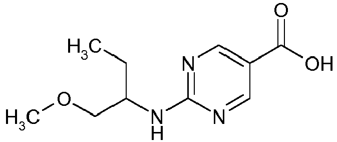

Analog zur Verbindung aus Beispiel 12A wurden 412 mg (1.63 mmol) der Verbindung aus Beispiel 13A umgesetzt. Es wurden 280 mg (76% d. Th.) der Titelverbindung isoliert.
LC-MS [Methode 8]: Rₜ = 1.46 min; MS (ESIpos): m/z = 226 (M + H)⁺
¹H-NMR (400MHz, DMSO-d₆): δ [ppm]= 0.86 (t, 3H), 1.39 - 1.53 (m, 1H), 1.53 - 1.68 (m, 1H), 3.24 (s, 3H), 3.27 -3.34 (m , 1H unter Wasser-Signal), 3.36 - 3.42 (m, 1H), 4.06 - 4.17 (m, 1H), 7.82 (d, 1H), 8.63 - 8.74 (m, 2H), 12.66 (br. s, 1H).

### Beispiel 15A

### (rac)-Ethyl-2-[(1-hydroxybutan-2-yl)amino]pyrimidin-5-carboxylat

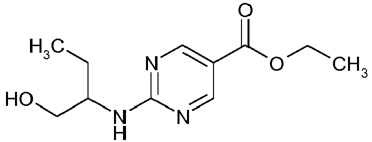

Analog der Verbindung aus Beispiel 11A wurden 0.45 ml (4.8 mmol) DL-2-Amino-1-butanol, 1.00 g (4.34 mmol) Ethyl-2-(methylsulfonyl)pyrimidin-5-carboxylat sowie 1.80 g (13.0 mmol) Kaliumcarbonat in 10 ml Acetonitril umgesetzt. Es wurden 485 mg (46 % d. Th.) der Titelverbindung isoliert.
LC-MS [Methode 1]: Rₜ = 0.75 min; MS (ESIpos): m/z = 240 (M + H)⁺
¹H-NMR (400MHz, DMSO-d₆): δ [ppm]= 0.81 - 0.90 (t, 3H), 1.28 (t, 3H), 1.37 - 1.51 (m, 1H), 1.60 - 1.73 (m, 1H), 3.34 - 3.41 (m, 1H), 3.42 - 3.50 (m, 1H), 3.89 - 3.99 (m, 1H), 4.25 (q, 2H), 4.66 (t, 1H), 7.78 (d, 1H), 8.70 (d, 2H).

### Beispiel 16A

### (rac)-2-[(1-Hydroxybutan-2-yl)amino]pyrimidin-5-carbonsäure

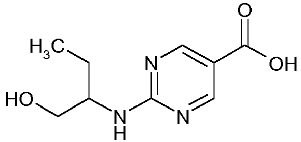

Eine Lösung von 485 mg (2.03 mmol) der Verbindung aus Beispiel 15A in 5.0 ml Ethanol wurden mit 1.4 ml 3N Natriumhydroxidlösung (4.05 mmol) versetzt und über Nacht bei RT gerührt. Zur Aufarbeitung wurde die Reaktionsmischung mit 1N HCl angesäuert. Der ausgefallene Niederschlag wurde abfiltriert, zweimal mit Wasser gewaschen und am HV getrocknet. Die wässrige Phase wurde anschließend noch zweimal mit je 30 ml Essigsäureethylester extrahiert, die organische Phase über Magnesiumsulfat getrocknet, filtriert und eingeengt. Es wurden in Summe 250 mg (58% d. Th.) der Titelverbindung isoliert.
LC-MS [Methode 1]: Rₜ = 0.44 min; MS (ESIpos): m/z = 212 (M + H)⁺
¹H-NMR (400MHz, DMSO-d₆): δ [ppm]= 0.86 (t, 3H), 1.34 - 1.52 (m, 1H), 1.56 - 1.74 (m, 1H), 3.31 - 3.50 (m, 3H), 3.84 - 3.99 (m, 1H), 7.67 (d, 1H), 8.68 (d, 2H), 12.67 (br. s, 1H).

### Beispiel 17A

### Methyl-2-(2-oxa-6-azaspiro[3.3]hept-6-yl)pyrimidin-5-carboxylat

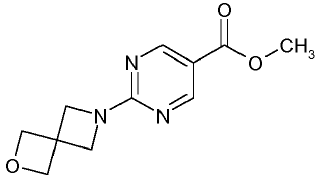

Es wurden 14.70 g (85.18 mmol) Methyl-2-chlorpyrimidin-5-carboxylat in 200 ml Acetonitril gelöst und mit 41.20 mg Kaliumcarbonat (298.14 mmol) versetzt. Anschließend wurden 24.17 g (127.77 mmol) 2-Oxa-6-azaspiro[3.3]heptane-Oxalatsalz, hergestellt nach Angew. Chem. Int. Ed. 2008, 47, 4512-4515, addiert und bei 60°C ca. 16 h gerührt. Anschließend wurde der Ansatz in Wasser ausgerührt und dreimal mit je 200 ml Essigsäureethylester extrahiert. Anschließend wurde die wässrige Phase noch einmal mit ca. 200 ml Dichlormethan extrahiert. Die vereinigten organischen Phasen wurden über Natriumsulfat getrocknet, filtriert und eingeengt. Der Rückstand wurde in ca. 200 ml Diethylether verrührt. Der ausgefallene Feststoff wurde abgesaugt, mit wenig Diethylether gewaschen und im HV getrocknet. Es wurden 17.70 g (88 % d. Th.) der Zielverbindung erhalten.
LC-MS [Methode 1]: Rₜ = 0.61 min; MS (ESIpos): m/z = 236 (M + H)⁺
¹H-NMR (400MHz, DMSO-d₆): δ [ppm]= 3.33 (s, 3H), 4.32 (s, 4H), 4.73 (s, 4H), 8.70 - 8.81 (m, 2H).

### Beispiel 18A

### 2-(2-Oxa-6-azaspiro[3.3]hept-6-yl)pyrimidin-5-carbonsäure

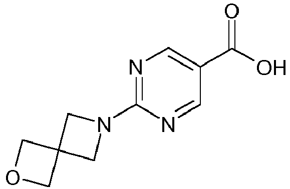

17.7 g Methyl-2-(2-oxa-6-azaspiro[3.3]hept-6-yl)pyrimidin-5-carboxylat (75mmol) wurden in 120 ml Ethanol vorgelegt und mit 148 ml 1 molarer Natriumhydroxidlösung versetzt und über Nacht bei RT gerührt. Der Ansatz wurde eingeengt und anschließend erst in ca. 150 ml Wasser gelöst und dann mit 1 M Salzsäure auf pH 5 gestellt. Das ausgefallene Produkt wurde abgesaugt und mit Wasser gewaschen. Es wurden 16.3 g Produkt (98 % d. Th.) isoliert.
LC-MS [Methode 7]: Rₜ = 0.53 min; MS (ESIpos): m/z = 222 (M + H)⁺
¹H-NMR (400MHz, DMSO-d₆): δ [ppm]= 4.30 (s, 4H), 4.73 (s, 4H), 8.74 (s, 2H), 12.87 (br. s, 1H).

### Beispiel 19A

### Ethyl-2-[(2R)-2-(tert-butoxycarbonyl)pyrrolidin-1-yl]pyrimidin-5-carboxylat

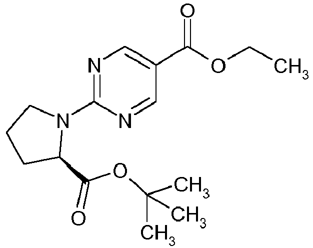

818 mg (4.78 mmol) D-Prolin-t-butylester wurden zu einer Suspension von 1.00 g (4.34 mmol) Ethyl-2-(methylsulfonyl)pyrimidin-5-carboxylat und 2.40 g (17.4 mmol) Kaliumcarbonat in 10 ml Acetonitril getropft. Nach Rühren über Nacht bei RT wurde die Reaktionsmischung mit Essigsäureethylester verdünnt, abfiltriert, der Rückstand mit Essigsäureethylester/Dichlormethan nachgewaschen und das Filtrat eingeengt. Das Rohprodukt wurde chromatographisch an Kieselgel (Elution mit Cyclohexan/Essigsäureethylester 95:5 - 70:30) gereingt wobei 564 mg (40 % d. Th.) der Titelverbindung isoliert wurden.
LC-MS [Methode 1]: Rₜ = 1.19 min; MS (ESIpos): m/z = 322 (M + H)⁺
¹H-NMR (400MHz, DMSO-d₆): δ [ppm]= 1.29 (t, 3H), 1.37 (s, 9H), 1.87 - 2.04 (m, 3H), 2.26 - 2.39 (m, 1H), 3.57 - 3.75 (m, 2H), 4.27 (q, 2H), 4.44 - 4.48 (m, 1H), 8.74 (d, 1H), 8.83 (d, 1H).

### Beispiel 20A

### 2-[(2R)-2-(tert-Butoxycarbonyl)pyrrolidin-1-yl]pyrimidin-5-carbonsäure

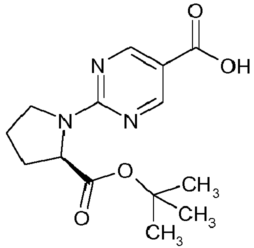

Eine Lösung von 564 mg (1.76 mmol) der Verbindung aus Beispiel 19A in 20 ml THF/Methanol (5:1) wurde mit 8.6 ml 1N Lithiumhydroxydlösung versetzt und über Nacht bei RT gerührt. Zur Aufarbeitung wurde die Reaktionsmischung eingeengt und mit 6N Salzsäure angesäuert und eingeengt. Der erhaltene Rückstand wurde mit Wasser ausgerührt. Der ausgefallene Niederschlag wurde abfiltriert, mit Wasser gewaschen und im Vakuumtrockenschrank bei 50°C getrocknet. Es wurden 400 mg (78% d. Th.) der Titelverbindung isoliert.
LC-MS [Methode 1]: Rₜ = 0.90 min; MS (ESIpos): m/z = 294 (M + H)⁺
¹H-NMR (400MHz, DMSO-d₆): δ [ppm]= 1.37 (s, 9H), 1.87 - 2.04 (m, 3H), 2.25 - 2.37 (m, 1H), 3.56 - 3.73 (m, 2H), 4.41 - 4.49 (m, 1H), 8.71 (d, 1H), 8.81 (d, 1H), 12.41 - 13.33 (br. s, 1H).

### Beispiel 21A

### tert-Butyl-1-(5-{[4-(3,4-dihydroisochinolin-2(1H)-yl)piperidin-1-yl]carbonyl}pyrimidin-2-yl)-D-prolinat

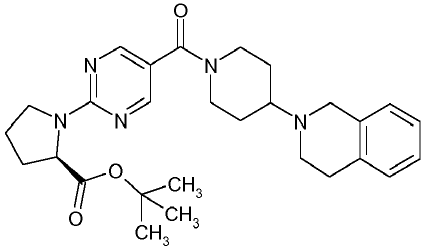

Analog zur Verbindung aus Beispiel 1 wurden 100 mg (0.341 mmol) der Verbindung aus Beispiel 20A, 99 mg (0.341 mmol) der Verbindung aus Beispiel 2A mit 0.42 ml (2.4 mmol) N,N-Diisopropylethylamin und 0.24 ml (0.41 mmol) T3P (50 Gew.-% Lsg. in Essigsäureethylester) umgesetzt. Es wurden 97 mg (58% d. Th.) der Titelverbindung isoliert.
LC-MS [Methode 8]: Rₜ = 2.98 min; MS (ESIpos): m/z = 492 (M + H)⁺
¹H-NMR (400MHz, DMSO-d₆): δ [ppm]= 1.38 (s, 9H), 1.45 - 1.62 (m, 2H), 1.76 - 1.90 (m, 2H), 1.90 - 2.04 (m, 3H), 2.26 - 2.37 (m, 1H), 2.65 - 2.74 (m, 1H), 2.77 (s, 4H), 2.80 - 3.25 (m, 2H), 3.5 - 5.0 (br m, 2H), 3.57 - 3.67 (m, 2H), 3.70 (s, 2H), 4.37 - 4.45 (m, 1H), 7.00 - 7.12 (m, 4H), 8.39 - 8.53 (m, 2H).

### Beispiel 22A

### Methyl-2-(1,1-dioxidothiomorpholin-4-yl)pyrimidin-5-carboxylat

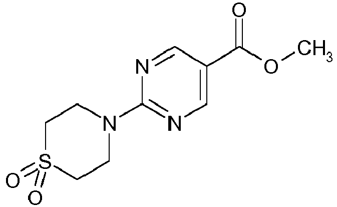

55 mg Methyl-2-chlorpyrimidin-5-carboxylat (0.32 mmol) und 43 mg Thiomorpholin-1,1-dioxid (0.32 mmol) wurden in 1 ml N-Methylmorpholinon vorgelegt und mit 40 mg Natriumcarbonat (0.38 mmol) versetzt. Anschließend wurde 20 h bei 100°C gerührt. Der Ansatz wurde mit Wasser ausgerührt und das ausgefallene Produkt abgesaugt und mit Wasser gewaschen. Es wurden 62 mg (72 % d. Th.) der Zielverbindung isoliert.
LC-MS [Methode 1]: Rₜ = 0.64 min; MS (ESIpos): m/z = 272 (M + H)⁺

### Beispiel 23A

### 2-(1,1-dioxidothiomorpholin-4-yl)pyrimidin-5-carbonsäure

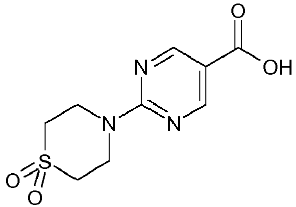

69 mg (0.25 mmol) der Verbindung aus Beispiel 22A wurden in 2 ml Methanol/THF 1/1 vorgelegt und anschließend mit 0.25 ml 2N Natriumhydroxidlösung (0.50 mmol) versetzt. Es wurde 1 h bei 70°C gerührt. Der Ansatz wurde eingeengt und in Wasser aufgenommen. Anschließend wurde mit 1N wässriger Salzsäure angesäuert und zweimal mit ca. 20 ml Essigsäureethylester extrahiert. Die vereinigten organischen Phasen wurden über Natriumsulfat getrocknet, filtriert und eingeengt. Der Rückstand wurde am HV getrocknet. Es wurden 52 mg (79 % d. Th.) der Zielverbindung erhalten, die ohne weitere Aufreinigung weiter umgesetzt wurde.
LC-MS [Methode 1]: Rₜ = 0.48 min; MS (ESIpos): m/z = 258 (M + H)⁺

### Beispiel 24A

### Methyl-2-[-2,6-dimethylmorpholin-4-yl]pyrimidin-5-carboxylat (cis- Isomer)

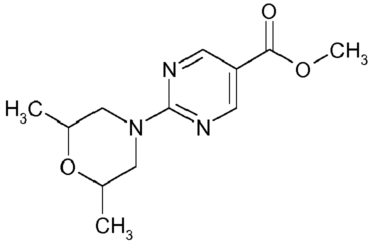

150 mg Methyl-2-chlorpyrimidin-5-carboxylat (0.87 mmol) und 150 mg 2,6-Dimethylmorpholin (1.30 mmol) wurden in 3 ml Acetonitril vorgelegt und mit 420 mg Kaliumcarbonat (3.04 mmol) versetzt. Anschließend wurde 20 h bei 60°C gerührt. Der Ansatz wurde mit Wasser ausgerührt und dann zweimal mit ca. 20 ml Essigsäureethylester extrahiert. Die organischen Phasen wurden über Natriumsulfat getrocknet, filtriert und eingeengt. Das Rohprodukt wurde durch Chromatographie an Kieselgel (Elutionsmittel: Cyclohexan/Essigsäureethylester 10:1 - 5:1) gereinigt. Es wurden 124 mg (57 % d. Th.) der Zielverbindung isoliert.
LC-MS [Methode 1]: Rₜ = 0.97 min; MS (ESIpos): m/z = 252 (M + H)⁺

### Beispiel 25A

### 2-[-2,6-dimethylmorpholin-4-yl]pyrimidin-5-carbonsäure (cis Isomer)

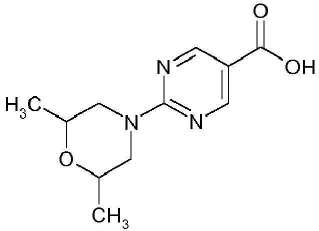

124 mg (0.49 mmol) der Verbindung aus Beispiel 24A wurden in 2 ml Methanol/THF 1:1 vorgelegt und anschließend mit 0.49 ml 2N Natriumhydroxidlösung versetzt. Es wurde 1 h bei 70°C gerührt. Der Ansatz wurde eingeengt und in Wasser aufgenommen. Anschließend wurde mit 1N wässriger Salzsäure angesäuert und zweimal mit ca. 20 ml Essigsäureethylester extrahiert. Die vereinigten organischen Phasen wurden über Natriumsulfat getrocknet, filtriert und eingeengt. Der Rückstand wurde am HV getrocknet. Es wurden 106 mg (91 % d. Th.) der Zielverbindung erhalten, die ohne weitere Aufreinigung weiter umgesetzt wurde.
LC-MS [Methode 1]: Rₜ = 0.72 min; MS (ESIpos): m/z = 238 (M + H)⁺

### Beispiel 26A

### Methyl-2-[-2,6-dimethylmorpholin-4-yl]pyrimidin-5-carboxylat (trans Isomer)

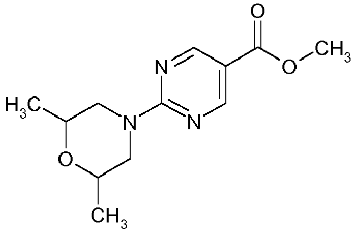

150 mg Methyl-2-chlorpyrimidin-5-carboxylat (0.87 mmol) und 150 mg 2,6-Dimethylmorpholin (1.30 mmol) wurden in 3 ml Acetonitril vorgelegt und mit 420 mg Kaliumcarbonat (3.04 mmol) versetzt. Anschließend wurde 20 h bei 60°C gerührt. Der Ansatz wurde mit Wasser ausgerührt und dann zweimal mit ca. 20 ml Essigsäureethylester extrahiert. Die organischen Phasen wurden über Natriumsulfat getrocknet, dann abfiltriert und eingeengt. Das Rohprodukt wurde durch Chromatographie an Kieselgel (Elutionsmittel: Cyclohexan/Essigsäureethylester 10:1 - 5:1) gereinigt. Es wurden 38 mg Produkt (17 % d. Th.) isoliert.
LC-MS [Methode 1]: Rₜ = 0.91 min; MS (ESIpos): m/z = 252 (M + H)⁺

### Beispiel 27A

### 2-[-2,6-dimethylmorpholin-4-yl]pyrimidin-5-carbonsäure (trans Isomer)

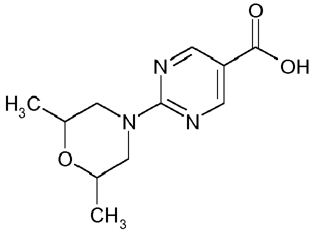

35 mg (0.14 mmol) der Verbindung aus Beispiel 26A wurden in 2 ml Methanol/THF 1:1 vorgelegt und anschließend mit 0.14 ml (0.28 mmol) 2N Natriumhydroxidlösung versetzt. Es wurde 1 h bei 70°C gerührt. Der Ansatz wurde eingeengt und in Wasser aufgenommen. Anschließend wurde mit 1N wässriger Salzsäure angesäuert und zweimal mit ca. 20 ml Essigsäureethylester extrahiert. Die vereinigten organischen Phasen wurden über Natriumsulfat getrocknet, filtriert und eingeengt. Der Rückstand wurde am HV getrocknet. Es wurden 27 mg (78 % d. Th.) der Zielverbindung erhalten, die ohne weitere Aufreinigung weiter umgesetzt wurde.
LC-MS [Methode 1]: Rₜ = 0.68 min; MS (ESIpos): m/z = 238 (M + H)⁺

### Beispiel 28A

### Methyl-2-(2,2-dimethylmorpholin-4-yl)pyrimidin-5-carboxylat

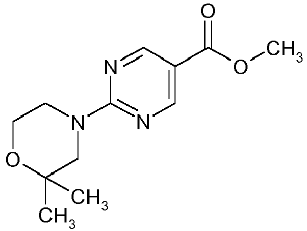

75 mg Methyl-2-chlorpyrimidin-5-carboxylat (0.44 mmol) und 99 mg 2,2-Dimethylmorpholin Hydrochlorid (0.65 mmol) wurden in 3 ml Acetonitril vorgelegt und mit 300 mg Kaliumcarbonat (2.17 mmol) versetzt. Anschließend wurde 20 h bei 60°C gerührt. Der Ansatz wurde mit Wasser ausgerührt und dann zweimal mit ca. 20 ml Essigsäureethylester extrahiert. Die organischen Phasen wurden über Natriumsulfat getrocknet, dann abfiltriert und eingeengt. Das Rohprodukt wurde durch Chromatographie an Kieselgel (Elutionsmittel: Cyclohexan/Essigsäureethylester 10:1 - 5:1) gereinigt. Es wurden 104 mg Produkt (95 % d. Th.) isoliert.
LC-MS [Methode 1]: Rₜ = 0.91 min; MS (ESIpos): m/z = 252 (M + H)⁺

### Beispiel 29A

### Methyl-2-(2,2-dimethylmorpholin-4-yl)pyrimidin-5-carbonsäure

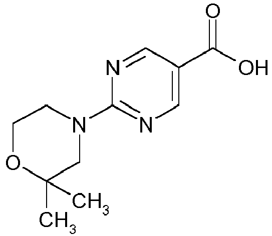

104 mg (0.41 mmol) der Verbindung aus Beispiel 28A wurden in 2 ml Methanol/THF 1:/1 vorgelegt und anschließend mit 0.41 ml (0.82 mmol) 2N Natriumhydroxidlösung versetzt. Es wurde 1 h bei 70°C gerührt. Der Ansatz wurde eingeengt und in Wasser aufgenommen. Anschließend wurde mit 1N wässriger Salzsäure angesäuert und zweimal mit ca. 20 ml Essigsäureethylester extrahiert. Die vereinigten organischen Phasen wurden über Natriumsulfat getrocknet, filtriert und eingeengt. Der Rückstand wurde am HV getrocknet. Es wurden 86 mg (88 % d. Th.) der Zielverbindung erhalten, die ohne weitere Aufreinigung weiter umgesetzt wurde.
LC-MS [Methode 1]: Rₜ = 0.67 min; MS (ESIpos): m/z = 238 (M + H)+

### Ausführungsbeispiele

### Beispiel 1

### [4-(3,4-Dihydroisochinolin-2(1H)-yl)piperidin-1-yl]{2-[(2-methoxyethyl)amino]pyrimidin-5-yl}methanon

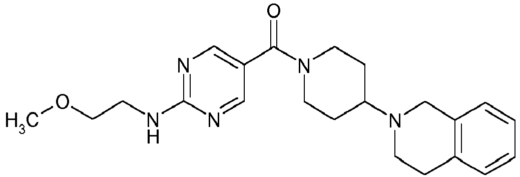

Eine Mischung von 56 mg (0.28 mmol) der Verbindung aus Beispiel 12A und 72 mg (0.29 mmol) der Verbindung aus Beispiel 2A in 2.4 ml Acetonitril wurde mit 0.35 ml (2.0 mmol) N,N-Diisopropylethylamin und 0.20 ml (0.34 mmol) T3P (50 Gew.-% Lsg. in Essigsäureethylester) versetzt und anschließend über Nacht bei RT gerührt. Zur Aufarbeitung wurde 1 ml gesättigte Natriumhydrogencarbonatlösung zugesetzt, 15 min gerührt, über eine Extrelut-Kartusche filtriert, mit Dichlormethan eleuiert und das Filtrat eingeengt. Das erhaltene Rohprodukt wurde mittels präparativer HPLC gereinigt [Methode 9] wobei 47 mg (41% d. Th.) der Titelverbindung isoliert wurden.
LC-MS [Methode 8]: Rₜ = 2.34 min; MS (ESIpos): m/z = 396 (M + H)⁺
¹H-NMR (400MHz, DMSO-d₆): δ [ppm]= 1.45 - 1.62 (m, 2H), 1.79 - 1.92 (m, 2H), 2.63 - 2.74 (m, 1H), 2.77 (s, 4H), 2.81 - 3.19 (m, 2H), 3.26 (s, 3H), 3.41 - 3.52 (m, 4H), 3.70 (s, 2H), 3.78 - 4.64 (m, 2H), 7.00 - 7.21 (m, 4H), 7.57 - 7.65 (m, 1H), 8.29 - 8.44 (m, 2H).

### Beispiel 2

### (rac)-[4-(7-Fluor-3,4-dihydroisochinolin-2(1H)-yl)piperidin-1-yl]{2-[(1-methoxybutan-2-yl)amino]pyrimidin-5-yl}methanon

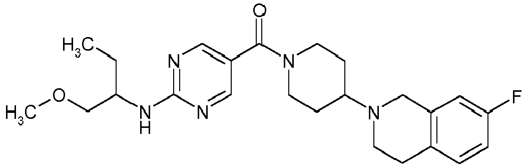

Analog zur Verbindung aus Beispiel 1 wurden 56 mg (0.249 mmol) der Verbindung aus Beispiel 13A, 76.4 mg (0.294 mmol) der Verbindung aus Beispiel 4A mit 0.30 ml (1.7 mmol) N,N-Diisopropylethylamin und 0.17 ml (0.30 mmol) T3P (50 Gew.-% Lsg. in Essigsäureethylester) umgesetzt. Es wurden 56.0 mg (51% d. Th.) der Titelverbindung isoliert.
LC-MS [Methode 8]: Rₜ = 2.63 min; MS (ESIpos): m/z = 442 (M + H)⁺
¹H-NMR (400MHz, DMSO-d₆): δ [ppm]= 0.87 (t, 3H), 1.39 - 1.70 (m, 4H), 1.77 - 1.91 (m, 2H), 2.63 - 2.80 (m, 5H), 2.81 - 3.16 (m, 2H), 3.24 (s, 3H), 3.27 - 3.34 (m, 1H unter dem Wasser-Signal), 3.36 - 3.43 (m, 1H), 3.70 (s, 2H), 3.79 - 4.47 (m, 3H), 6.85 - 6.97 (m, 2H), 7.06 - 7.15 (m, 1H), 7.44 (d, 1H), 8.36 (s, 2H).

### Beispiel 3

### (rac)-[4-(6-Fluor-3,4-dihydroisochinolin-2(1H)-yl)piperidin-1-yl]{2-[(1-methoxybutan-2-yl)amino]pyrimidin-5-yl}methanon

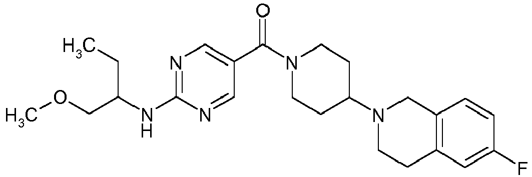

Analog zur Verbindung aus Beispiel 1 wurden 56 mg (0.249 mmol) der Verbindung aus Beispiel 13A, 76.4 mg (0.294 mmol) der Verbindung aus Beispiel 6A mit 0.30 ml (1.7 mmol) N,N-Diisopropylethylamin und 0.17 ml (0.30 mmol) T3P (50 Gew.-% Lsg. in Essigsäureethylester) umgesetzt. Es wurden 61 mg (55% d. Th.) der Titelverbindung isoliert.
LC-MS [Methode 8]: Rₜ = 2.62 min; MS (ESIpos): m/z = 442 (M + H)⁺
¹H-NMR (400MHz, DMSO-d₆): δ [ppm]= 0.87 (t, 3H), 1.40 - 1.67 (m, 4H), 1.78 - 1.92 (m, 2H), 2.64 - 2.83 (m, 5H), 2.83 - 3.14 (m, 2H), 3.24 (s, 3H), 3.27 - 3.35 (m, 1H unter Wasser-Signal), 3.35 - 3.42 (m, 1H), 3.68 (s, 2H), 3.72 - 4.55 (m, 3H), 6.88 - 6.96 (m, 2H), 7.04 - 7.12 (m, 1H), 7.43 (d, 1H), 8.36 (s, 2H).

### Beispiel 4

### (rac)-{2-[(1-Methoxybutan-2-yl)amino]pyrimidin-5-yl}[4-(7-methoxy-3,4-dihydroisochinolin-2(1H)-yl)piperidin-1-yl]methanon

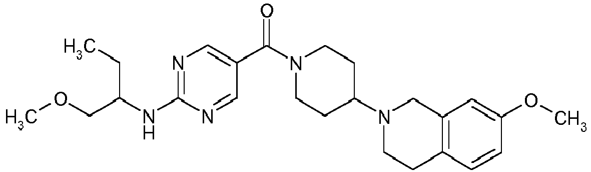

Analog zur Verbindung aus Beispiel 1 wurden 56 mg (0.25 mmol) der Verbindung aus Beispiel 13A, 79 mg (0.29 mmol) der Verbindung aus Beispiel 8A mit 0.30 ml (1.7 mmol) N,N-Diisopropylethylamin und 0.17 ml (0.30 mmol) T3P (50 Gew.-% Lsg. in Essigsäureethylester) umgesetzt. Es wurden 67 mg (59% d. Th.) der Titelverbindung isoliert.
LC-MS [Methode 8]: Rₜ = 2.57 min; MS (ESIpos): m/z = 454 (M + H)⁺
¹H-NMR (400MHz, DMSO-d₆): δ [ppm]= 0.87 (t, 3H), 1.39 - 1.70 (m, 4H), 1.78 - 1.90 (m, 2H), 2.63 - 2.78 (m, 5H), 2.81 - 3.13 (m, 2H), 3.24 (s, 3H), 3.31 (m, 1H unter Wasser-Signal), 3.35 - 3.42 (m, 1H), 3.63 - 3.73 (m, 5H), 3.74 - 4.51 (m, 3H), 6.61 (d, 1H), 6.65 - 6.71 (m, 1H), 6.98 (d, 1H), 7.43 (d, 1H), 8.36 (s, 2H).

### Beispiel 5

### (rac)-{2-[(1-Methoxybutan-2-yl)amino]pyrimidin-5-yl}[4-(6-methoxy-3,4-dihydroisochinolin-2(1H)-yl)piperidin-1-yl]methanon

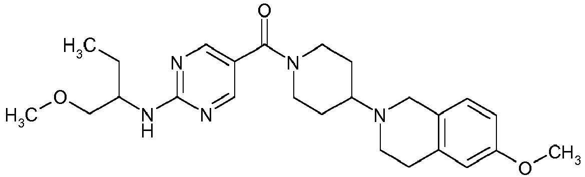

Analog zur Verbindung aus Beispiel 1 wurden 56 mg (0.25 mmol) der Verbindung aus Beispiel 13A, 79 mg (0.29 mmol) der Verbindung aus Beispiel 10A mit 0.30 ml (1.7 mmol) N,N-Diisopropylethylamin und 0.17 ml (0.30 mmol) T3P (50 Gew.-% Lsg. in Essigsäureethylester) umgesetzt. Es wurden 52 mg (46% d. Th.) der Titelverbindung isoliert.
LC-MS [Methode 8]: Rₜ = 2.55 min; MS (ESIpos): m/z = 454 (M + H)⁺
¹H-NMR (400MHz, DMSO-d₆): δ [ppm]= 0.87 (t, 3H), 1.39 - 1.67 (m, 4H), 1.77 - 1.90 (m, 2H), 2.62 - 2.79 (m, 5H), 2.81 - 3.13 (m, 2H), 3.24 (s, 3H), 3.27 - 3.34 (m, 1H unter Wasser-Signal), 3.35 - 3.43 (m, 1H), 3.63 (s, 2H), 3.69 (s, 3H), 3.82 - 4.43 (m, 3H), 6.64 (d, 1H), 6.65 - 6.71 (m, 1H), 6.95 (d, 1H), 7.43 (d, 1H), 8.36 (s, 2H).

### Beispiel 6

### (rac)-[4-(3,4-Dihydroisochinolin-2(1H)-yl)piperidin-1-yl]{2-[(1-methoxybutan-2-yl)amino]pyrimidin-5-yl}methanon

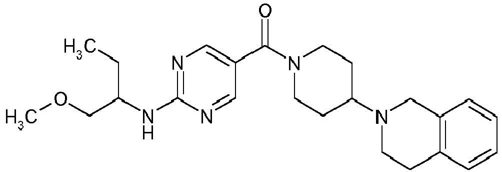

Analog zur Verbindung aus Beispiel 1 wurden 56 mg (0.25 mmol) der Verbindung aus Beispiel 13A und 63 mg (0.29 mmol) der Verbindung aus Beispiel 2A mit 0.30 ml (1.7 mmol) N,N-Diisopropylethylamin und 0.17 ml (0.30 mmol) T3P (50 Gew.-% Lsg. in Essigsäureethylester) umgesetzt. Es wurden 52 mg (46% d. Th.) der Titelverbindung isoliert.
LC-MS [Methode 8]: Rₜ = 2.60 min; MS (ESIpos): m/z = 424 (M + H)⁺
¹H-NMR (400MHz, DMSO-d₆): δ [ppm]= 0.87 (t, 3H), 1.38 - 1.68 (m, 4H), 1.79 - 1.92 (m, 2H), 2.64 - 2.74 (m, 1H), 2.77 (s, 4H), 2.81 - 3.12 (m, 2H), 3.24 (s, 3H), 3.27 - 3.33 (m, 1H unter Wasser-Signal), 3.35 - 3.42 (m, 1H), 3.70 (s, 2H), 3.75 - 4.40 (m, 3H), 6.99 - 7.14 (m, 4H), 7.43 (d, 1H), 8.36 (s, 2H).

### Beispiel 7

### (rac)-[4-(3,4-Dihydroisochinolin-2(1H)-yl)piperidin-1-yl]{2-[(1-hydroxybutan-2-yl)amino]pyrimidin-5-yl}methanon

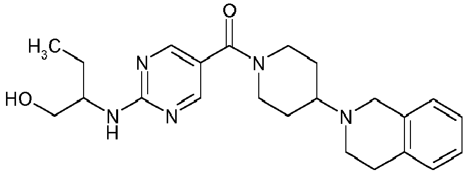

Eine Mischung von 50 mg (0.24 mmol) der Verbindung aus Beispiel 16A und 60 mg (0.24 mmol) der Verbindung aus Beispiel 2A in 2.0 ml Acetonitril wurde mit 0.29 ml (1.7 mmol) N,N-Diisopropylethylamin und 0.17 ml (0.28 mmol) T3P (50 Gew.-% Lsg. in Essigsäureethylester) versetzt und anschließend über Nacht bei RT gerührt. Zur Aufarbeitung wurde 1 ml gesättigter Natriumhydrogencarbonatlösung zugesetzt, 15 min gerührt, über eine Extrelut-Kartusche filtriert, mit Dichlormethan eluiert und das Filtrat eingeengt. Das erhaltene Rohprodukt wurde mittels präparativer HPLC gereinigt [Methode 9] wobei 53 mg (54% d. Th.) der Titelverbindung isoliert wurden.
LC-MS [Methode 8]: Rₜ = 2.29 min; MS (ESIpos): m/z = 410 (M + H)⁺
¹H-NMR (400MHz, DMSO-d₆): δ [ppm]= 0.87 (t, 3H), 1.38 - 1.60 (m, 3H), 1.60 - 1.74 (m, 1H), 1.80 - 1.91 (m, 2H), 2.65 - 2.74 (m, 1H), 2.77 (s, 4H), 2.80 - 3.15 (m, 2H), 3.33 - 3.40 (m, 1H), 3.42 - 3.50 (m, 1H), 3.70 (s, 2H), 3.82 - 4.56 (m, 3H), 4.62 (t, 1H), 7.01 - 7.12 (m, 4H), 7.23 - 7.31 (m, 1H), 8.35 (s, 2H).

### Beispiel 8

### [4-(3,4-Dihydroisochinolin-2(1H)-yl)piperidin-1-yl][2-(2-oxa-6-azaspiro[3.3]hept-6-yl)pyrimidin-5-yl]methanon

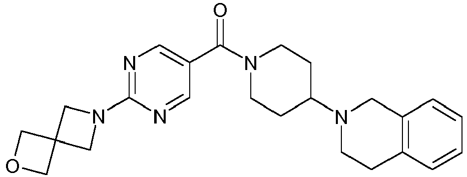

Eine Mischung von 15.5 g (70.1 mmol) der Verbindung aus Beispiel 18A und 20.27 g (70.1 mmol) der Verbindung aus Beispiel 2A in 320 ml Acetonitril wurde mit 61.0 ml (350.3 mmol) N,N-Diisopropylethylamin und 50.05 ml (84.1 mmol) T3P (50 Gew.-% Lsg. in Essigsäureethylester) versetzt und anschließend 3 h bei RT gerührt. Zur Aufarbeitung wurden 100 ml gesättigte Natriumhydrogencarbonatlösung zugesetzt und 10 min bei RT gerührt. Anschließend wurde mit weiteren 200 ml gesättigter Natriumhydrogencarbonatlösung versetzt und mi 500 ml Essigsäureethylester extrahiert. Die organische Phase wurde je einmal mit gesättigter Natriumhydrogencarbonatlösung und Natriumchloridlösung gewaschen, über Natriumsulfat getrocknet, filtriert und eingeengt. Das erhaltene Rohprodukt wurde mit 100 ml Methanol versezt auf 55°C erhitzt wobei keine klare Lösung entstand. Unter Rühren wurde auf RT abgekühlt und mit 250 ml Diethylether versetzt. Nach 30 min wurde der ausgefallene Feststoff abgesaugt, mit wenig Diethylether gewaschen und im HV getrocknet. Es wurden 17.2 g (59% d. Th.) der Zielverbindung erhalten.
LC-MS [Methode 1]: Rₜ = 0.50 min; MS (ESIpos): m/z = 420 (M + H)⁺
¹H-NMR (400MHz, DMSO-d₆): δ [ppm]= 1.43 - 1.60 (m, 2H), 1.77 - 1.93 (m, 2H), 2.63 - 2.73 (m, 1H), 2.77 (s, 4H), 2.81 - 3.15 (m, 2H), 3.5 - 4.7 (br. M, 2H), 3.70 (s, 2H), 4.26 (s, 4H), 4.73 (s, 4H), 6.99 - 7.13 (m, 4H), 8.43 (s, 2H).

### Beispiel 9

### [4-(7-Fluor-3,4-dihydroisochinolin-2(1H)-yl)piperidin-1-yl][2-(2-oxa-6-azaspiro[3.3]hept-6-yl)pyrimidin-5-yl] methanon

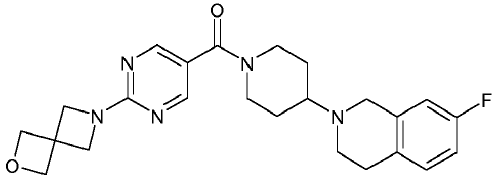

Eine Mischung von 58 mg (0.23 mmol) der Verbindung aus Beipiel 18A und 69 mg (0.23 mmol) der Verbindung aus Beispiel 4A in 1.9 ml Acetonitril wurde mit 0.28 ml (1.6 mmol) N,N-Diisopropylethylamin und 0.16 ml (0.27 mmol) T3P (50 Gew.-% Lsg. in Essigsäureethylester) versetzt und anschließend über Nacht bei RT gerührt. Zur Aufarbeitung wurde 1 ml gesättigter Natriumhydrogencarbonatlösung zugesetzt, 15 min gerührt, über eine Extrelut-Kartusche filtriert, mit Dichlormethan eluiert und das Filtrat eingeengt. Das erhaltene Rohprodukt wurde mittels präparativer HPLC gereinigt [Methode 9] wobei 30 mg (29% d. Th.) der Titelverbindung isoliert wurden.
LC-MS [Methode 8]: Rₜ = 2.29 min; MS (ESIpos): m/z = 438 (M + H)⁺
¹H-NMR (400MHz, DMSO-d₆): δ [ppm]= 1.43 - 1.57 (m, 2H), 1.77 - 1.89 (m, 2H), 2.75 (s, 5H), 2.79 - 3.24 (m, 2H), 3.70 (s, 2H), 3.00 - 5.00 (br m, 2H unter Wasser-Signal), 4.26 (s, 4H), 4.73 (s, 4H), 6.86 - 6.96 (m, 2H), 7.11 (dd, 1H), 8.43 (s, 2H).

### Beispiel 10

### [4-(6-Methoxy-3,4-dihydroisochinolin-2(1H)-yl)piperidin-1-yl][2-(2-oxa-6-azaspiro[3.3]hept-6-yl)pyrimidin-5-yl] methanon

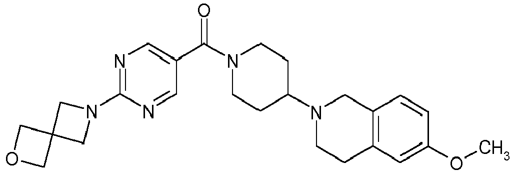

Eine Mischung von 58 mg (0.23 mmol) der Verbindung aus Beipiel 18A und 72 mg (0.23 mmol) der Verbindung aus Beipiel 10A in 1.9 ml Acetonitril wurde mit 0.28 ml (1.6 mmol) N,N-Diisopropylethylamin und 0.16 ml (0.27 mmol) T3P (50 Gew.-% Lsg. in Essigsäureethylester) versetzt und anschließend über Nacht bei RT gerührt. Zur Aufarbeitung wurde 1 ml gesättigter Natriumhydrogencarbonatlösung zugesetzt, 15 min gerührt, über eine Extrelut-Kartusche filtriert, mit Dichlormethan eluiert und das Filtrat eingeengt. Das erhaltene Rohprodukt wurde mittels präparativer HPLC gereinigt [Methode 9] wobei 30 mg (29% d. Th.) der Titelverbindung isoliert wurden.
LC-MS [Methode 8]: Rₜ = 2.21 min; MS (ESIpos): m/z = 450 (M + H)⁺
¹H-NMR (400MHz, DMSO-d₆): δ [ppm]= 1.44 - 1.58 (m, 2H), 1.79 - 1.89 (m, 2H), 2.60 - 2.78 (m, 5H), 2.79 - 3.21 (m, 2H), 3.00 - 5.00 (br m, 2H unter Wasser-Signal), 3.62 (s, 2H), 3.69 (s, 3H), 4.26 (s, 4H), 4.72 (s, 4H), 6.62 - 6.70 (m, 2H), 6.94 (d, 1H), 8.43 (s, 2H).

### Beispiel 11

### 1-(5-{[4-(3,4-Dihydroisochinolin-2(1H)-yl)piperidin-1-yl]carbonyl}pyrimidin-2-yl)-D-prolin Hydrochlorid

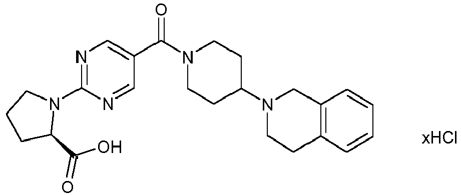

Eine Lösung von 90 mg (0.183 mmol) der Verbindung aus Beispiel 21A in 3.5 ml Dichlormethan wurde mit 0.46 ml 4N Chlorwassertstoff in Dioxan versetzt und über Nacht bei RT gerührt. Anschließend wurden erneut 0.46 ml 4N Chlorwasserstoff in Dioxan zugesetzt und bis zum vollständigen Umsatz des Startmaterials gerührt. Anschließend wurde das Reaktionsgemisch eingeengt und der erhaltene Rückstand wurde mit Diethylether ausgerührt. Der Feststoff wurde abfiltriert und im HV getrocknet wobei 82 mg (94% d. Th.) der Titelverbindung erhalten wurden.
LC-MS [Methode 1]: Rₜ = 0.53 min; MS (ESIpos): m/z = 436 (M + H)⁺
¹H-NMR (400MHz, DMSO-d₆): δ [ppm]= 1.71 - 1.89 (m, 2H), 1.89 - 2.09 (m, 3H), 2.09 - 2.25 (m, 2H), 2.29 - 2.38 (m, 1H), 2.80 - 3.42 (m, 6H), 3.00 - 5.00 (br m, 3H unter Wasser-Signal), 3.86 - 4.38 (m, 2H), 4.39 - 4.51 (m, 3H), 7.17 - 7.34 (m, 4H), 8.41 - 8.56 (m, 2H), 10.51 - 10.65 (m, 1H), 11.54 - 13.23 (m, 1H).

### Beispiel 12

### [4-(3,4-Dihydroisochinolin-2(1H)-yl)piperidin-1-yl][2-(1,1-dioxidothiomorpholin-4-yl)pyrimidin-5-yl]methanon

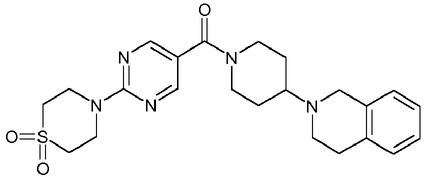

51 mg (0.20 mmol) der Verbindung aus Beispiel 23A und 57 mg (0.20 mmol) der Verbindung aus Beispiel 2A wurden in 2 ml Acetonitril vorgelgt und mit 0.17 ml N,N-Diisopropylethylamin (0.99 mmol) versetzt. Anschließend wurden 0.14 ml (0.24 mmol) T3P (50 Gew.-% Lsg. in Essigsäureethylester) zugetropft und über Nacht bei RT gerührt. Nach Einengen wurde der Rückstand mit 20 ml Essigsäureethylester verdünnt und mit ca. 10 ml gesättigter wässriger Natriumhydrogencarbonatlösung versetzt. Nach 10 min wurde mit Wasser verdünnt und zweimal mit je 20 ml Essigsäureethylester extrahiert. Die vereinigten organischen Phasen wurden über Natriumsulfat getrocknet, anschließend filtriert und das Filtrat eingeengt. Das erhaltene Rohprodukt wurde mittels präparativer HPLC gereinigt [Methode 9]. Es wurden 62 mg (68 % d. Th.) der Zielverbindung isoliert.
LC-MS [Methode 1]: Rₜ = 0.54 min; MS (ESIpos): m/z = 456 (M + H)⁺
¹H-NMR (400MHz, DMSO-d₆): δ [ppm]= 1.37 - 1.68 (m, 2H), 1.75 - 1.99 (m, 2H), 2.44 - 2.52 (m, 4H), 2.78 (br. s, 4H), 3.13 - 3.27 (m, 4H), 3.50 - 4.07 (m, 3H), 4.25 (br. s., 4H), 7.00 - 7.17 (m, 4H), 8.54 (s, 2H).

### Beispiel 13

### [4-(3,4-Dihydroisochinolin-2(1H)-yl)piperidin-1-yl][2-(2,6-dimethylmorpholin-4-yl)pyrimidin-5-yl]methanon (cis Isomer)

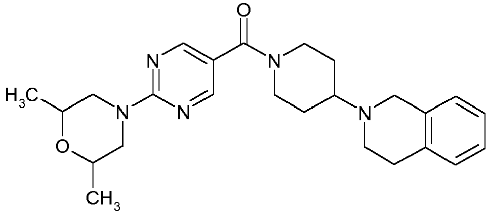

106 mg (0.45 mmol) der Verbindung aus Beispiel 25A und 130 mg (0.45 mmol) der Verbindung aus Beispiel 2A wurden in 2 ml Acetonitril vorgelegt und mit 0.39 ml N,N-Diisopropylethylamin (2.23 mmol) versetzt. Anschließend wurden 0.32 ml (0.54 mmol) T3P (50 Gew.-% Lsg. in Essigsäureethylester) zugetropft und über Nacht bei RT gerührt. Nach Einengen wurde der Rückstand mit 20 ml Essigsäureethylester verdünnt und mit ca. 10 ml gesättigter wässriger Natriumhydrogencarbonatlösung versetzt. Nach 10 min wurde mit Wasser verdünnt und zweimal mit je 20 ml Essigsäureethylester extrahiert. Die vereinigten organischen Phasen wurden über Natriumsulfat getrocknet, anschließend filtriert und das Filtrat eingeengt. Das erhaltene Rohprodukt wurde mittels präparativer HPLC gereinigt [Methode 9]. Es wurden 125 mg (58 % d. Th.) der Zielverbindung isoliert.
LC-MS [Methode 1]: Rₜ = 0.63 min; MS (ESIpos): m/z = 436 (M + H)⁺
¹H-NMR (400MHz, DMSO-d₆): δ [ppm]= 1.15 (s, 3H), 1.16 (s, 3H), 1.46 - 1.58 (m, 2H), 1.79 - 1.93 (m, 2H), 2.54 - 2.63 (m, 2H), 2.65 - 2.73 (m, 1H), 2.77 (s, 4H), 2.80 - 3.20 (br. m, 2H), 3.31 (s, 2H), 3.50 - 3.61 (m, 2H), 3.80 - 4.50 (br. m, 2H), 4.51 - 4.60 (m, 2H), 7.00 - 7.12 (m, 4H), 8.46 (s, 2H).

### Beispiel 14

### [4-(3,4-Dihydroisochinolin-2(1H)-yl)piperidin-1-yl][2-(2,6-dimethylmorpholin-4-yl)pyrimidin-5-yl]methanon (trans Isomer)

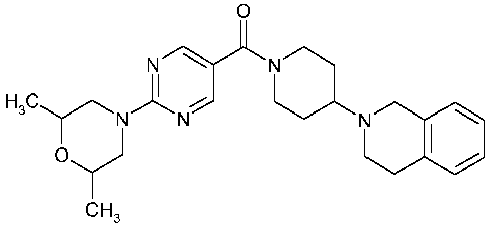

27 mg (0.11 mmol) der Verbindung aus Beispiel 27A und 33 mg (0.11 mmol) der Verbindung aus Beispiel 2A wurden in 2 ml Acetonitril vorgelgt und mit 0.10 ml N,N-Diisopropylethylamin (0.57 mmol) versetzt. Anschließend wurden 0.08 ml (0.14 mmol) T3P (50 Gew.-% Lsg. in Essigsäureethylester) zugetropft und über Nacht bei RT gerührt. Nach Einengen wurde der Rückstand mit 20 ml Essigsäureethylester verdünnt und mit ca. 10 ml gesättigter wässriger Natriumhydrogencarbonatlösung versetzt. Nach 10 min wurde mit Wasser verdünnt und zweimal mit je 20 ml Essigsäureethylester extrahiert. Die vereinigten organischen Phasen wurden über Natriumsulfat getrocknet, anschließend filtriert und das Filtrat eingeengt. Das erhaltene Rohprodukt wurde mittels präparativer HPLC gereinigt [Methode 9]. Es wurden 32 mg (65 % d. Th.) der Zielverbindung isoliert.
LC-MS [Methode 1]: Rₜ = 0.61 min; MS (ESIpos): m/z = 436 (M + H)⁺
¹H-NMR (400MHz, DMSO-d₆): δ [ppm]= 1.12 (s, 3H), 1.14 (s, 3H), 1.44 - 1.61 (m, 2H), 1.79 - 1.91 (m, 2H), 2.64 - 2.80 (m, 5H), 2.80 - 3.20 (br. m, 2H), 3.45 - 3.56 (m, 2H), 3.70 (s, 2H), 3.80 - 4.50 (br. m, 2H), 3.83 - 3.93 (m, 2H), 3.94 - 4.06 (m, 2H), 6.99 - 7.12 (m, 4H), 8.45 (s, 2H).

### Beispiel 15

### [4-(3,4-Dihydroisochinolin-2(1H)-yl)piperidin-1-yl][2-(2,2-dimethylmorpholin-4-yl)pyrimidin-5-yl]methanon

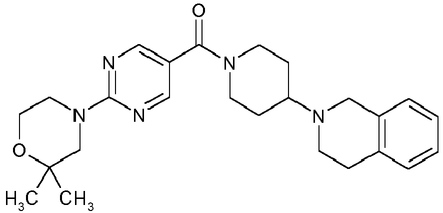

86 mg (0.36 mmol) der Verbindung aus Beispiel 29A und 105 mg (0.36 mmol) der Verbindung aus Beispiel 2A wurden in 2 ml Acetonitril vorgelgt und mit 0.32 ml N,N-Diisopropylethylamin (1.81 mmol) versetzt. Anschließend wurden 0.26 ml (0.44 mmol) T3P (50 Gew.-% Lsg. in Essigsäureethylester) zugetropft und über Nacht bei RT gerührt. Nach Einengen wurde der Rückstand mit 20 ml Essigsäureethylester verdünnt und mit ca. 10 ml gesättigter wässriger Natriumhydrogencarbonatlösung versetzt. Nach 10 min wurde mit Wasser verdünnt und zweimal mit je 20 ml Essigsäureethylester extrahiert. Die vereinigten organischen Phasen wurden über Natriumsulfat getrocknet, anschließend filtriert und das Filtrat eingeengt. Das erhaltene Rohprodukt wurde mittels präparativer HPLC gereinigt [Methode 9]. Es wurden 116 mg (73 % d. Th.) der Zielverbindung isoliert. LC-MS [Methode 1]: Rₜ = 0.70 min; MS (ESIpos): m/z = 436 (M + H)⁺
¹H-NMR (400MHz, DMSO-d₆): δ [ppm]= 1.17 (s, 6H), 1.45 - 1.60 (m, 2H), 1.80 - 1.91 (m, 2H), 2.65 - 2.80 (m, 6H), 2.80 - 3.20 (br. m, 2H), 3.64 (s, 2H), 3.67 - 3.72 (m, 3H), 3.73 - 3.79 (m, 2H), 3.80 - 4.50 (br. m, 2H), 7.00 - 7.13 (m, 4H), 8.46 (s, 2H).

### B) Bewertung der physiologischen Wirksamkeit

Die Eignung der erfindungsgemäßen Verbindungen zur Behandlung von kardiovaskulären Erkrankungen kann in folgenden Assaysystemen gezeigt werden:

### B-1) In vitro Assays

### B-1a) Antagonismus auf Adrenorezeptoren

Antagonismus auf dem Adrenorezeptor α_{1A} wurde an einer rekombinanten humanen α_{1A} Rezeptor CHO Zelllinie getestet, die zusätzlich auch rekombinant mtAeq (mitochondriales Aequorin) exprimiert. Antagonismus auf dem Adrenorezeptor α_{2A} wurde an einer rekombinanten humanen α_{2A}-Gα16 Rezeptor-Fusionsprotein CHO Zelllinie (PerkinElmer Life Sciences) getestet, die zusätzlich auch rekombinant mtAeq exprimiert. Antagonismus auf dem Adrenorezeptor α_{2B} wurde an einer rekombinanten humanen α_{2B} Rezeptor CHO Zelllinie (PerkinElmer Life Sciences) getestet, die zusätzlich auch rekombinant mtAeq exprimiert. Antagonismus auf dem Adrenorezeptor α_{2C} wurde an einer rekombinanten humanen α_{2C} Rezeptor CHO Zelllinie getestet, die zusätzlich auch rekombinant ein chimäres G Protein (Gaqi3) und mtOb (mitochondriales Obelin) exprimiert.

Die Zellen wurden bei 37°C und 5% CO₂ kultiviert in Dulbecco's modifiziertem Eagle's Medium/NUT mix F12 mit L-Glutamin, welches zusätzlich 10% (v/v) inaktiviertes fötales Kälberserum, 1 mM Natriumpyruvat, 0.9 mM Natriumbicarbonat, 50 U/ml Penicillin, 50 µg/ml Streptomycin, 2.5 µg/ml Amphotericin B und 1 mg/ml Geneticin enthält. Die Zellen wurden mit enzymfreiem Hank's-basierten Zelldissoziationspuffer passagiert. Alle verwendeten Zellkultur-Reagentien stammten von Invitrogen (Carlsbad, USA).

Lumineszenz-Messungen wurden auf weißen 384-Loch Mikrotiterplatten durchgeführt. 2000 Zellen/Loch wurden in einem Volumen von 25 µl ausplattiert und für einen Tag bei 30°C und 5% CO₂ in Zellkultur-Medium mit Coelenterazin (α_{2A} und α_{2B}: 5 µg/ml; α_{1a/c} und α_{2C}: 2.5 µg/ml) kultiviert. Serielle Verdünnungen der Testsubstanzen (10 µl) wurden auf die Zellen gegeben. Nach 5 Minuten wurde Noradrenalin auf die Zellen gegeben (35 µl; Endkonzentrationen: 20 nM (α_{1a/c} und α_{2C}) bzw. 200 nM (α_{2A} und α_{2B})) und das emittierte Licht wurde für 50 Sekunden mittels einer CCD- (Charge-Coupled Device) Kamera (Hamamatsu Corporation, Shizuoka, Japan) in einer lichtdichten Box gemessen. Die Testsubstanzen wurden bis zu einer maximalen Konzentration von 10 µM getestet. Die IC₅₀-Werte wurden aus den entsprechenden Dosis-Wirkungskurven berechnet. Die Ergebnisse für den Antagonismus auf dem Adrenorezeptor α_{2C} sind in Tabelle 1 gezeigt:

**Tabelle 1:**

| **Beispiel-Nr.** | **IC₅₀ [nM]** | **Beispiel-Nr.** | **IC₅₀ [nM]** | **Beispiel-Nr.** | **IC₅₀ [nM]** |
|---|---|---|---|---|---|
| **1** | 82 | **2** | 26 | **3** | 24 |
| **4** | 5 | **5** | 20 | **6** | 31 |
| **7** | 68 | **8** | 23 | **9** | 16 |
| **10** | 30 | **11** | 96 | **12** | 26 |
| **13** | 47 | **14** | 24 | **15** | 68 |

### B-1b) Bindungsstudien an humanen α1- und α2-adrenergen-Rezeptoren

Zur Herstellung von Zellmembranen mit humanen α₁- und α₂-adrenergen-Rezeptoren, werden α₁- und α ₂-adrenerge-Rezeptor stabil überexprimierende CHO Zellen lysiert und anschließend differentiell zentrifugiert. Nach der Lyse in Bindungspuffer (50 mM Tris-(hydroxymethyl)-aminomethan / 1 N Salzsäure, 5 mM Magnesiumchlorid, pH 7.4) mit einem Ultra Turrax (Jahnke&Kunkel, Ika-Werk), wird das Homogenat mit 1000g bei 4°C für 10 min abzentrifugiert. Das resultierende Sediment wird verworfen und der Überstand wird mit 20000g bei 4°C für 30 min zentrifugiert. Der Überstand wird verworfen und das Sediment in Bindungspuffer resuspendiert und bis zum Bindungsversuch bei -70°C gelagert. Für den Bindungsversuch werden die Radioliganden ³H-MK-912 (2.2 - 3.2 TBq/mmol, PerkinElmer) (0.4 nM bei α_{2C}-adrRez und 1 nM bei α_{2A}-adrRez), 0.25 nM ³H-Prazosin (α_{1AC}-adrRez; 2.6 - 3.3 TBq/mmol, PerkinElmer), 0.25 nM ³H-Rauwolscine (α_{2B}-adrRez, 2.6 - 3.2 TBq/mmol, PerkinElmer) 60 Minuten lang mit 5 - 20 µg Zellmembranen in Bindungspuffer (Testgesamtvolumen 0.2 ml) in Gegenwart der Testsubstanzen bei 30°C in 96-Loch Filterplatten (FC/B Glasfaser, Multiscreen Millipore) inkubiert. Nach Beendigung der Inkubation durch Absaugen der nichtgebundenen Radioaktivität, werden die Platten mit Bindungspuffer gewaschen und anschließend bei 40°C 1 Stunde lang getrocknet. Danach wird Flüssigkeitsszintillator (Ultima Gold, PerkinElmer) hinzugefügt und die auf den Platten verblieben Radioaktivität in Flüssigkeitsszintillationszähler (Microbeta, Wallac) gemessen. Die nicht-spezifische Bindung wird als Radioaktivität in Gegenwart von 1-10 µM WB-4101 (α_{2C}-adrRez und α_{2A}-adrRez), Prazosin (α_{2B}-adrRez und α_{1AC}-adrRez) (alle von Sigma) definiert und beträgt in der Regel <25% der gebundenen Gesamtradioaktivität. Die Bindungsdaten (IC₅₀ und Dissoziationskonstante Kᵢ) werden mittels des Programms GraphPad Prism Version 4.0 bestimmt.

### B-2) In vivo Assays

### B-2a) Relaxationsmessung an isolierten Schwanzarterien der Ratte

Männliche Wistar Ratten (200-250 g) werden mit Kohlendioxid euthanisiert. Die Schwanzarterie wird präpariert und im Krebs-Henseleit Puffer bei 4°C 17 h inkubiert (Zusammensetzung in mmol/l: NaCl 112, KCl 5.9, CaCl₂ 2.0 MgCl₂ 1.2, NaH₂PO₄ 1.2, NaHCO₃ 25, Glucose 11.5). Die Arterie wird in 2 mm lange Ringe geschnitten, in einen Organbad gefüllt mit 5 ml Krebs-Henseleit Puffer übertragen und an einen Draht-Myograph (DMT, Dänemark) angeschlossen. Der Puffer wird auf 27°C gewärmt und mit 95% O₂, 5% CO₂ begast. Vor jedem Experiment wird die Ansprechbarkeit des Präparates durch Zugabe von Kaliumhaltiger Krebs-Henseleit Lösung (50 mmol/l KCl) überprüft. Nach einer 60-minütiger Äquilibrierungsphase wird mit 30 nmol/l UK 14.304 eine Kontraktion der Gefäßringe induziert. Anschließend wird die Testsubstanz in ansteigender Konzentration kumulativ hinzugefügt. Die Relaxation wird als Reduktion der von UK 14.304-induzierter Kontraktion dargestellt.

### B-2b) Hämodynamik CHF Ratte

Männliche alte Wistar, ZDF/Crl-Lepr fa/fa, SHR-SP oder Sprague Dawley Ratten (Charles River; 250 - 300 g) werden mit 5% Isofluran im Narkosekäfig narkotisiert, intubiert und anschließend künstlich beatmet (Frequenz: 60 Atemzüge/Min; Verhältnis Inspiration zu Exspiration: 50:50; positiver endexspiratorische Druck: 1 cm H₂O; Atemzugvolumen: 10 ml/kg KGW; FIO₂: 0.5; 2% Isofluran). Die Körpertemperatur wird durch eine Wärmematte auf 37-38°C gehalten. Als Schmerzmittel werden 0.05 mg/kg s.c. Temgesic gegeben. Für die hämodynamische Messung werden die Ratten tracheotomiert und künstlich beatmet (Frequenz: 60 Atemzüge/Min; Verhältnis Inspiration zu Exspiration: 50:50; positiver endexspiratorische Druck: 1 cm H₂O; Atemzugvolumen: 10 ml/kg KGW; FIO₂: 0.5). Die Narkose wird durch Isofluran-Inhalationsnarkose aufrechterhalten. Der linksventrikuläre Druck wird über die linke A. carotis mittels eines Millar-Microtip-Katheters (Millar SPR-320 2F) ermittelt. Als abgeleitete Parameter werden der systolische linksventrikuläre Druck (sLVP), der enddiastolische Ventrikeldruck (LVEDP), die Kontraktilität (+dPdt) und die Relaxationskraft (-dPdt) bestimmt. Im Anschluss an die Hämodynamik wird das Herz entnommen und das Verhältnis rechter zu linker Ventrikel inclusive Septum bestimmt. Weiterhin werden Plasmaproben zur Bestimmung von Plasmabiomarkern und Plasmasubstanzspiegeln gewonnen.

### B-2c) Messung von Blutfluss und Blutdruck in Ratten

250 - 350 g schwere Wistar Ratten (Hsd Cpb:Wu) bzw. 330 - 520 g schwere ZDF Ratten (ZDF/Crl-Lepr fa/fa) wurden mittels 2.5% Isofluran im Sauerstoff - Lachgasgemisch (40:60) narkotisiert. Zur Bestimmung des Blutflusses in der A. carotis, A. femoralis wurde die narkotisierte Ratte in Rückenlage gebracht und anschließend die linkseitige A. carotis und rechtsseitige A. femoralis vorsichtig freipräpariert. Der Blutfluss wurde durch das Anbringen von Flusssonden (Transonic Flowprobe) an den Gefäßen gemessen. Durch das Einbringen eines PE50-Arterien-Katheter in die linksseitige A. femoralis wurde der Blutdruck und die Herzrate (Transducer Ref. 5203660: Fa.Braun CH) bestimmt. Die Substanzgabe erfolgte als Bolus bzw. Dauerinfusion über einen Venen-Katheder in der linksseitigen V. femoralis.

Nach der Präparation der Tiere wurde ein 5 min Basislinien-Intervall abgewartet. Anschliessend startete die Infusion des AR alpha2C Rezeptor Antagonisten. Im steady state (32 min nach Start des Versuches) wurde der Femoralfluss in Relation (% Unterschied) zum Ausgangsfluss bestimmt.

Die Verbindung von Beispiel 8 zeigte eine dosisabhängige Steigerung des Femoralflusses in diabetischen ZDF fa/fa Tieren in den Dosen von 0,1, 0,3 und 1 µg/kg. In der Wistar Ratte wurde bis zu einer Dosis von 1µg/kg/min kein Anstieg des Femoralflusses beobachtet. Zeitgleich wurden keine Veränderungen auf Blutdruck und Herzfrequenz gemessen. Placebo: 10%Ethanol/40%PEG400/50%NaCl. Die Daten (Mittelwert) sind in Tabelle 2 dargestellt:

**Tabelle 2:**

| | % Veränderung im Femoralfluss | |
|---|---|---|
| | ZDF Ratte (n=3) | Wistar Ratte |
| Placebo | 6,3 | -1,2 (n=4) |
| Beispiel 8; 0,1 µg/kg/min | 12,3 | Nicht gemessen |
| Beispiel 8; 0,3 µg/kg/min | 65,0 | Nicht gemessen |
| Beispiel 8; 1 µg/kg/min | 131,3 | -6,7 (n=7) |

### B-2d) Prüfung von durchblutungsfördernden Substanzen (Hämodynamik)

Zur Erzeugung einer Minderperfusion wird bei Ratten (z.B. ZDF/Crl-Lepr fa/fa) in Narkose (z.B. Inhalationsnarkose Isofluran, Enfluran) unter sterilen Bedingungen die rechte A. iliaca externa ligiert. Je nach Kollateralisierungsgrad der Tiere muss zur Erzeugung einer Minderperfusion zusätzlich noch die A. femoralis ligiert werden. Im Anschluss an die Operation oder auch präventiv werden die Versuchstiere oral, intragastral (Magensonde, Futter- oder Trinkwasseraufnahme), intraperitoneal, intravenös, intraarteriell, intramuskulär, inhalativ oder subcutan mit den Prüfsubstanzen behandelt. Die Prüfsubstanzen werden für bis zu 50 Wochen einmal oder mehrfach enteral oder parenteral täglich appliziert oder es erfolgt eine Dauerapplikation über subkutan implantierte osmotische Minipumpen (z.B. Alzet Pumpen). Die Mikroperfusion und Temperatur der unteren Extremitäten werden während dem Versuch dokumentiert. Dabei wird den Ratten unter Narkose eine temperatursensitive Laserdopplersonde (Periflux) auf die Pfoten geklebt und somit Microperfusion und Hauttemperatur gemessen. Je nach Versuchsplan werden Proben wie Blut (Interimsdiagnostik) und sonstige Körperflüssigkeiten, Urin oder Organe entnommen, um in vitro weitere Untersuchungen durchzuführen oder es wird zur Dokumentation der Hämodynamik über einen Katheter in der A. carotis Blutdruck und Herzfrequenz gemessen. Am Ende des Experiments werden die Tiere schmerzlos getötet.

### B-2e) Prüfung von durchblutungsfördernden Substanzen (Mikrozirkulation)

Bei diabetischen (ZDFfa/fa) und gesunden Ratten (Wistar) wurde unter anaesthesierten Bedingungen (Isoflurannarkose) an der Fußsohle zur Messung der cutanen Mikrozirkulation eine Laserdopplersonde angebracht. Die Versuchstiere wurden einmalig oral mit den Prüfsubstanzen behandelt. Die Mikroperfusion und Temperatur der unteren Extremitäten wurden während des Versuchs fortlaufend dokumentiert. Dabei wurde den Tieren eine temperatursensitive Laserdopplersonde (Periflux, O2C) auf die Pfoten geklebt und hierüber Mikroperfusion und Hauttemperatur gemessen. Die Messwerte der Mikrozirkulation 30min nach der oralen Gabe der Prüfsubstanz wurden an beiden Pfoten gemessen. Aus diesen Daten wurden Mittelwerte gebildet und mit Placebo behandelten Tieren verglichen. Dargestellt sind die minimal effektiven Dosen (MED), bei denen die Prüfsubstanzen eine signifikante Verbesserung der Mikrozirkulation gegen Placebo (Vehikel = 10%EtOH+30%PEG400+60%Wasser für Injektionszwecke; 1 ml/kg) gezeigt haben und der Faktor, um den die Mikrozirkulation im Vergleich zu Placebo bei dieser Dosis erhöht ist. Zusätzlich ist auch die MED für die signifikante Erhöhung der Hauttemperatur angegeben (ttest).

Mikrozirkulationsdaten zu Adrenorezeptor α_{2C} Rezeptor Antagonist der Verbindung von Beispiel 8 und zu der Vergleichssubstanz ORM12741, ein AR α2c Rezeptor Antagonist der Fa. Orion sind in Tabelle 3 gezeigt:

**Tabelle 3:**

| Beispiel-Nr. | MED [mg/kg] Mikrozirkulation | MED [mg/kg] Hauttemperatur |
|---|---|---|
| 8 | 0,03 (1,8x) | 0,01 |
| ORM-12741 (Orion) | 0,1 (1,9x) | 0,01 |

### B-2f) Prüfung von durchblutungsfördernden Substanzen (Motorische Funktion) im Laufradversuch

Zur Bestimmung der motorischen Funktion wird das Laufverhalten von Mäusen (z.B. eNOS knock out Mäuse, Wildtyp Mäuse C-57 B16 oder ApoE knock out Mäuse) in Laufrädern untersucht. Um die Mäuse an die freiwillige Benutzung des Laufrades zu gewöhnen, werden 4-5 Wochen vor Beginn des Versuches die Tiere in Käfigen mit Laufrad vereinzelt und trainiert. 2 Wochen vor Start des Experimentes werden die Bewegungen der Mäuse im Laufrad durch eine Photozelle mittels Computer aufgezeichnet und verschiedene Laufparameter wie z.B. die tägliche Laufstrecke, die zurückgelegten Einzelstrecken, aber auch Ihre zeitliche Verteilung über den Tag bestimmt. Die Tiere werden nach ihrem natürlichen Laufverhalten in Gruppen (8-12 Tiere) randomisiert (Kontrollgruppe, Sham Gruppe und ein bis mehrere Substanzgruppen). Nach der 2-wöchigen Einlaufphase wird zur Erzeugung einer Minderperfusion in den Hinterläufen unter Narkose unter sterilen Bedingungen (z.B. Inhalationsnarkose mit Isofluran) beidseitig die A. femoralis ligiert. Im Anschluss an die Operation oder auch präventiv werden die Versuchstiere oral, intragastral (Magensonde, Futter- oder Trinkwasseraufnahme), intraperitoneal, intravenös, intraarteriell, intramuskulär, inhalativ oder subcutan mit den Prüfsubstanzen behandelt. Die Prüfsubstanzen werden für bis zu 5 Wochen einmal oder mehrfach enteral oder parenteral täglich appliziert oder es erfolgt eine Dauerapplikation über subkutan implantierte osmotische Minipumpen. Das Laufverhalten der Tiere wird über mehrere Wochen nach der Operation beobachtet und aufgezeichnet. Am Ende des Experiments werden die Tiere schmerzlos getötet. Je nach Versuchsplan werden Proben wie Blut und sonstige Körperflüssigkeiten oder Organe entnommen, um in vitro weitere Untersuchungen durchzuführen (S. Vogelsberger Neue Tiermodelle für die Indikation Claudicatio Intermittens (Taschenbuch), Verlag: VVB Laufersweiler Verlag (März 2006), ISBN-10: 383595007X, ISBN-13: 978-3835950078).

### B-2g) Prüfung von durchblutungsfördernden Substanzen (Verschlussdruckmessung)

Zur Erzeugung einer Minderperfusion wird bei Ratten (z.B. ZDF Ratten) in Narkose (z.B. Inhalationsnarkose Isofluran) unter sterilen Bedingungen die rechte A. iliaca externa ligiert. Je nach Kollateralisierungsgrad der Tiere muss zur Erzeugung einer Minderperfusion zusätzlich noch die A. femoralis ligiert werden. Im Anschluss an die Operation oder auch präventiv werden die Versuchstiere oral, intragastral (Magensonde, Futter- oder Trinkwasseraufnahme), intraperitoneal, intravenös, intraarteriell, intramuskulär, inhalativ oder subcutan mit den Prüfsubstanzen behandelt. Die Prüfsubstanzen werden für bis zu 5 Wochen einmal oder mehrfach enteral oder parenteral täglich appliziert oder es erfolgt eine Dauerapplikation über subkutan implantierte osmotische Minipumpen (z.B. Alzet Pumpen). Die Verschlussdrücke der Tiere werden vor der Operation (anschließende Randomisierung) und einmal wöchentlich für einen Zeitraum von bis zu 2 Monaten nach der Operation gemessen. Dabei wird den Ratten unter Narkose eine aufblasbare Manschette um die Hinterläufe gelegt und eine temperierbare Laserdopplersonde (Periflux) auf die Pfoten geklebt. Die Manschetten werden aufgepumpt bis von den Laserdopplersonden kein Blutfluss mehr gemessen wird. Anschließend wird der Druck der Manschetten kontinuierlich graduiert abgelassen und der Druck bestimmt, bei dem wieder Blutfluss detektiert wird. Je nach Versuchsplan werden Proben wie Blut (Interimsdiagnostik) und sonstige Körperflüssigkeiten oder Organe entnommen, um in vitro weitere Untersuchungen durchzuführen. Am Ende des Experiments werden die Tiere schmerzlos getötet (S. Vogelsberger Neue Tiermodelle für die Indikation Claudicatio Intermittens (Taschenbuch), Verlag: VVB Laufersweiler Verlag (März 2006), ISBN-10: 383595007X, ISBN-13: 978-3835950078.)

### B-2h) Prüfung von die Wundheilung beeinflussenden Substanzen (Ulcermodell)

Zur Induktion einer oberflächlichen Wunde wurden diabetische Mäuse (db/db, i.e. BKS.Cg-m Dock7m +/+ Leprdb /J mice) durch Isofluran narkotisiert. Auf einem enthaarten, desinfizierten Hautareal aus der linken Flanke wurde eine durchgehende Läsion (10 mm x 10 mm) gesetzt. Die Tiere wurden anschließend auf die unterschiedlichen Versuchsgruppen randomisiert. In allen Gruppen wurden die Wunden mit Verbänden (Systagenix Wound Management, UK) verschlossen. Die Tiere wurden täglich (ab Tag 1 nach der Wundsetzung) mittels Gavage (200/µl, Vehikel = 10%EtOH+30%PEG400+60%Wasser für Injektionszwecke) mit den Substanzen in den angegebenen Dosierungen behandelt. An den Tagen 4, 8, 12, 16 und 20 wurden die Tiere anaesthesiert, die Verbände entfernt und die Wundgröße wurde mittels Digitalfotos gemessen. Die Aufnahmen wurden mittels automatisierten, kalibrierten planimetrischen Verfahren ausgewertet.

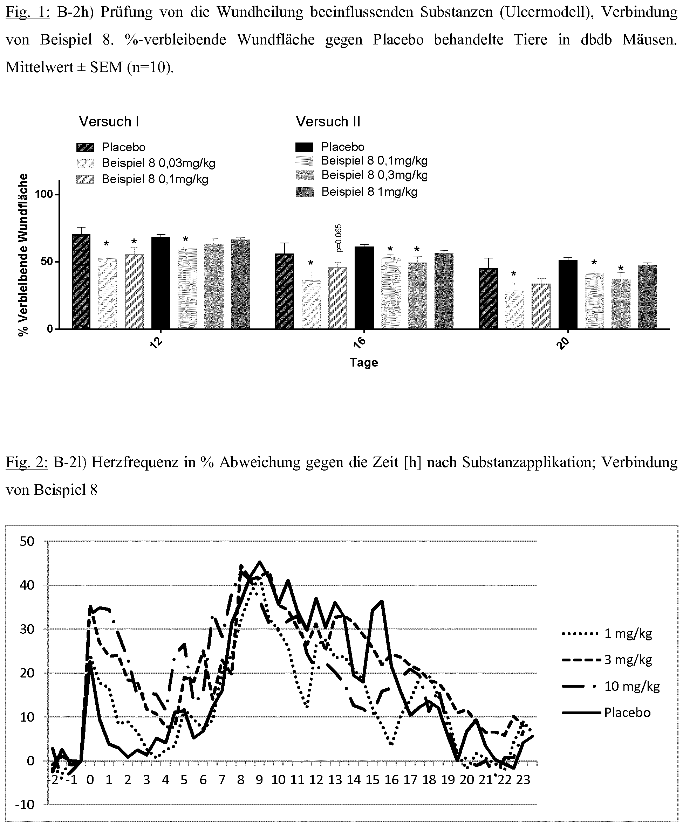

Die Ergebnisse sind in Fig. 1 dargestellt als verbleibende Wundgrößen über den Versuchsverlauf. Dazu wurden alle Einzelwerte prozentual auf das individualisierte Tier am Tag der Wundsetzung bezogen. Dargestellt sind Mittelwerte +/- SEM.

### B-2i) Prüfung auf die Nierenfunktion beeinflussende Substanzen

Bei Tieren mit akuter und oder krankheitsbedingter Nierenschädigung (z.B. STZ Ratte, ZDF Ratte, ZDF Ratte mit DOCA Implantat, UUO Nierenschädigungsmodell, Glomerulonephritismodell, Diabetes, Atherosklerose) wird in regelmäßigen Abtsänden vor bzw. unter Dauerbehandlung mit den Prüfsubstanzen eine Diurese durchgeführt. Die Versuchstiere werden oral, intragastral (Magensonde, Futter- oder Trinkwasseraufnahme), intraperitoneal, intravenös, intraarteriell, intramuskulär, inhalativ oder subcutan mit den Prüfsubstanzen behandelt. Die Prüfsubstanzen werden einmal oder mehrfach enteral oder parenteral täglich appliziert oder es erfolgt eine Dauerapplikation über subkutan implantierte osmotische Minipumpen (z.B. Alzet Pumpen). Die Plasma- und Urinparameter werden während der gesamten Versuchsdauer bestimmt.

### B-2j) Hämodynamik im narkotisierten Hund

Es werden 25-35 kg schwere gesunde oder herzinsuffiziente Mongrel® Hunde (Marshall BioResources, Marshall Farms Inc; Clyde NY; USA) beiderlei Geschlechts verwendet. Die Narkose wird eingeleitet durch langsame i.v. Gabe von 25 mg/kg Natrium Thiopental (Trapanal®) und 0.15 mg/kg Alcuronium Chlorid (Alloferin®) und während des Experimentes aufrechterhalten mittels einer Dauerinfusion aus 0.04 mg/kg*h Fentanyl (Fentanyl®), 0.25 mg/kg*h Droperidol (Dihydrobenzperidol®) und 15 µg/kg/h Alcuronium Chloride (Alloferin®). Nach der Intubation werden die Tiere über die Beatmungsmaschine mit konstanten Atemvolumen beatmet, so dass eine endtidale CO₂ Konzentration von etwa 5% erreicht wird. Die Beatmung erfolgt mit Raumluft, angereichert mit ca. 30% Sauerstoff (Normoxie). Zur Messung der hämodynamischen Parameter wird ein mit Flüssigkeit gefüllter Katheter in die A. femoralis zur Messung des Blutdruckes implantiert. Ein 2-lumiger Swan-Ganz® Katheter wird über die V. jugularis in die Pulmonalarterie eingeschwemmt (distales Lumen zur Messung des pulmonalarteriellen Druckes, proximales Lumen zur Messung des zentralen Venendruckes). Mittels Temperaturfühler an der Spitze des Katheters wird der kontinuierliche Cardiac Output (CCO) bestimmt. Der Blutfluss wird an unterschiedlichen Gefäßbetten wie z.B. der Koronararterie, der A. Carotis oder der Femoralarterie durch das Anbringen von Flusssonden (Transonic Flowprobe) an den jeweiligen Gefäßen gemessen. Der linksventrikuläre Druck wird nach Einführung eines Mikro-Tip-Katheters (Millar® Instruments) über die A. carotis in den linken Ventrikel gemessen und davon das dP/dt als Maß für die Kontraktilität abgeleitet. Substanzen werden i.v. über die V. femoralis oder intraduodenal als kumulative Dosis-Wirkungskurve (Bolus oder Dauerinfusion) appliziert. Die hämodynamischen Signale werden mittels Druckaufnehmern / Verstärkern und PONEMAH® als Datenerfassungssoftware aufgezeichnet und ausgewertet.

Zur Induktion von Herzinsuffizienz wird den Hunden unter sterilen Bedingungen ein Schrittmacher implantiert. Nach Induktion der Narkose mittels Pentobarbital-Na (15 to 30 mg kg⁻¹ i.v.) gefolgt von einer Intubation und anschliessender Ventilation (room air; Sulla 808, Dräger®, Germany) wird die Narkose durch kontinuierliche Infusion von Pentobarbital (1-5 mg kg⁻¹ h⁻¹) und Fentanyl (10-40 µg kg⁻¹ h⁻¹) aufrechterhalten. Ein Schrittmacherkabel (Setrox S60®, Biotronik, Germany) wird implantiert über eine Insertion der linken Vena Jugularis und im rechten Ventrikel plaziert. Das Kabel wird mit dem Schrittmacher (Logos®, Biotronik, Germany) verbunden, der in einer kleinen subkutanen Tasche zwischen den Schulterblättern positioniert wird. Erst 7 Tage nach dem Eingriff wird das ventrikuläre Pacing zur Erzielung einer Herzinsuffiziens bei einer Frequenz von 220 Schlägen/minüber einen Zeitraum von 10-28 Tagen gestartet.

### B-2k) Bestimmung der antidepressiven Wirkung im Rat-forced-swimming-test

Ratten, die in einem engen Raum, aus dem es kein Entkommen gibt, gezwungen werden zu schwimmen, adaptieren sich nach einer ersten Phase gesteigerter Aktivität, indem sie eine charakteristische unbewegliche Haltung einnehmen, und nur noch absolut notwendige Bewegungen ausüben, um den Kopf über Wasser zu halten. Diese Immobilität kann durch eine Reihe klinisch aktiver Antidepressiva reduziert werden (z.B. Cryan JF, Markou A, Lucki I. Assessing antidepressant activity in rodents: recent developments and future needs. Trends Pharmacol. Sci. 2002; 23:238-245). Die hier verwendete Methode basiert auf dem Protokoll von Porsolt et al. (Porsolt RD, Anton G, Blavet N, Jalfre M. Behavioural despair in rats: a new model sensitive to antidepressant treatments. Eur. J. Pharmacol. 1978; 47:379-91; und Porsolt RD, Brossard G, Hautbois C, Roux S. Rodent models of depression: forced swimming and tail suspension behavioral despair tests in rats and mice. Curr. Protoc. Neurosci. 2001; Chapter 8:Unit 8.10A, 1-10) und De Vry et al. (De Vry J, Maurel S, Schreiber R, de Beun R, Jentzsch KR. Comparison of hypericum extracts with imipramine and fluoxetine in animal models of depression and alcoholism. Eur. Neuropsychopharmacology 1999; 9:461-468). In 2 Sitzungen (Training und Test) mit einem Abstand von 24 h werden die Ratten in einem Wasser gefüllten engen Zylinder, aus dem sie nicht entkommen können, gezwungen, zu schwimmen. Die Trainingssitzung (15 min Dauer) wird vor der Substanzbehandlung durchgeführt ohne Aufzeichnung des Verhaltens, um die Ratten an die 5 minütige Testsitzung 24 h später zu gewöhnen. Während beider Sitzungen werden die Ratten einzeln in die wassergefüllten Zylinder gesetzt, die optisch voneinander getrennt sind. Nach der Sitzung werden die Ratten aus dem Wasser genommen und getrocknet. Die Ratten werden mit der Testsubstanz oder der Vehikel Lösung ungefähr 24, 5 und 1 h vor der Testsitzung behandelt, die erste Applikation wird unverzüglich nach der Trainingssitzung verabreicht. 3 Substanz Applikationen vor der Testsitzung führen zu stabileren pharmakologischen Ergebnissen als eine einzelne Applikation. Die Testsitzungen werden mittels einer Videoüberwachungskamera elektronisch aufgezeichnet und mittels Computer nach Speicherung off-line analysiert.Die Analyse des Verhaltens wird bei jedem Tier von 3-4 unabhängigen Beobachtern durchgeführt, die die Gesamtzeit der Immobilität in Sekunden während der 5 minütigen Testsitzung scoren.

Passives Verhalten oder Immobilität sind definiert als eine Ratte, die in aufrechter Position im Wasser treibt und dabei nur kleine Bewegungen ausübt, um den Kopf über Wasser zu halten oder Ihren Körper in einer ausbalanzierten stabilen Position zu halten. Im Gegensatz dazu ist aktives Verhalten gekennzeichnet durch aktive Schwimmbewegungen, z.B. heftige Bewegeungen der Vorder oder Hinterbeine und/oder des Schwanzes, klettern oder tauchen.

Die Dauer der festgestellten Immobilität durch die Untersucher wird pro Tier und Behandlungsgruppe gemittelt. Unterschiede in der Dauer der Immobilität zwischen den Gruppen werden statistisch mittels ANOVA oder eines geeigneten nicht-parametrischen Test mit p <0,05 als Signifikanzlevel untersucht.

### B-2l) Radiotelemetrische Messung von Blutdruck und Herzfrequenz an wachen Ratten

Für die im Folgenden beschriebenen Messungen an wachen Ratten wurde ein im Handel erhältliches Telemetriesystem der Firma Data Sciences International DSI, USA, eingesetzt. Das System besteht aus 3 Hauptkomponenten: (1) Implantierbare Sender (Physiotel® Telemetrietransmitter), (2) Empfänger (Physiotel® Receiver), die über einen Multiplexer (DSI Data Exchange Matrix) mit einem (3) Datenakquisitionscomputer verbunden sind. Die Telemetrieanlage ermöglicht eine kontinuierliche Erfassung von Blutdruck, Herzfrequenz und Körperbewegung an wachen Tieren in ihrem gewohnten Lebensraum.

Die Untersuchungen wurden an ausgewachsenen weiblichen Wistar-Ratten mit einem Körpergewicht von > 200 g durchgeführt. Die Versuchstiere wurden nach Senderimplantation einzeln in Makrolon®-Käfigen Typ III gehalten. Sie hatten freien Zugang zu Standardfutter und Wasser. Der Tag/Nacht-Rhythmus im Versuchslabor wurde durch Wechsel der Raumbeleuchtung eingestellt.

### Senderimplantation:

Die eingesetzten Telemetriesender (PA-C40, DSI) wurden den Versuchstieren mindestens 14 Tage vor dem ersten Versuchseinsatz unter aseptischen Bedingungen chirurgisch implantiert.

Zur Implantation wurden die nüchternen Tiere mit Isofluran (IsoFlo®, Abbott, Einleitung 5%, Erhaltung 2%) narkotisiert und an der Bauchseite weiträumig rasiert und desinfiziert. Nach Eröffnung des Bauchraumes entlang der *Linea alba* wurde der flüssigkeitsgefüllte Messkatheter des Systems oberhalb der Bifurcation nach cranial in die *Aorta descendens* eingesetzt und mit Gewebekleber (VetBond™, 3M) befestigt. Das Sendergehäuse wurde intraperitoneal an der Bauchwandmuskulatur fixiert und die Wunde schichtweise verschlossen. Postoperativ wurde zur Infektionsprophylaxe ein Antibiotikum (Ursocyclin® 10%, 60 mg/kg s.c., 0.06 ml/100 g Körpergewicht, Serumwerk Bernburg AG, Deutschland) sowie ein Analgetikum (Rimadyl®, 4 mg/kg s.c., Pfizer, Deutschland) verabreicht.

### Substanzen und Lösungen:

Wenn nicht anders beschrieben, wurden die zu untersuchenden Substanzen jeweils einer Gruppe von Tieren (n = 6) oral verabreicht. Entsprechend einem Applikationsvolumen von 2 ml/kg Körpergewicht wurden die Testsubstanzen in geeigneten Lösungsmittelgemischen gelöst. Eine Lösungsmittelbehandelte Gruppe von Tieren (Placebo/Vehikel = Diethylenglycol monoethylether, Transcutol®, 2ml/kg p.o.) wurde als Kontrolle eingesetzt.

### Versuchsablauf:

Die Telemetrie-Messeinrichtung ist für 24 Tiere konfiguriert.

Den in der Anlage lebenden instrumentierten Ratten war jeweils eine eigene Empfangsantenne zugeordnet (RPC-1 Receiver, DSI). Die implantierten Sender waren über einen eingebauten Magnetschalter von außen aktivierbar und wurden bei Versuchsvorlauf auf Sendung geschaltet. Die ausgestrahlten Signale wurden durch ein Datenakquisitionssystem (Dataquest™ A.R.T. for Windows, DSI) online erfasst und entsprechend aufgearbeitet.

Im Standardablauf wurden über je 10 Sekunden Dauer gemessen: (1) systolischer Blutdruck (SBP), (2) diastolischer Blutdruck (DBP), (3) arterieller Mitteldruck (MAP), (4) Herzfrequenz (HR) und (5) Aktivität (ACT). Diese Parameter wurden im Anschluss an die Applikation über 24 Stunden gemessen.

Die Messwerterfassung wurde rechnergesteuert in 5 Minuten-Abständen wiederholt. Die als Absolutwert erhobenen Quelldaten wurden im Diagramm mit dem aktuell gemessenen Barometerdruck (Ambient Pressure Reference Monitor, APR-1, DSI) korrigiert.

### Auswertung:

Nach Versuchsende wurden die erhobenen Einzeldaten mit der Analysis-Software (Dataquest™ A.R.T. 4.1 Analysis) sortiert. Als Leerwert wurde der Mittelwert des Vorlaufs (d.h. vor Substanzapplikation) genommen (4 Absolutwerte) und dieser mit dem Absolutwert der Messung verglichen, woraus sich die % Abweichung ergab. Die Daten wurden über eine voreinstellbare Zeit durch Mittelwertbestimmung geglättet (15 Minuten-Mittelwert).

### Literatur:

K. Witte, K. Hu, J. Swiatek, C. Müssig, G. Ertl und B. Lemmer, Experimental heart failure in rats: effects on cardiovascular circadian rhythms and on myocardial β-adrenergic signaling, Cardiovasc. Res. 47 (2): 203-405, 2000.

### Ergebnisse:

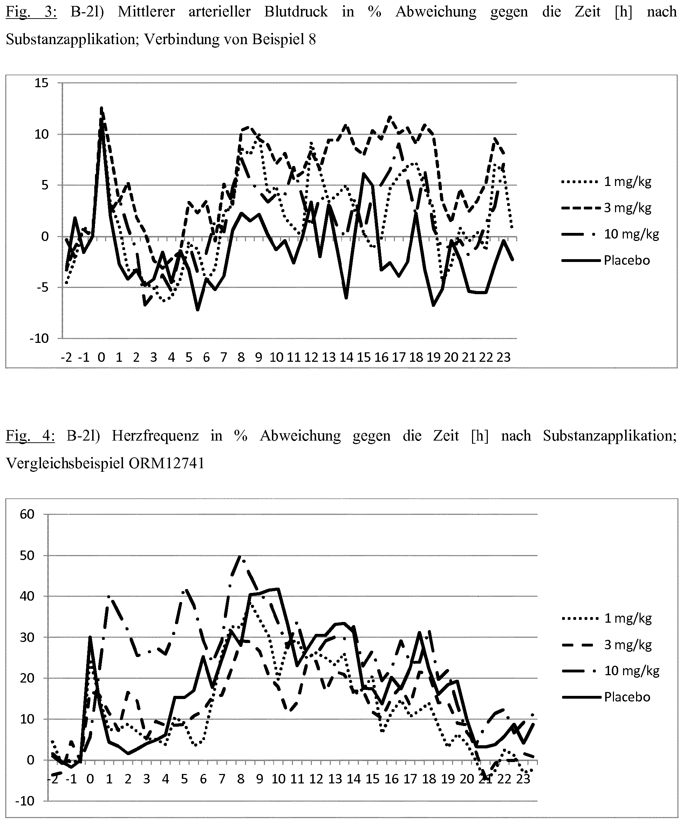

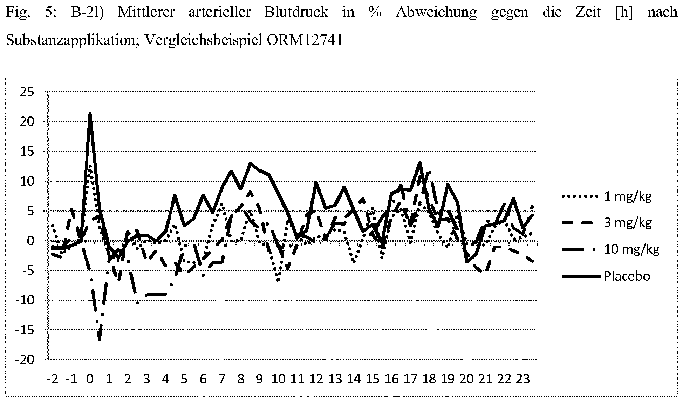

Die Ergebnisse sind für die Verbindung von Beispiel 8 im Vergleich zu einem Adrenorezeptor α_{2C} Rezeptor Antagonist von Orion (ORM-12741), der zur Therapie der Alzheimer'schen Erkrankung und des Raynaud Syndroms getestet wurde, in den Abbildungen 2 bis 5 dargestellt.

Beispiel 8 zeigte keine hämodynamischen Effekte (Blutdruck, Herzfrequenz) bis zu einer per oralen Dosierung von 1mg/kg; mit 3 und 10 mg/kg wurde ein geringer transienter Anstieg der Herzfrequenz beobachtet. Im Gegensatz dazu zeigte die Vergleichssubstanz ORM-12741, ein AR α2c Rezeptor Antagonist der Fa. Orion, bei 10mg/kg einen zusätzlichen Blutdruckabfall.

### Erklärung der Abbildungen:

Fig. 1: B-2h) Prüfung von die Wundheilung beeinflussenden Substanzen (Ulcermodell). %-verbleibende Wundfläche gegen Placebo behandelte Tiere in dbdb Mäusen. Mittelwert ± SEM (n=10).
Fig. 2: B-2l) Herzfrequenz in % Abweichung gegen die Zeit [h] nach Substanzapplikation; Beispiel 8
Fig. 3: B-2l) Mittlerer arterieller Blutdruck in % Abweichung gegen die Zeit [h] nach Substanzapplikation; Beispiel 8
Fig. 4: B-2l) Herzfrequenz in % Abweichung gegen die Zeit [h] nach Substanzapplikation; Vergleichsbeispiel ORM12741
Fig. 5: B-2l) Mittlerer arterieller Blutdruck in % Abweichung gegen die Zeit [h] nach Substanzapplikation; Vergleichsbeispiel ORM12741

### C) Ausführungsbeispiele für pharmazeutische Zusammensetzungen

Die erfindungsgemäßen Substanzen können folgendermaßen in pharmazeutische Zubereitungen überführt werden:

### Tablette:

### Zusammensetzung:

100 mg der Verbindung des Beispiels 1, 50 mg Lactose (Monohydrat), 50 mg Maisstärke, 10 mg Polyvinylpyrolidon (PVP 25) (Fa. BASF, Deutschland) und 2 mg Magnesiumstearat.

Tablettengewicht 212 mg. Durchmesser 8 mm, Wölbungsradius 12 mm.

### Herstellung:

Die Mischung aus der Verbindung des Beispiels 1, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben).

### Orale Suspension:

### Zusammensetzung:

1000 mg der Verbindung des Beispiels 1, 1000 mg Ethanol (96%), 400 mg Rhodigel (Xanthan gum) (Fa. FMC, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die Verbindung des Beispiels 1 wird der Suspension zugefügt. Unter Rühren erfolgt die Zugabe des Wassers. Bis zum Abschluss der Quellung des Rhodigels wird ca. 6h gerührt.

### Intravenös applizierbare Lösung:

### Zusammensetzung:

1 mg der Verbindung von Beispiel 1, 15 g Polyethylenglykol 400 und 250 g Wasser für Injektionszwecke.

### Herstellung:

Die Verbindung von Beispiel 1 wird zusammen mit Polyethylenglykol 400 in dem Wasser unter Rühren gelöst. Die Lösung wird sterilfiltriert (Porendurchmesser 0.22 µm) und unter aseptischen Bedingungen in hitzesterilisierte Infusionsflaschen abgefüllt. Diese werden mit Infusionsstopfen und Bördelkappen verschlossen.

## Patentansprüche

1. Adrenorezeptor α2C Rezeptor Antagonisten zur Verwendung in einem Verfahren zur Behandlung und/oder Prophylaxe von Begleit- und/oder Folgeerkrankungen von Diabetes mellitus, diabetischen Herzerkrankungen, diabetischen koronaren Herzerkrankungen, diabetischen koronaren mikrovaskulären Herzerkrankungen, diabetischer Herzinsuffizienz, diabetischer Kardiomyopathie und Herzinfarkt, diabetischer Mikroangiopathie, diabetischer Retinopathie, diabetischer Neuropathie, diabetischer Nephropathie, diabetischer erektiler Dysfunktion, diabetischen Geschwüren an den Extremitäten, diabetischen Fußulzera, zur Förderung der diabetischen Wundheilung und zur Förderung der Wundheilung von diabetischen Fußulzera.

2. Adrenorezeptor α2C Rezeptor Antagonisten gemäß Anspruch 1, **dadurch gekennzeichnet, dass** diese zur Behandlung und/oder Prophylaxe von Begleit- und/oder Folgeerkrankungen von primären und sekundären Formen von Herzinsuffizienz, peripheren und kardialen Gefäßerkrankungen, thromboembolischen Erkrankungen und Ischämien, peripheren Durchblutungsstörungen, Raynaud-Phänomen, Mikrozirkulationsstörungen, Claudicatio intermittens, peripheren und autonomen Neuropathien, diabetischen Mikroangiopathien, diabetischer Nephropathie, diabetischer Retinopathie, diabetischen Geschwüren an den Extremitäten und CREST-Syndrom, sowie zur diabetischen Wundheilung, insbesondere zur Förderung der Wundheilung von diabetischen Fußulzera verwendet werden.

3. Adrenorezeptor α2C Rezeptor Antagonisten gemäß Anspruch 1, **dadurch gekennzeichnet, dass** diese zur Behandlung und/oder Prophylaxe von Begleit- und/oder Folgeerkrankungen von primären und sekundären Formen von diabetischen Mikroangiopathien, diabetischer Wundheilung, diabetischen Geschwüren an den Extremitäten, insbesondere zur Förderung der Wundheilung von diabetischen Fußulzera, diabetischer Retinopathie, diabetischer Nephropathie, diabetischer erektiler Dysfunktion, diabetischer Herzinsuffizienz, diabetischen koronaren mikrovaskulären Herzerkrankungen, peripheren und kardialen Gefäßerkrankungen, thromboembolischen Erkrankungen und Ischämien, peripheren Durchblutungsstörungen, Raynaud-Phänomen, CREST-Syndrom, Mikrozirkulationsstörungen, Claudicatio intermittens, und peripheren und autonomen Neuropathien.
